(19) **Europäisches Patentamt / European Patent Office / Office européen des brevets**

(11) **EP 2 908 724 B1**

(12) **FASCICULE DE BREVET EUROPEEN**

(45) Date de publication et mention
de la délivrance du brevet:
**08.06.2022 Bulletin 2022/23**

(21) Numéro de dépôt: **13785562.3**

(22) Date de dépôt: **15.10.2013**

(51) Classification Internationale des Brevets (IPC):
*A61B 5/055* (2006.01)   *A61B 5/00* (2006.01)
*G01R 33/563* (2006.01)   *G06T 7/00* (2017.01)
*G06T 7/238* (2017.01)   *G16H 30/40* (2018.01)
*G16H 50/20* (2018.01)   *G16H 50/30* (2018.01)

(52) Classification Coopérative des Brevets (CPC):
**A61B 5/055; A61B 5/4064; A61B 5/7264;
A61B 5/7267; A61B 5/7275; G01R 33/56341;
G06T 7/0012; G06T 7/238; G16H 30/40;
G16H 50/20;** A61B 5/4842; A61B 2576/026;
G06T 2207/10088; G06T 2207/30016; G16H 50/30

(86) Numéro de dépôt international:
**PCT/FR2013/052454**

(87) Numéro de publication internationale:
**WO 2014/060695 (24.04.2014 Gazette 2014/17)**

(54) **PROCÉDÉ DE SUIVI DE L'ÉVOLUTION D'UNE LÉSION CÉRÉBRALE**

VERFAHREN ZUR VERFOLGUNG DER ENTWICKLUNG EINER GEHIRNVERLETZUNG

METHOD FOR TRACKING THE DEVELOPMENT OF A BRAIN INJURY

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **17.10.2012 FR 1259910
07.02.2013 FR 1351046
07.02.2013 FR 1351047**

(43) Date de publication de la demande:
**26.08.2015 Bulletin 2015/35**

(73) Titulaire: **Assistance Publique - Hôpitaux de Paris
75004 Paris (FR)**

(72) Inventeurs:
• **PUYBASSET, Louis**
**F-75016 Paris (FR)**
• **GALANAUD, Damien**
**F-75014 Paris (FR)**
• **BENALI, Habib**
**F-91700 Sainte-Geneviève-des-Bois (FR)**
• **PERLBARG, Vincent**
**F-75013 Paris (FR)**
• **LEHERICY, Stéphane**
**F-75014 Paris (FR)**

(74) Mandataire: **Lebkiri, Alexandre
Cabinet Camus Lebkiri
25, Rue de Maubeuge
75009 Paris (FR)**

(56) Documents cités:
• **KAREN CAEYENBERGHS ET AL:
"Brain-behavior relationships in young traumatic
brain injury patients: DTI metrics are highly
correlated with postural control", HUMAN BRAIN
MAPPING, vol. 31, no. 7, 8 juillet 2010
(2010-07-08), pages 992-1002, XP055069954,
ISSN: 1065-9471, DOI: 10.1002/hbm.20911**
• **S. WANG ET AL: "Mild Hypoxic-Ischemic Injury
in the Neonatal Rat Brain: Longitudinal
Evaluation of White Matter Using Diffusion
Tensor MR Imaging", AJNR, AMERICAN
JOURNAL OF NEURORADIOLOGY, vol. 30, no.
10, 1 novembre 2009 (2009-11-01), pages
1907-1913, XP055069764, ISSN: 0195-6108, DOI:
10.3174/ajnr.A1697**

- METWALLI N S ET AL: "Utility of axial and radial diffusivity from diffusion tensor MRI as markers of neurodegeneration in amyotrophic lateral sclerosis", BRAIN RESEARCH, ELSEVIER, AMSTERDAM, NL, vol. 1348, 12 août 2010 (2010-08-12), pages 156-164, XP027174295, ISSN: 0006-8993 [extrait le 2010-06-01]
- CHAN K C ET AL: "Late measures of microstructural alterations in severe neonatal hypoxic-ischemic encephalopathy by MR diffusion tensor imaging", INTERNATIONAL JOURNAL OF DEVELOPMENTAL NEUROSCIENCE, PERGAMON, OXFORD, GB, vol. 27, no. 6, 1 octobre 2009 (2009-10-01), pages 607-615, XP026469807, ISSN: 0736-5748, DOI: 10.1016/J.IJDEVNEU.2009.05.012 [extrait le 2009-06-06]
- MIIA PITKONEN ET AL: "Long-term evolution of diffusion tensor indices after temporary experimental ischemic stroke in rats", BRAIN RESEARCH, ELSEVIER, AMSTERDAM, NL, vol. 1445, 19 janvier 2012 (2012-01-19), pages 103-110, XP028467880, ISSN: 0006-8993, DOI: 10.1016/J.BRAINRES.2012.01.043 [extrait le 2012-01-28]
- VINCENT PERLBARG ET AL: "Relation between brain lesion location and clinical outcome in patients with severe traumatic brain injury: A diffusion tensor imaging study using voxel-based approaches", HUMAN BRAIN MAPPING, vol. 30, no. 12, 8 juin 2009 (2009-06-08), pages 3924-3933, XP055033741, ISSN: 1065-9471, DOI: 10.1002/hbm.20817
- JOSHUA BETZ ET AL: "Prognostic Value of Diffusion Tensor Imaging Parameters in Severe Traumatic Brain Injury", JOURNAL OF NEUROTRAUMA, vol. 29, no. 7, 17 avril 2012 (2012-04-17), pages 1292-1305, XP055076969, ISSN: 0897-7151, DOI: 10.1089/neu.2011.2215
- ONI MARGARET B ET AL: "Diffusion Tensor Imaging Analysis of Frontal Lobes in Pediatric Traumatic Brain Injury", JOURNAL OF CHILD NEUROLOGY, DECKER PERIODICALS, HAMILTON, CA, vol. 25, no. 8, 1 août 2010 (2010-08-01), pages 976-984, XP008168487, ISSN: 0883-0738, DOI: 10.1177/0883073809356034 [extrait le 2010-03-23]
- Anonymous: "Lésion (médecine) - Wikipédia", , 30 April 2019 (2019-04-30), XP055808703, Retrieved from the Internet: URL:https://fr.wikipedia.org/wiki/Lésion_( méde cine) [retrieved on 2021-05-28]
- A. Sidaros ET AL: "Diffusion tensor imaging during recovery from severe traumatic brain injury and relation to clinical outcome: a longitudinal study", Brain, vol. 131, no. 2, 1 February 2008 (2008-02-01), pages 559-572, XP055076998, ISSN: 0006-8950, DOI: 10.1093/brain/awm294

## Description

### Domaine technique

La présente invention se rapporte à un procédé ex-vivo de suivi de l'évolution d'une lésion cérébrale.
La présente invention trouve notamment une application dans le domaine médicale et dans le domaine des études cliniques.
Dans la description ci-dessous, les références entre crochets ([ ]) renvoient à la liste des références présentée à la fin du texte.

### Etat de la technique

Chez l'homme, les traumatismes crâniens sont la principale cause de mortalité, de coma et de handicap sévère avant 45 ans. Ils sont dus, principalement à des accidents de la voie publique, environ 50 %, les accidents sportifs, les accidents de travail, les accidents domestiques, les agressions. Par exemple Il est important de savoir que le traumatisme crânien correspond à 75 % des causes de mortalité chez les jeunes de moins de 30 ans. Il est considéré comme un réel problème de santé publique.
En France, 180 000 personnes sont actuellement hospitalisées pour un traumatisme crânien sévère. Généralement, un traumatisé crânien modéré nécessite une hospitalisation de 6 mois environ, et un traumatisé crânien grave de 1 an ou plus. Les coûts liés à ces hospitalisations sont colossaux. Du côté des Etats-Unis, hors personnes civiles, entre 180 000 et 320 000 soldats américains ont été diagnostiqués avec un tel traumatisme depuis 2000.
Des lésions similaires à celles provoquées par les traumatismes crâniens peuvent être également provoquées sans traumatisme. Il peut s'agir par exemple des lésions cérébrales anoxiques ou hémorragiques ou hypoglycémiques.
Ces différents événements représentent dans la plus part des cas des accidents neurologiques importants provoquant la mort ou l'incapacité.
Après un accident neurologique aigu, par exemple après traumatisme crânien sévère essentiellement, la personne cérébro-lésée a souvent droit à une indemnisation lorsqu'il existe un tiers responsable ou qu'il s'agit d'un accident du travail. Cette indemnisation repose aujourd'hui sur l'évaluation clinique des séquelles. Cependant, cette évaluation clinique reste subjective. Les troubles portant essentiellement sur les fonctions supérieures, l'analyse du handicap comporte des dimensions spécifiques, comme la capacité d'intégration sociale et professionnelle du blessé, qu'il est souvent difficile d'objectiver. A cela, peuvent s'ajouter des troubles neuropsychologiques comme l'anosognosie, c'est-à-dire la perte de conscience de ses propres troubles qui rendent l'évaluation encore plus subjective.
La prise en charge par les assurances, par exemple par l'assurance maladie et/ou les assurances complémentaires est actuellement essentiellement basée sur une évaluation clinique du patient. Toutefois, cette évaluation est aléatoire, et ne permet pas de déterminer de façon certaine l'impact du traumatisme.
Il existe donc un réel besoin d'un procédé et/ou d'une méthode permettant notamment de déterminer avec une fiabilité de 100% de l'impact d'une ou plusieurs lésions, afin, par exemple de déterminer la prise en charge et la compensation financière due par les assurances.
Il existe dans l'état de la technique des procédés de quantification de lésions cérébrale basés sur, par exemple sur l'imagerie morphologique de routine utilisant un Scanner et/ou un IRM morphologique. Par exemple, les articles suivants décrivent la valeur prédictive de l'imagerie à tenseur de diffusion pour une lésion cérébrale :

Caeyenberghs et al. (2010), Human Brain Mapping vol. 31, no. 7, pages 992-1002 ;
Wang et al. (2009), American Journal of Neuroradiology vol. 30, no. 10, pages 1907-1913 ;
Sidaros et al. (2008), Brain vol. 131, no. 2, pages 559-572.

Toutefois les procédés connus dans l'état de la technique ne permettent pas de quantifier précisément une lésion cérébrale, en particulier, ils ne permettent pas de déterminer et/ou de détecter les lésions des fibres de la matière blanche, notamment les lésions diffuses, à l'origine des principaux troubles à long terme.
En outre, les procédés connus dans l'état de la technique ne permettent pas de suivre l'évolution d'une lésion cérébrale, avec ou sans traitement du patient, en particulier une lésion des fibres de la matière blanche.
Il existe donc un réel besoin d'un procédé et/ou d'une méthode palliant ces défauts, inconvénients et obstacles de l'art antérieur, en particulier d'un procédé et/ou d'une méthode efficace(s) permettant notamment de suivre l'évolution d'une lésion des fibres de la matière blanche.

### Description de l'invention

La présente invention permet précisément de résoudre et de surmonter les obstacles et inconvénients de l'art antérieur précités en fournissant un procédé tel que défini par les revendications.

Un procédé ex-vivo de prédiction de la sortie du coma, de l'état végétatif ou de l'état de conscience minimale d'un sujet test est également décrit, ce procédé, qui ne fait pas partie de l'invention telle que définie par les revendications, comprenant les étapes suivantes :

a) Mesure de la Fraction d'Anisotropie $FA_1$ dans au moins une première région du cerveau sur une image obtenue par imagerie par Résonance Magnétique (IRM) du cerveau d'un sujet test,
b) Mesure de la Diffusibilité Axiale $DA_1$ dans au moins une deuxième région du cerveau sur une image obtenue par imagerie par Résonance Magnétique (IRM) du cerveau d'un sujet test,
c) Mesure de la Diffusibilité Radiale $DR_1$ dans au moins une troisième région du cerveau sur une image obtenue par imagerie par Résonance Magnétique (IRM) du cerveau d'un sujet test,
d) Détermination des rapports de diffusion SFAp, SDAp, SDRp par comparaison des valeurs $FA_1$, $DA_1$, $DR_1$ mesurées par rapport à des valeurs régionales normales de Fraction d'anisotropie $FA_n$, de Diffusibilité Axiale $DA_n$ et de Diffusibilité Radiale $DR_n$ d'un groupe de sujets sains de référence, pour lesdites régions selon les formules suivantes :

$$S_{FAp} = (FA_1/FA_n)$$

$$S_{DAp} = (DA_1/DA_n)$$

$$S_{DRp} = (DR_1/DR_n)$$

e) Détermination d'un algorithme F prédictif à partir de rapport de diffusion $S_{FAref}$; $S_{DAref}$ $S_{DRref}$ de régions déterminées du cerveau d'un groupe de sujets de référence comme dans les étapes a) à d) précitées et d'un logiciel de classification supervisée permettant de classer statistiquement les données $S_{FAref}$; $S_{DAref}$ $S_{DRref}$ en fonction des états de sortie du coma, de l'état végétatif ou de l'état de conscience minimale des sujets du groupe de référence ;
f) Calcul d'une valeur de prédiction sDTI du sujet test par application de l'algorithme F déterminé à l'étape e) et des valeurs $S_{FAp}$; $S_{DAp}$ $S_{DRp}$ obtenues à l'étape d) ;
g) Détermination d'au moins deux plages de valeurs de prédiction par application de l'algorithme F sur les rapports de diffusions $S_{FAref}$; $S_{DAref}$, $S_{DRref}$, une plage positive et une plage négative, la plage positive délimitant les valeurs pour lesquelles prédiction de la sortie du coma, de l'état végétatif ou de l'état de conscience minimale est favorable, la plage négative l'inverse,
h) Comparaison de la valeur de prédiction de l'étape f) avec lesdites au moins deux plages déterminées à l'étape g).

Dans la présente, par « IRM » on entend une méthode d'imagerie médicale basée sur le phénomène de la résonance magnétique, qui permet d'obtenir des images tomographiques de tissus, par exemple de tissus mous.

Dans la présente, par « image IRM » on entend toute image obtenue à partir d'un dispositif IRM, par exemple un appareil IRM 1,5 Teslas, 3,0 Teslas ou 7,0 Teslas, par exemple de la société Philips, de la société Général Electric (GE), ou de la société Siemens ou de toute autre société. Selon l'invention l'image IRM peut être toute image obtenue par un dispositif IRM, par exemple une image non pondérée, de préférence une image pondérée en diffusion.

Dans la présente par « IRM pondérée en diffusion » on entend une séquence sensible aux caractéristiques locales de la diffusion des molécules d'eau dans les tissus comme décrit dans Basser et al. 1994 [1]. Dans le cerveau, l'organisation des axones en faisceaux de fibres induit une diffusion anisotrope des molécules d'eau, plus importante dans la direction des fibres que dans le plan transversal. L'IRM du tenseur de diffusion (DTI) permet de quantifier cette anisotropie localement en mesurant la diffusion locale dans les trois directions principales ($\lambda 1$, $\lambda 2$ et $\lambda 3$) du modèle du tenseur à partir de mesures de diffusion répétées dans différentes directions de l'espace comme décrit dans Basser and Pierpaoli 1996 [2].

Les mesures permettent de mesurer :

- la diffusibilité axiale ou DA (pour « axial diffusivity ») égale à $\lambda 1$,
- la diffusibilité radiaire ou DR (pour « radial diffusivity ») égale à $(\lambda 2 + \lambda 3)/2$,
- la diffusibilité moyenne ou MD (pour « mean diffusivity ») égale à $(\lambda 1 + \lambda 2 + \lambda 3)/3$, et
- la fraction d'anisotropie ou FA (pour « fractionnal anisotropy »

$$\text{égale à } FA = sqrt(1/2) \times sqrt((\lambda 1-\lambda 2)^2+(\lambda 1-\lambda 3)^2+(\lambda 2-\lambda 3)^2) / sqrt(\lambda 1^2+\lambda 2^2+\lambda 3^2)).$$

Dans la présente par « région du cerveau » on entend une région choisie parmi le pédoncule cérébelleux moyen (ICBM #1), le tronc cérébral antérieur (ICBM #2,7,8), le tronc cérébral postérieur (ICBM #9,10,11,12,13,14) , le genou du corps calleux (ICBM #3), le tronc du corps calleux (ICBM #4), le splénium du corps calleux (ICBM #5), le pédoncule cérébral droit (ICBM #15), le pédoncule cérébral gauche (ICBM #16), le stratum sagittal droit (ICBM #21,29,31,47), le stratum sagittal gauche (ICBM #22,30,32,48), le Faisceau longitudinal supérieur droit (ICBM #41), le faisceau longitudinal supérieur gauche (ICBM #42), le bras antérieur de la capsule interne droit (ICBM #17) le bras antérieur de la capsule interne gauche (ICBM #18), le bras postérieur de la capsule interne droit (ICBM #19), la Bras postérieur de la capsule interne gauche (ICBM #20), le capsule externe droite (ICBM #33), le capsule externe gauche (ICBM #34), la couronne radiée droite (ICBM #23,25,27), la couronne radiée gauche (ICBM #24,26,28).

Selon l'invention, les mesures de Fraction d'anisotropie, Diffusibilité Axiale, Diffusibilité Radiale peuvent être effectuées indépendamment dans au moins une, au moins deux, au moins trois au moins quatre, au moins cinq, au moins six, au moins sept, au moins huit, au moins neuf, au moins dix, au moins onze, au moins douze, au moins treize, au moins quatorze, au moins quinze, au moins seize, au moins dix sept, au moins dix huit, au moins dix neuf, au moins vingt des régions du cerveau citées ci-dessus. Des mesures dans d'autres régions du cerveau peuvent être également réalisées.

Selon un mode de réalisation, les mesures de Fraction d'anisotropie, Diffusibilité Axiale, Diffusibilité Radiale peuvent être effectuées dans l'ensemble des régions précitées, à savoir le pédoncule cérébelleux moyen (ICBM #1), le tronc cérébral antérieur (ICBM #2,7,8), le tronc cérébral postérieur (ICBM #9,10,11,12,13,14), le genou du corps calleux (ICBM #3), le tronc du corps calleux (ICBM #4), le splénium du corps calleux (ICBM #5), le pédoncule cérébral droit (ICBM #15), le pédoncule cérébral gauche (ICBM #16), le stratum sagittal droit (ICBM #21,29,31,47), le stratum sagittal gauche (ICBM #22,30,32,48), le Faisceau longitudinal supérieur droit (ICBM #41), le faisceau longitudinal supérieur gauche (ICBM #42), le bras antérieur de la capsule interne droit (ICBM #17) le bras antérieur de la capsule interne gauche (ICBM #18), le bras postérieur de la capsule interne droit (ICBM #19), la Bras postérieur de la capsule interne gauche (ICBM #20), le capsule externe droite (ICBM #33), le capsule externe gauche (ICBM #34), la couronne radiée droite (ICBM #23,25,27), la couronne radiée gauche (ICBM #24,26,28).

Dans la présente, « ICBM #n » fait référence à la nième région de l'atlas de 48 régions de matière blanche construit à partir de données de diffusion de 81 sujets sains (l'atlas 'ICBM-DTI-81' (Mori et al. 2005 [9]) disponible dans le logiciel FSL (Smith et al. 2004 [7]).

Dans la présente ladite au moins une première région du cerveau, ladite au moins une seconde région du cerveau, ladite au moins une troisième région du cerveau peuvent être indépendamment identiques ou différentes.

Dans la présente, par « lésion cérébrale », on entend toute lésion susceptible d'avoir touché le cerveau, par exemple une cérébrolésion et/ou un traumatisme crânien, notamment pouvant être provoqué par un choc physique, et/ou une hémorragie, par exemple une hémorragie méningée, par exemple une hémorragie méningée anévrysmale, un hématome intracarébral spontané ou secondaire à une pathologie vasculaire et/ou un accident ischémique et/ou un accident hémorragique, par exemple un accident hémorragique intra-parenchymateux et/ou anoxie cérébrale, par exemple des suites d'un arrêt cardiaque ou circulatoire.

Selon l'invention, par « sujet test », on entend un patient ayant subi une lésion telle que précitée. Il peut s'agir par exemple de tout être vivant cérébré, par exemple d'un humain ou d'un animal, quel que soit son âge et son sexe.

Dans la présente par « groupe de sujets de référence » on entend un groupe comprenant au moins 10 sujets de référence tel que définis ci-dessus, par exemple au moins 40, au moins 60, au moins 100 sujets de référence. Il peut s'agir par exemple d'un groupe comprenant de 30 à 500 sujets, de 40 à 200, de 45 à 110 sujets de référence.

Dans la présente par « sujet sain de référence », on entend un être cérébré, par exemple un être humain ou un animal, n'ayant pas subi de choc, d'hémorragie, de problème circulatoire ou d'arrêt cardiaque, ou toute autre atteinte corporelle susceptible d'entrainer une lésion telle que définie ci-dessus. Il peut s'agir d'un sujet identique ou différent du sujet test.

Le procédé de l'invention est réalisé à partir de valeurs $FA_n$, $DA_n$ et $RD_n$ mesurées sur « un groupe de sujets sains de référence ». Par « groupe de sujets sains de référence » on entend un groupe d'êtres vivants cérébrés, par exemple un groupe d'êtres humains ou d'animaux, de toute préférence identiques par leur espèce, quel que soit l'âge et le sexe, de préférence du même âge et/ou de la même tranche d'âge, vis-à-vis du sujet test, et du même sexe que celui du sujet test.

Par « tranche d'âge », dans la présente, on entend de préférence +/- 10 ans par rapport au sujet test, de préférence +/- 9 ans, de préférence +/- 8 ans, de préférence +/- 7 ans, de préférence +/- 6 ans, de préférence +/- 5 ans, de préférence +/- 5 ans, de préférence +/- 5 ans, de préférence +/- 5 ans, de préférence +/- 4 ans, de préférence +/- 3 ans, de préférence +/- 2 ans, de préférence +/- 1 ans

Dans la présente, par « valeurs normales» on entend indépendamment les valeurs de Fraction d'Anisotropie, de Diffusion radiale et/ou de Diffusion axiale mesurées dans une région donnée chez un sujet sain de référence, ou chez un groupe de sujets sains de référence, tel que défini ci-dessus. Il peut par exemple s'agir de valeurs moyennes de référence mesurées pour une région donnée du cerveau tel que défini ci-dessus chez au moins un sujet sain de référence ou un groupe de sujet sains de référence.

Ces valeurs normales peuvent également avoir été mesurées chez le sujet test avant qu'il n'ait subi de choc, d'hémorragie, de problème circulatoire ou d'arrêt cardiaque, ou toute autre atteinte corporelle susceptible d'entrainer une lésion telle que définie ci-dessus. Il peut s'agir par exemple de mesures réalisées sur un patient à risque d'une des causes de lésion cérébrale précitées, par exemple une personne présentant une insuffisance cardiaque, des troubles du rythme cardiaque, une hypercholestérolémie, un anévrysme intracrânien asymptomatique, une épathologie vasculaire intracérébrale, par exemple une malformation artérioveineuse asymptomatique, une angiopathie amyloïde et/ou toute autre pathologie vasculaire intracérébrale et/ou une séquelle neurologique.

Le procédé de détection et/ou de quantification de lésions cérébrales décrit dans le présent document peut comprendre en outre une étape f) de détermination de l'intensité des lésions par mesure de la variation par rapport à la moyenne des valeurs de références. Il peut s'agir par exemple du calcul de l'intensité des lésions par région (Iles) selon la formule suivante :

$$I_{les}= (((FA_1-FA_{ref})x100)/FA_{ref}) + (((DA_1-DA_{ref}) \, x \, 100)/DA_{ref})+ (((DR_1-DR_{ref})x100)/DR_{ref})$$

Selon l'invention, dans le procédé ex-vivo de suivi de l'évolution d'une lésion cérébrale chez un sujet test, les mesures au temps to peuvent être réalisées sur une image IRM prise dans une période de 1 à 180 jours, par exemple suivant une lésion cérébrale, de 1 heure à 31 jours, de 1 heures à 48 heures, ou dans un délai inférieur à 31 jours.

Dans la présente, les mesures au temps $t_1$ peuvent être réalisées sur une image IRM prise dans une période d'environ 1 à plusieurs mois, par exemple de 1 à 12 mois, par exemple de 1 à 9 mois, par exemple 6 mois, de 1 à 6 mois, par exemple 3 mois, de 1 à 3 mois, d'environ un an à plusieurs années, par exemple de 1 à 30 ans, de 1 à 20 ans, de 1 à 10 ans de 1 à 5 ans, de 1 à 3 ans, de 1 à 2 ans suivant les mesures au temps to.

La présente invention trouve avantageusement une application dans le domaine médical où elle pourra être utilisée, par exemple lors d'essais cliniques afin de détecter et de quantifier des lésions du cerveau, lors de période de rémission, par exemple suite à un traumatisme cérébral de quelque nature que ce soit ou d'origine traumatique, anoxique ou hémorragique.

En outre le procédé de l'invention permet avantageusement l'obtention d'un résultat fiable, reproductible et qui peut être comparé entre patients, à différent temps. Il peut ainsi permettre, avantageusement, par exemple de comparer les capacités de récupération en fonction du traumatisme, du patient, du traitement médicamenteux ou de traitements physiques ou de tout autre traitement de rééducation entrepris.

Le procédé de la présente invention permet également avantageusement de corréler l'évolution d'une lésion avec l'évolution des séquelles et/ou l'apparition de séquelles chez un sujet test. La présente invention peut également permettre d'effectuer des correspondances entre les lésions et/ou séquelles telles que quantifiées à l'IRM avec celles évaluées cliniquement.

Par « valeur de prédiction », on entend la valeur ou score obtenu après l'application de l'algorithme F à l'étape g) du un procédé ex-vivo de prédiction de la sortie du coma, de l'état végétatif ou l'état de conscience minimale d'un sujet test.

Dans le procédé ex-vivo de prédiction de la sortie du coma, de l'état végétatif ou de l'état de conscience minimale d'un sujet test, l'étape g) de détermination d'au moins deux plages peut être réalisée via la comparaison des résultats obtenus suite à l'application de l'algorithme prédictif F et le devenir des sujets de référence correspondant. En d'autres termes les scores/résultats obtenus après application de l'algorithme prédictif F peuvent être corrélés avec le devenir du patient, en particulier sa sortie du coma, de l'état végétatif ou de l'état de conscience. Cette corrélation permet, par exemple, de déterminer des plages de valeurs de prédictions dans lesquels la probabilité de sorti ou non du coma, de l'état végétatif ou de l'état de conscience minimale est certaine et fiable à 100%

On décrit également l'utilisation d'un logiciel de classification supervisée pour la constitution d'une base de référence de diagnostic ex-vivo de prédiction de la sortie du coma, de l'état végétatif ou de l'état de conscience minimale d'un sujet test. Cette utilisation ne fait pas partie de la présente invention.

On décrit également l'utilisation d'une base de référence obtenue au moyen d'un logiciel de classification supervisée dans un procédé diagnostic ex-vivo de prédiction de la sortie du coma, de l'état végétatif ou de l'état de conscience minimale d'un sujet test. Cette utilisation ne fait pas partie de la présente invention.

Par « logiciel de classification supervisée » on entend un logiciel capable de mettre en œuvre, à partir d'une base de données de référence (ou d'apprentissage), un algorithme (ou fonction) de prédiction noté F qui, à une entrée x, associe une sortie F(x). L'algorithme d'apprentissage supervisé généralise pour de nouvelles entrées une règle de décision déterminée à partir de données connues et expertisées.

Ces logiciels de classification supervisée constituent les moyens de mise en œuvre de méthodes de classifications supervisées.

Parmi les méthodes de classification supervisée, on peut citer par exemple les réseaux de neurones, la méthode des k plus proches voisins, les arbres de décisions ou les machine à vecteurs supports. Ces méthodes sont implémentées par exemple dans le logiciel R (http://www.r-project.org/) ou le logiciel LIBSVM (http://www.csie.ntu.edu.tw/~cjlin/libsvm/).

Par « Base de référence » on entend une base de donnés constituée à partir de sujets de référence. Ces données sont obtenues à partir de mesures d'IRM en tenseur de diffusion de régions déterminées du cerveau desdits patients comme dans les étapes a) à d) précitées. Ces mesures sont introduites dans un logiciel de classification supervisée qui détermine un algorithme, linéaire ou non, permettant par la séparation des valeurs à la fois de calculer une valeur de prédiction et de définir des plages de valeurs de prédiction.

En d'autres termes, les rapports obtenus à l'étape d) du procédé à partir de plusieurs sujets tests peut constituer une base de référence susceptibles d'être utilisée dans un logiciel de classification supervisée qui détermine un algorithme, linéaire ou non, permettant par la séparation des valeurs et à la fois de calculer une valeur de prédiction et de définir des plages de valeurs de prédiction.

Ces plages de valeurs de prédiction définissent des plages de valeurs positives, indécises ou négatives telles que définies ci-dessus.

Cette base de référence a pour vocation d'être évolutive en particulier de s'enrichir avec le nombre de sujets de référence constituant ladite base.

Avantageusement lors de la mise en œuvre du procédé de prédiction de la sortie du coma, de l'état végétatif ou de l'état de conscience minimale les sujets de référence peuvent présenter des lésions cérébrales ou non et peuvent permettre de constituer ladite base de référence. Il peut s'agir par exemple d'un ensemble de sujets ou groupe de sujet de référence tel que défini ci-dessus, présentant des lésions cérébrales, et éventuellement de sujets sains afin de constituer une base de référence complète permettant de représenter tous les états possibles d'un sujet testé.

Les sujets de la base peuvent être des sujets dans le coma, dans un de l'état végétatif ou un état de conscience minimale ou non. Il peut s'agir également de sujet ayant des lésions cérébrales. Ce qui est entendu par « lésion cérébrale » est défini ci-dessus. Les lésions peuvent faire suite par exemple à un traumatisme crânien, une anoxie cérébrale ou une rupture d'anévrysme intracrânien.

Par « coma », on entend une abolition complète des fonctions de la vie de relation, sans ouverture des yeux, alors que les fonctions de la vie végétative sont conservées.

Par « état végétatif », on entend une altération du fonctionnement cérébral si profonde que les patients se réveillent du coma, c'est-à-dire qu'ils ouvrent les yeux, sans retrouver leur conscience. Ils ont perdu leurs fonctions cognitives et toute possibilité de vie relationnelle. Seules quelques fonctions vitales demeurent : cycles veille-sommeil, respiration, parfois déglutition. Cet état est dit « chronique » lorsqu'il persiste plus d'un an en cas de lésions traumatiques, ou plus de trois mois en cas de lésions anoxiques.

Par « état de conscience minimale », on entend un sujet incapable de suivre de manière consistante des instructions simples, par exemple serrer la main, mais qui démontre néanmoins un état de conscience de son environnement en répondant à certains stimuli, par exemple par des pleurs, des sourires, ou d'autres expressions émotionnelles. La réponse aux ordres est fluctuante et irrégulière.

Lors de la mise en œuvre du procédé de prédiction de la sortie du coma, de l'état végétatif ou de l'état de conscience minimale, la mesure de la fraction d'anisotropie de l'étape a) peut être effectuée dans au moins une, 2, ou 3, ou 4 ou 5 ou 6 ou 7 ou 8 ou 9 ou 10 des régions du cerveau choisie dans le groupe comprenant le pédoncule cérébelleux moyen (ICBM #1), le tronc cérébral antérieur (ICBM #2,7,8), le tronc cérébral postérieur (ICBM #9,10,11,12,13,14) , le genou du corps calleux (ICBM #3), le tronc du corps calleux (ICBM #4), le splénium du corps calleux (ICBM #5), le pédoncule cérébral droit (ICBM #15), le pédoncule cérébral gauche (ICBM #16), le stratum sagittal, le bras antérieur de la capsule interne droit (ICBM #17) le bras antérieur de la capsule interne gauche (ICBM #18).

Avantageusement lors de la mise en œuvre du procédé de prédiction de la sortie du coma, de l'état végétatif ou de l'état de conscience minimale, la mesure de la Diffusibilité Radiale de l'étapes c) peut être effectuée dans au moins une 2, ou 3, ou 4 ou 5 ou 6 ou 7 ou 8 ou 9 ou 10 ou 11 des régions du cerveau choisies dans le groupe comprenant le pédoncule cérébelleux, le tronc cérébral antérieur (ICBM #2,7,8), le tronc cérébral postérieur (ICBM #10), le genou du corps calleux (ICBM #3), le tronc du corps calleux (ICBM #4), le pédoncule cérébral droit (ICBM #15), le pédoncule cérébral gauche (ICBM #16), le bras antérieur de la capsule interne gauche (ICBM #18), le bras postérieur de la capsule interne droit (ICBM #19), le bras postérieur de la capsule interne gauche (ICBM #20).

Avantageusement lors de la mise en œuvre du procédé de prédiction de la sortie du coma, de l'état végétatif ou de l'état de conscience minimale, la mesure de la Diffusibilité Axiale de l'étapes b) peut être effectuée dans au moins une moins 2, ou 3, ou 4 ou 5 des régions du cerveau choisies dans le groupe comprenant le tronc cérébral postérieur (ICBM # 10), le genou du corps calleux (ICBM #3), le splénium du corps calleux (ICBM #5), le pédoncule cérébral gauche (ICBM #16), le bras postérieur de la capsule interne gauche (ICBM #20).

Le procédé de prédiction de la sortie du coma, de l'état végétatif ou de l'état de conscience minimale, le procédé peut comprendre en outre une étape i) de détermination de la sortie du coma, de l'état végétatif ou de l'état de conscience minimale par comparaison de la valeur de prédiction obtenue avec les plages de prédiction, la sortie du coma étant prédite si la valeur obtenue est comprise dans la plage positive, la non sortie du coma étant prédite si la valeur est comprise dans la plage négative.

On décrit également un procédé ex-vivo de prédiction de la sortie du coma, de l'état végétatif ou de l'état de conscience minimale d'un sujet test, ledit procédé ne faisant pas partie de l'invention et comprenant les étapes suivantes :

a) Mesure de la Fraction d'Anisotropie FA dans au moins une première région du cerveau sur une image obtenue par imagerie par Résonance Magnétique (IRM) du cerveau d'un sujet test,

b) Mesure d'un score selon la formule suivante :

$$scoreDTI = \frac{1}{1+e^{beta_0 + \sum_i beta_i . X_i}}$$

dans laquelle $X_i$ représente les valeurs de mesure de la Fraction d'Anisotropie FA, $beta_0$ est un nombre réel et $beta_i$ des nombres réels.

c) un scoreDTI compris entre 0 et 0,83 Indiquant une prédiction favorable de la sortie du coma, de l'état végétatif ou de l'état de conscience minimale, un scoreDTI compris de 0,83 à 1 Indiquant une prédiction défavorable de la sortie du coma, de l'état végétatif ou de l'état de conscience minimale.

Le sujet test, la région du cerveau, la mesure de Fraction d'anisotropie sont tels que définie ci-dessus.

Les valeurs $beta_0$ et $beta_i$ peuvent être des nombres réels tels que décrits dans le tableau ci-dessous :

| Régions | $beta_i$ |
|---|---|
| pédoncule cérébelleux moyen (ICBM #1) | de 0,0 à 0,0 |
| tronc cérébral antérieur (ICBM #2,7,8) | de 0,0 à 0,0 |
| tronc cérébral postérieur (ICBM #9,10,11,12,13,14) | de 0,0 à 0,0 |
| genou du corps calleux (ICBM #3) | de 6,466252753 à 7,28446365 |
| tronc du corps calleux (ICBM #4) | de 3,636447939 à 4,502330614 |
| splénium du corps calleux (ICBM #5) | de 11,42226758 à 12,288150254 |
| pédoncule cérébral droit (ICBM #15) | de -5,208057762 à -4,493633664 |
| pédoncule cérébral gauche (ICBM #16) | de 1,277114354 à 2,596620002 |
| stratum sagittal droit (ICBM #21,29,31,47) | de 3,795844311 à 4,669302645 |
| stratum sagittal gauche (ICBM #22,30,32,48) | de -3,80915557531 à -2,87934927167 |
| Faisceau longitudinal supérieur droit (ICBM #41) | de -7,26452800004 à -6,43552940029 |
| faisceau longitudinal supérieur gauche (ICBM #42) | de -5,33860234505 à -4,65103436119 |
| bras antérieur de la capsule interne droit (ICBM #17) | de 0,73250989 à 2,43884734109 |
| le bras antérieur de la capsule interne gauche (ICBM #18) | de 6,86347919816 à 7,58775407373 |
| bras postérieur de la capsule interne droit (ICBM #19) | de 1,52413579894 à 2,22785801866 |
| Bras postérieur de la capsule interne gauche (ICBM #20) | de -0,638297963914 à 0,17271458225 |

(suite)

| Régions | beta$_i$ |
|---|---|
| capsule externe droite (ICBM #33) | de 6,0227582126 à 10,560807638 |
| capsule externe gauche (ICBM #34) | de -0,378850609298 à 0,423449686126 |
| couronne radiée droite (ICBM #23,25,27) | de -0,410910494678 à 0,292109783525 |
| couronne radiée gauche (ICBM #24,26,28) | de -0,331246786435 à 7,783966523 |
| beta0 | de -21,034986402 à -24,317724924 |

Avantageusement lors de la mise en œuvre du procédé de prédiction de la sortie du coma, de l'état végétatif ou de l'état de conscience minimale, la mesure de la fraction d'anisotropie de l'étape a) peut être effectuée dans au moins 4 ou 5 ou 6 ou 7 ou 8 ou 9 ou 10 ou l'ensemble des régions du cerveau choisie dans le groupe comprenant le pédoncule cérébelleux moyen (ICBM #1), le tronc cérébral antérieur (ICBM #2,7,8), le tronc cérébral postérieur (ICBM #9,10,11,12,13,14), le genou du corps calleux (ICBM #3), le tronc du corps calleux (ICBM #4), le splénium du corps calleux (ICBM #5), le pédoncule cérébral droit (ICBM #15), le pédoncule cérébral gauche (ICBM #16), le stratum sagittal droit (ICBM #21,29,31,47), le stratum sagittal gauche (ICBM #22,30,32,48), le Faisceau longitudinal supérieur droit (ICBM #41), le faisceau longitudinal supérieur gauche (ICBM #42), le bras antérieur de la capsule interne droit (ICBM #17) le bras antérieur de la capsule interne gauche (ICBM #18), le bras postérieur de la capsule interne droit (ICBM #19), la Bras postérieur de la capsule interne gauche (ICBM #20), la capsule externe droite (ICBM #33), la capsule externe gauche (ICBM #34), la couronne radiée droite (ICBM #23,25,27), la couronne radiée gauche (ICBM #24,26,28).

Avantageusement, lors de la mise en œuvre du procédé de prédiction de la sortie du coma, de l'état végétatif ou de l'état de conscience minimale, lorsque la mesure de la fraction d'anisotropie de l'étape a) est effectuée dans au moins 4 régions du cerveau, il peut s'agir des régions suivantes le pédoncule cérébral gauche (ICBM #16), le bras antérieur de la capsule interne droit (ICBM #17) le bras antérieur de la capsule interne gauche (ICBM #18), la capsule externe droite (ICBM #33), la couronne radiée gauche (ICBM #24,26,28).

Avantageusement, lors de la mise en œuvre du procédé de prédiction de la sortie du coma, de l'état végétatif ou de l'état de conscience minimale, lorsque la mesure de la fraction d'anisotropie de l'étape a) est effectuée dans les 20 régions du cerveau mentionnées ci-dessus, les valeurs de beta i peuvent être comprise et/ou égales aux valeurs mentionnées dans le tableau ci-dessous :

| Régions | beta$_i$ | beta$_i$ de préférence |
|---|---|---|
| pédoncule cérébelleux moyen (ICBM #1) | de 0,0 à 0,0 | 0,0 |
| tronc cérébral antérieur (ICBM #2,7,8) | de 0,0 à 0,0 | 0,0 |
| tronc cérébral postérieur (ICBM #9,10,11,12,13, 14) | de 0,0 à 0,0 | 0,0 |
| genou du corps calleux (ICBM #3) | De 6,466252753 à 7,28446365 | 6,87535790908 |
| tronc du corps calleux (ICBM #4) | de 3,636447939 à 4,502330614 | 4,06938927662 |
| splénium du corps calleux (ICBM #5) | De 11,42226758 à 12,288150254 | 11,855208917 |
| pédoncule cérébral droit (ICBM #15) | De -5,208057762 à - 4,493633664 | -4,85084571328 |
| pédoncule cérébral gauche (ICBM #16) | De 1,723161668 à 2,596620002 | 2,15989083485 |
| stratum sagittal droit (ICBM #21,29,31,47) | De 3,795844311 à 4,669302645 | 4,2325734777 |
| stratum sagittal gauche (ICBM #22,30,32,48) | de -3,80915557531 à - 2,87934927167 | -3,34425242349 |
| Faisceau longitudinal supérieur droit (ICBM #41) | de -7,26452800004 à - 6,43552940029 | -6,85002870016 |
| faisceau longitudinal supérieur gauche (ICBM #42) | de -5,33860234505 à - 4,65103436119 | -4,99481835312 |
| bras antérieur de la capsule interne droit (ICBM #17) | de 1,6587492639 à 2,43884734109 | 2,0487983025 |

(suite)

| Régions | $beta_i$ | $beta_i$ de préférence |
|---|---|---|
| le bras antérieur de la capsule interne gauche (ICBM #18) | de 6,86347919816 à 7,58775407373 | 7,22561663595 |
| bras postérieur de la capsule interne droit (ICBM #19) | de 1,52413579894 à 2,22785801866 | 1,8759969088 |
| Bras postérieur de la capsule interne gauche (ICBM #20) | de -0,638297963914 à 0,17271458225 | -0,232791690832 |
| capsule externe droite (ICBM #33) | de 6,0227582126 à 6,76329905685 | 6,39302863473 |
| capsule externe gauche (ICBM #34) | de -0,378850609298 à 0,423449686126 | 0,0222995384139 |
| couronne radiée droite (ICBM #23,25,27) | de -0,410910494678 à 0,292109783525 | -0,0594003555767 |
| couronne radiée gauche (ICBM #24,26,28) | de -0,331246786435 à 0,62156911529 | 0,145161164427 |
| beta0 | De -24,725252173 à -24,317724924 | -24,5214885485 |

Les inventeurs ont démontré de manière surprenante que le procédé comprenant le calcul du scoreDTI avec la mesure de la FA dans au moins quatre régions du cerveau permet avantageusement d'obtenir un score permettant une prédiction défavorable avec une sensibilité et une spécificité de 73% et 100% respectivement.

Avantageusement, lors de la mise en œuvre du procédé de prédiction de la sortie du coma, de l'état végétatif ou de l'état de conscience minimale, lorsque la mesure de la fraction d'anisotropie de l'étape a) est effectuée dans les 4 régions du cerveau suivantes le pédoncule cérébral gauche (ICBM #16), le bras antérieur de la capsule interne droit (ICBM #17), le bras antérieur de la capsule interne gauche (ICBM #18), la capsule externe droite (ICBM #33), la couronne radiée gauche (ICBM #24,26,28), les valeurs de beta i peuvent être comprise et/ou égales aux valeurs mentionnées dans le tableau ci-dessous :

| Régions | $beta_i$ | $beta_i$ de préférence |
|---|---|---|
| pédoncule cérébral gauche (ICBM #16) | de 1,277114354 à 2,466668701 | 1,87189152758 |
| bras antérieur de la capsule interne droit (ICBM #17) | de 0,73250989 à 1,657549987 | 1,19502993841 |
| bras antérieur de la capsule interne gauche (ICBM #18) | de 1,296825232 à 2,16904842 | 1,7329368258 |
| capsule externe droite (ICBM #33) | de 9,754756364 à 10,560807638 | 10,1577820013 |
| la couronne radiée gauche (ICBM #24,26,28) | de 6,9999277456 à 7,783966523 | 7,3919471341 |
| beta0 | de -21,034986402 à -20,522064457 | -20,7785254295 |

Avantageusement, la mise en œuvre du procédé comprenant le calcul du scoreDTI avec la mesure de la FA dans quatre régions sélectionnées du cerveau permet d'augmenter la sensibilité et la spécificité du scoreDTI.

Avantageusement, le procédé comprenant le calcul du scoreDTI permet de prédire, de manière fiable, reproductible la sortie du coma, de l'état végétatif ou de l'état de conscience minimale d'un sujet. En particulier, le procédé permet d'obtenir un score permettant une prédiction avec une sensibilité et une spécificité de 80% et 100% respectivement.

Un programme d'ordinateur comprenant des instructions de code de programme pour l'exécution des étapes de procédé selon l'invention, lorsque ledit programme est exécuté sur un ordinateur, est également prévu.

La présente invention permet pour la première fois d'obtenir des résultats de mesure de lésions cérébrales objectifs et fiables, reproductibles et comparables entre patients.

Le procédé de la présente invention est également le premier permettant de déterminer, de manière fiable, reproductible et quantifiable, le pourcentage de lésions cérébrales et les impacts et évolutions cliniques et des séquelles réalistes à terme pour le patient.

La présente invention trouve avantageusement des applications dans le domaine médical où il peut être utilisé, par exemple afin de détecter et de quantifier des lésions du cerveau, notamment afin que le praticien puisse prendre les décisions cliniques et/ou pharmacologiques adéquats pour le traitement du patient.

La présente invention peut ainsi être avantageusement utilisée lors de la détermination de l'ampleur et des effets à terme de séquelles post-traumatiques et/ou dans la détermination de dédommagements financiers suite à un accident traumatique et/ou tout traumatisme cérébral intervenu par exemple lors d'un accident par exemple un accident de voiture, de deux roues, de piétons renversés par un véhicule à moteur sur la voie publique, d'accident du travail par chute notamment, et/ou lors d'un traumatisme lors de la pratique d'un sport, par exemple du rugby, boxe, catch, notamment en cas de questionnement sur la responsabilité et/ou la prise en charge des frais et dédommagements par une assurance ou tout autre procédé de dédommagement personnel ou professionnel.

Le procédé trouve avantageusement une application dans le domaine médical où il pourra être utilisé afin de prédire de manière fiable le pourcentage de prédiction de sortie du coma, de l'état végétatif ou de conscience minimale d'un sujet test. En outre, par exemple, le procédé de l'invention permettra avantageusement, de déterminer si un traitement, par exemple lors d'essais cliniques peut influencer la probabilité de sortie de coma, de l'état végétatif ou de conscience minimale d'un patient montrant ainsi les éventuels bénéfices du traitement. Le procédé de prédiction de la sortie du coma, de l'état végétatif ou de l'état de conscience minimale permet également d'éviter un éventuel acharnement thérapeutique, par exemple pour des patients test dans le coma, dans un état végétatif ou de conscience minimale dont il est certain qu'ils n'en sortiront jamais.

En outre le procédé de l'invention permet avantageusement l'obtention d'un résultat fiable, reproductible et qui peut être comparé entre patients, à différent temps, notamment par exemple en fonction de la prise d'un traitement médicamenteux ou de toute intervention de rééducation physique ou neurologique.

D'autres avantages pourront encore apparaître à l'homme du métier à la lecture des exemples ci-dessous, illustrés par les figures annexées, donnés à titre illustratif.

**Brève description des figures**

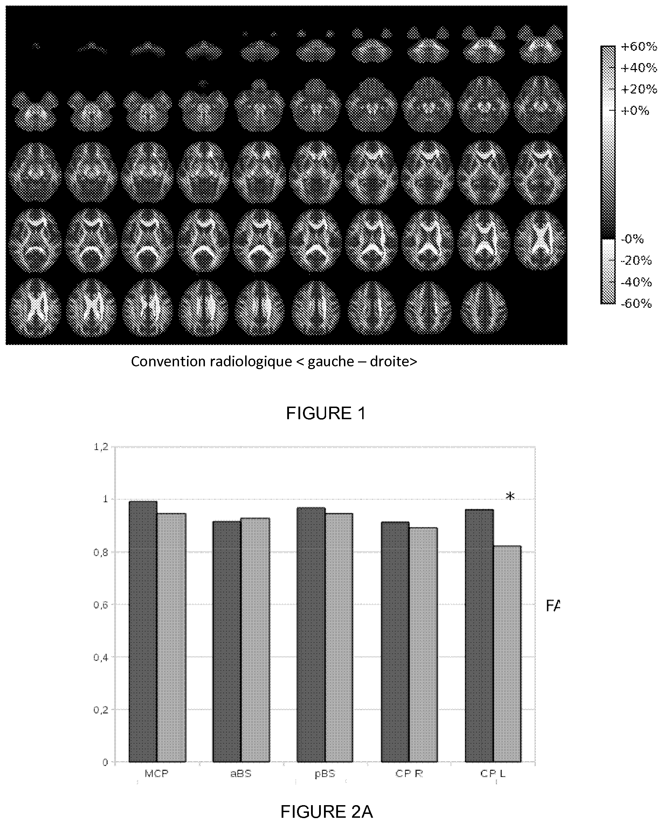

- La figure 1 est une image représentant une carte de lésion en Anisotropie Fractionnelle pour un patient. Sur cette image, les régions les plus claires correspondent aux zones lésées, c'est-à-dire pour lesquelles la mesure présente un écart à la normale significatif.
- La figure 2 A est un diagramme en bâtons représentant les valeurs moyennes des FA régionales normalisées mesurées dans les régions profondes du cerveau, à savoir le pédoncule cérébelleux moyen (ICBM #1), le tronc cérébral antérieur (ICBM #2,7,8), le tronc cérébral postérieur (ICBM #9,10,11,12,13,14), le pédoncule cérébral droit (ICBM #15) et le pédoncule cérébral gauche (ICBM #16). Les bâtons foncés représentent les valeurs obtenues chez les patients sans séquelles un an après l'accident, les bâtons clairs les valeurs obtenues chez les patients avec des séquelles neuropsychiques un an après l'accident.

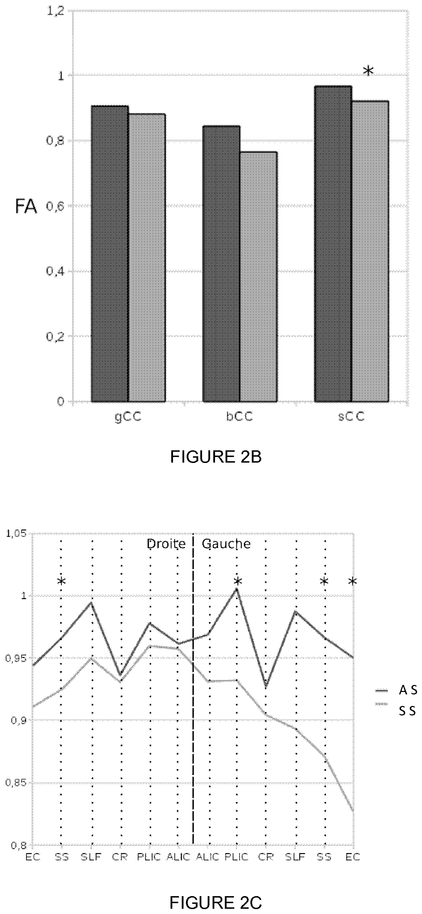

- La figure 2 B est diagramme en bâtons représentant les valeurs moyennes des FA régionales normalisées mesurées sur les régions du corps calleux, à savoir le genou du corps calleux (ICBM #3), le tronc du corps calleux (ICBM #4) et le splénium du corps calleux (ICBM #5). Les bâtons foncés représentent les valeurs obtenues chez les patients sans séquelles un an après l'accident, les bâtons clairs les valeurs obtenues chez les patients avec des séquelles neuropsychiques un an après l'accident.
- La figure 2 C est un diagramme représentant les valeurs moyennes des FA régionales normalisées mesurées sur les régions supérieures du cerveau, à savoir le stratum sagittal droit (ICBM #21,29,31,47), le stratum sagittal gauche (ICBM #22,30,32,48), le Faisceau longitudinal supérieur droit (ICBM #41), le faisceau longitudinal supérieur gauche (ICBM #42), le bras antérieur de la capsule interne droit (ICBM #17) le bras antérieur de la capsule interne gauche (ICBM #18), le bras postérieur de la capsule interne droit (ICBM #19), la Bras postérieur de la capsule interne gauche (ICBM #20), le capsule externe droite (ICBM #33), le capsule externe gauche (ICBM #34), la couronne radiée droite (ICBM #23,25,27) et la couronne radiée gauche (ICBM #24,26,28). Les courbes foncées représentent les valeurs obtenues chez les patients sans séquelles un an après l'accident (SS), les courbes claires les valeurs obtenues chez les patients avec des séquelles neuropsychiques un an après l'accident (AS).

Sur les figures 2 A à 2C les abréviations suivantes signifient : MCP : pédoncule cérébelleux moyen ; aBS : tronc cérébral antérieur ; pBS : tronc cérébral postérieur ; CP_R : pédoncule cérébral droit ; CP_L : pédoncule cérébral gauche; gCC : genou du corps calleux; bCC : tronc du corps calleux ; sCC : splénium du corps calleux ; EC: capsule externe ; SS : stratum sagittal ; SLF : faisceau longitudinal supérieur ; CR : couronne radiée ; PLIC : bras postérieur de la capsule interne ; ALIC : bras antérieur de la capsule interne.

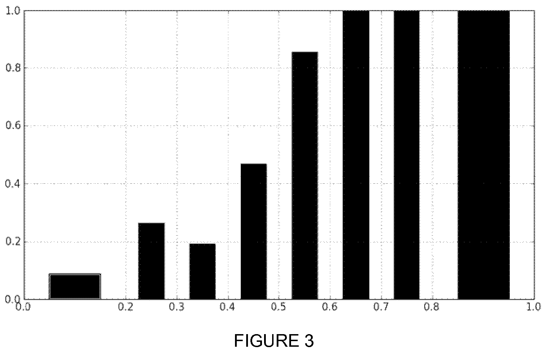

- La figure 3 est un diagramme en bâton représentant la probabilité de non sortie du coma, de l'état végétatif ou de l'état de conscience minimale en fonction de la valeur de prédiction. Sur ce diagramme, une valeur égale à 1 indiquant une certitude de non sortie du coma de l'état végétatif ou de l'état de conscience minimale, une valeur égale à 0 indiquant une certitude de sortie du coma de l'état végétatif ou de l'état de conscience minimale.

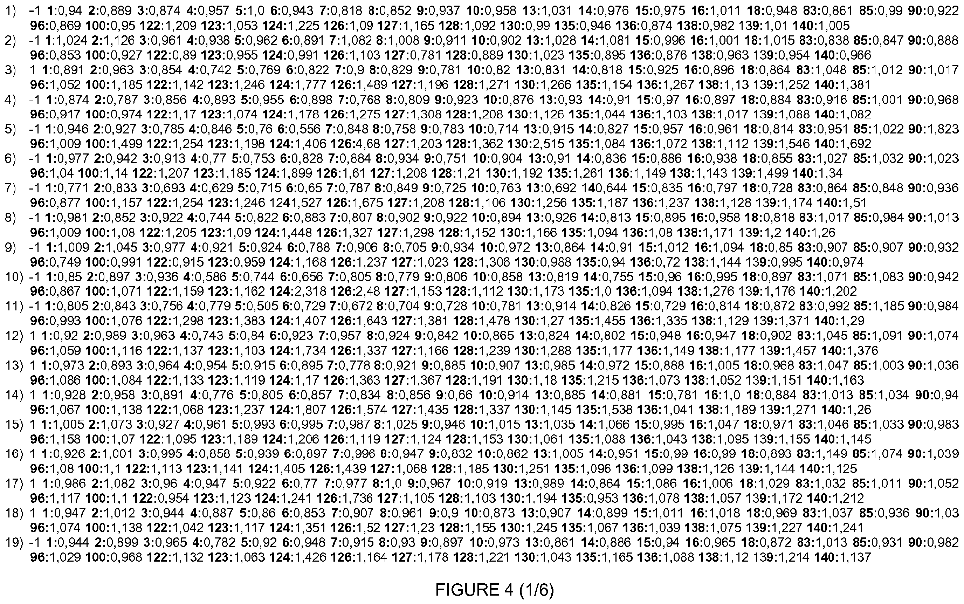

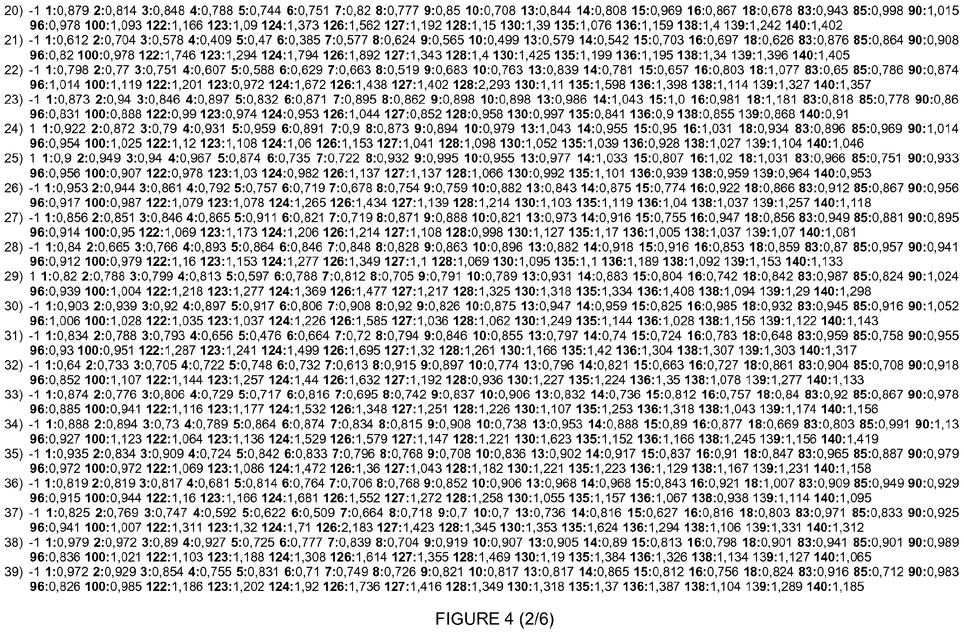

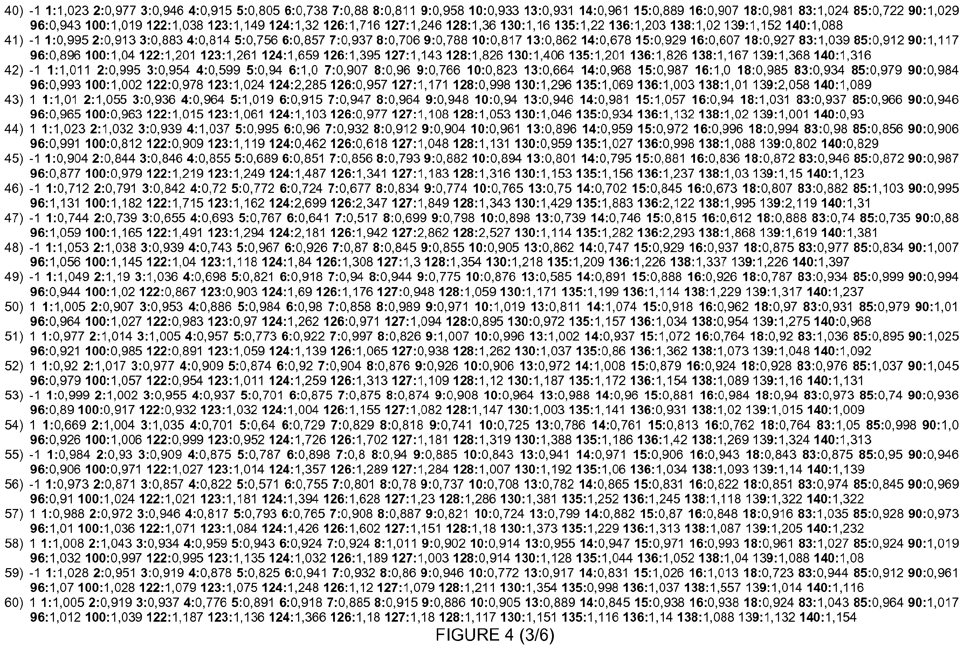

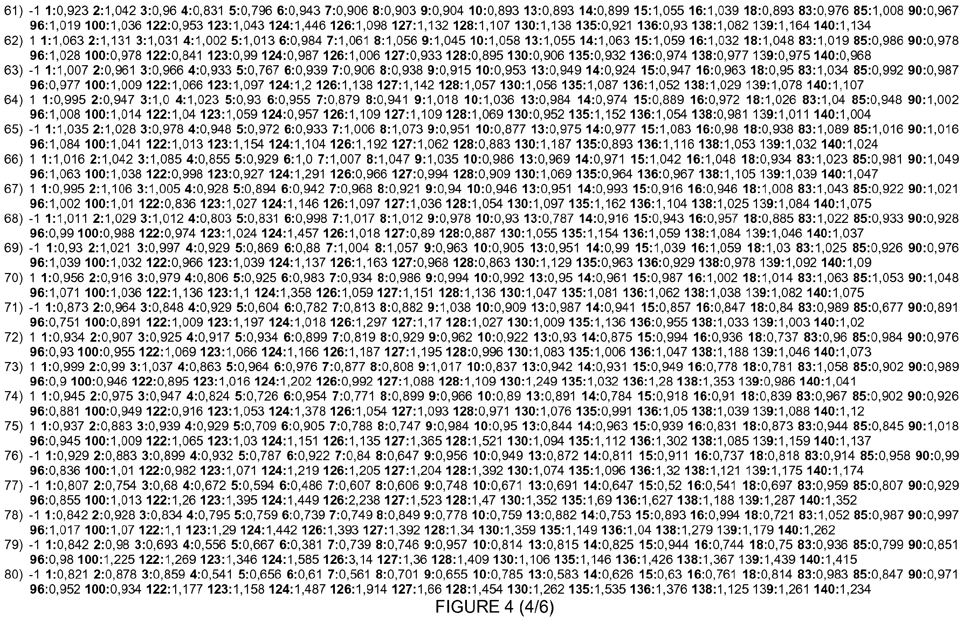

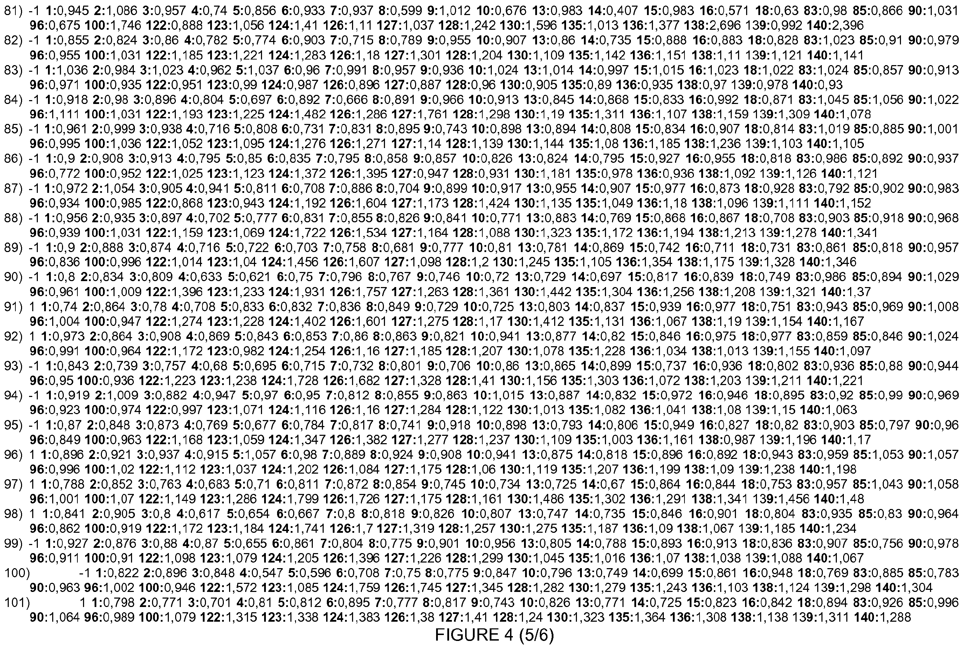

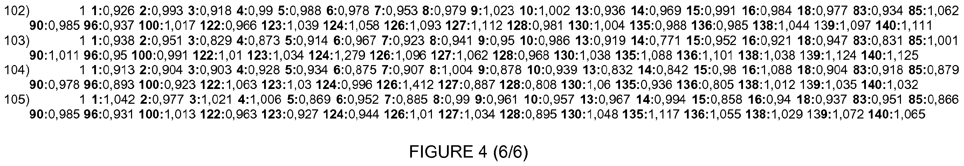

- La figure 4 représente les valeurs obtenues pour les 32 variables utilisées par l'algorithme F du logicielle de classification supervisée pour les 105 patients de la base de référence. Sur cette figure, les chiffres en gras indiquent le numéro des variables tel que définies dans le tableau 6.

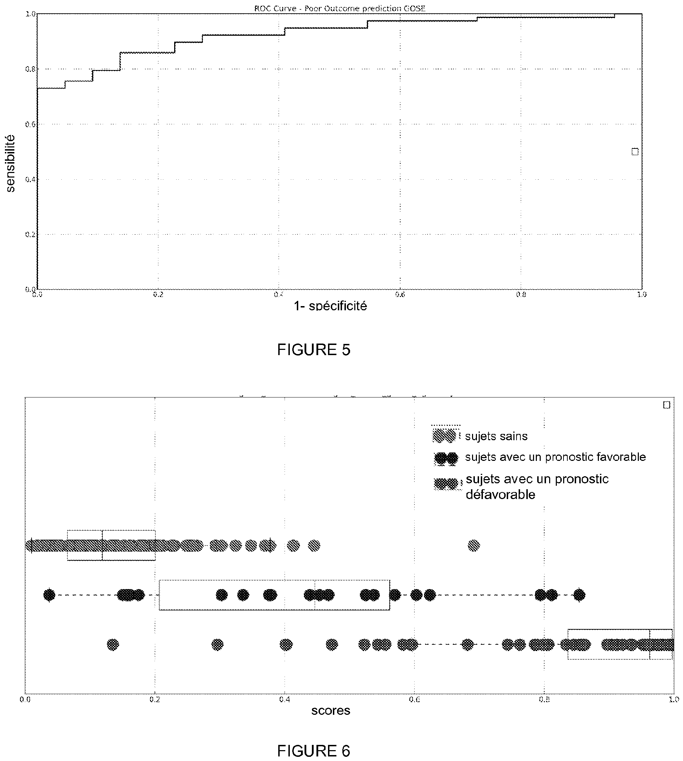

- La figure 5 représente une courbe ROC pour la prédiction du pronostic défavorable des patients après arrêt cardiaque avec le scoreDTI calculé à partir des 20 régions, l'ordonné représente la sensibilité, l'abscisse 1-spécificité.

- La figure 6 représente une distribution du scoreDTI calculé à partir des 20 régions pour les patients arrêt cardiaque de pronostic favorable (points au milieux/ligne du milieu) et défavorable (points inférieurs, ligne inférieure) et le groupe de 105 sujets sains (points supérieurs, ligne supérieure). Le scoreDTI compact pour les patients est obtenu par validation croisée (« leave-one-out »), celui pour les contrôles a été obtenu par le calcul direct suivant la formule 1 et les paramètres du tableau 8

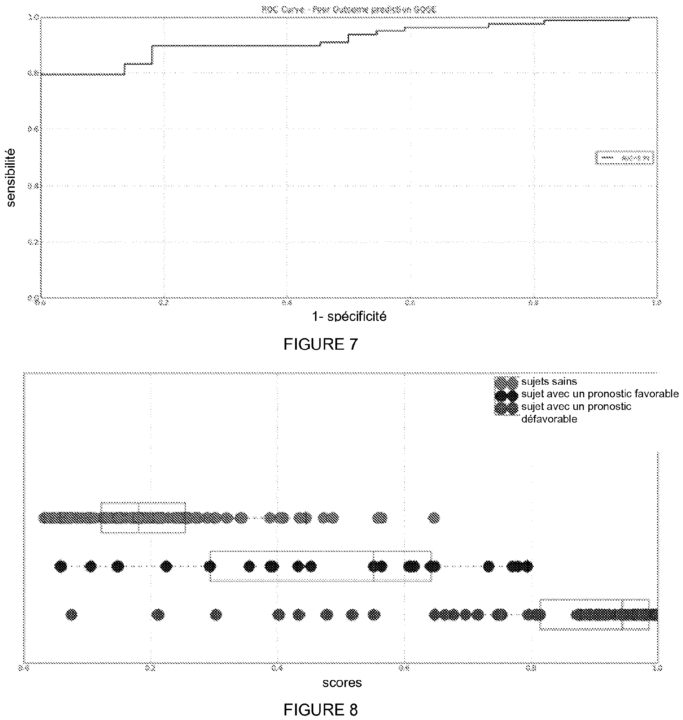

- La figure 7 représente une courbe ROC pour la prédiction du pronostic défavorable des patients après arrêt cardiaque, l'ordonné représente la sensibilité, l'abscisse 1-spécificité. Le scoreDTI calculé à partir des cinq régions sélectionnées (le pédoncule cérébral gauche (ICBM #16), le bras antérieur de la capsule interne droit (ICBM #17), le bras antérieur de la capsule interne gauche (ICBM #18), la capsule externe droite (ICBM #33), la couronne radiée gauche (ICBM #24,26,28)) pour les patients a été obtenu par validation croisée (« leave-one-out »).

La figure 8 représente distribution du scoreDTI calculé à partir des cinq régions sélectionnées (le pédoncule cérébral gauche (ICBM #16), le bras antérieur de la capsule interne droit (ICBM #17), le bras antérieur de la capsule interne gauche (ICBM #18), la capsule externe droite (ICBM #33), la couronne radiée gauche (ICBM #24,26,28)) pour les patients arrêt cardiaque de pronostic favorable (points intermédiaires, ligne du milieu) et défavorable (points inférieurs, ligne inférieure) et le groupe de 105 sujets sains (points supérieurs, ligne supérieure).

Les procédés des exemples 1 et 3 ci-dessous ne font pas partie de l'invention revendiquée en tant que telle.

## Exemples

### Exemple 1 : mise en œuvre du procédé de quantification de lésions cérébrales.

Le procédé de quantification de lésions cérébrales, en particulier la quantification régionale des lésions des fibres de matière blanche du cerveau repose sur les mesures d'anisotropie fractionnelle (FA) reflétant l'intégrité globale des fibres, de diffusibilité axiale (AD) reflétant l'intégrité axonale et de diffusibilité radiaire (DR) reflétant l'intégrité de la gaine de myéline.

Pour se faire, il se compose de plusieurs étapes successives:

1. Une acquisition IRM du tenseur de diffusion (DTI) comportant une acquisition pondérée en T2 (correspondant à un facteur b=0) et des acquisitions avec des gradients de pondération en diffusion (b ~=1000 s/mm$^2$). Pour appliquer le modèle du tenseur, l'acquisition avec des gradients dans au moins 6 directions différentes de l'espace est nécessaire.

2. Une série de prétraitement des données DTI brutes a été effectuée à l'aide du logiciel FSL (http://www.fmrib.ox.ac.uk/fsl/, Smith et al. 2004 [7]).

3. Correction des distorsions induites par les courants de Foucault (avec la fonction « eddycorrect »). Cette correction a consisté à recaler (recalage rigide) les volumes pondérés en diffusion sur le volume pondéré en T2 comme décrit dans Jenkinson et al. 2002 [4].

4. Extraction du masque du cerveau en retirant du volume tous les tissus hors cerveau (avec la fonction bet) comme décrit dans Smith 2002 [6].

5. Calcul des 3 valeurs propres ($\lambda 1$, $\lambda 2$ et $\lambda 3$) du modèle du tenseur pour chaque voxel comme décrit dans Basser et al. 1996 [2] permettant le calcul des cartes paramétrique de FA, AD et DR (avec la fonction dtifit).

De manière à comparer les cartes paramétriques aux cartes de référence (calculées sur des sujets contrôles sains et sur des groupes de patients expertisés), celles-ci ont été projetées dans un espace standard.

Pour cela, les cartes de FA individuelles ont été tout d'abord recalées par un recalage non-linéaire FNIRT (« FMRIB's Non-linear Image Registration Tool ») Andersson et al. 2007a [10], Andersson et al. 2007b [11] dans un espace de référence caractérisé par une image de référence calculée sur 58 sujets sains (FMRIB58_FA). Pour ne tenir compte que des valeurs maximales de FA le long des faisceaux, ces valeurs locales maximales ont été projetées sur le squelette des principaux faisceaux de FA suivant la méthode TBSS décrite dans Smith et al. 2006 [8]. Ce squelette représente les centres communs au groupe des principaux faisceaux de matière blanche dans le cerveau. Les valeurs correspondantes de AD et DR ont été projetées sur ce même squelette suivant la même transformation.

Par ailleurs, 20 régions d'intérêt (ROIs) ont été définies sur la base de l'atlas de 48 régions de matière blanche construit à partir de données de diffusion de 81 sujets sains (l'atlas 'ICBM-DTI-81' disponible dans le logiciel fsl). Ces 20 ROIs ont été choisis par un collège d'experts (2 neuroradiologues et 1 neuroréanimateur) en tenant compte de leur taille, les petites ROIs d'origines ont été éliminées ou fusionnées, et de leur potentiel intérêt diagnostic. Ces 20 régions d'intérêts sont représentées sur la figure 2, elles sont indiquées par un numéro de 1 à 20 en fonction de la coloration de l'image en corrélation avec l'échelle de dégradé. Il s'agit du Pédoncule cérébelleux moyen indiqué 1 (ICBM #1), du tronc cérébral antérieur indiqué 2 (ICBM #2,7,8), du tronc cérébral postérieur indiqué 3 (ICBM #9,10,11,12,13,14), du genou du corps calleux indiqué 4 (ICBM #3), du tronc du corps calleux indiqué ( (ICBM #4), du splénium du corps calleux indiqué 6 (ICBM #5), du pédoncule cérébral droit indiqué 7(ICBM #15), du pédoncule cérébral gauche indiqué 8 (ICBM #16), du stratum sagittal droit indiqué 9 (ICBM #21,29,31,47), du stratum sagittal gauche indiqué 10 (ICBM #22,30,32,48), du faisceau longitudinal supérieur droit indiqué 11 (ICBM #41), du faisceau longitudinal supérieur gauche indiqué 12 (ICBM #42), du bras antérieur de la capsule interne droit indiqué 13 (ICBM #17), du bras antérieur de la capsule interne gauche indiqué 14 (ICBM #18), du bras postérieur de la capsule interne droit indiqué 15 (ICBM #19), du bras postérieur de la capsule interne gauche indiqué 16 (ICBM #20), de la capsule externe droite indiqué 17 (ICBM #33), de la capsule externe gauche indiqué 18 (ICBM #34), de la couronne radiée droite indiqué 19 (ICBM #23,25,27) et de la couronne radiée gauche indiqué 20 (ICBM #24,26,28).

Les 20 paramètres régionaux de FA de chaque patient sont les moyennes dans chaque ROI de la FA sur le squelette. Les 20 paramètres de MD, AD et RD ont été calculés de la même manière.

Chaque patient a donc été caractérisé par 20 paramètres de FA (moyenne de la FA sur le squelette dans chaque ROI), 20 paramètres de AD et 20 paramètres de RD reflétant l'intégrité régionale des faisceaux de matière blanche. Ces paramètres ont été extraits par masquage des cartes de FA projetées sur le squelette avec le masque des 20 ROIs.

Pour que ces paramètres puissent être interprétables par rapport à un niveau normal de référence, la valeur de FA mesurée dans chaque ROI a été normalisée par rapport à une valeur moyenne calculée sur une population de sujets sains de même âge, à savoir au moins 10 individus, à partir de la même machine et des mêmes protocoles d'acquisition d'IRM.

Pour chacune des ROIs, on a donc calculé donc les scores suivants :

$$S\ FA\_n(ROI\#i) = FA(ROI\#i)/mean\_controls(FA(ROI\#i))$$

$$S\ MD\_n(ROI\#i) = MD(ROI\#i)/mean\_controls(MDROI\#i))$$

$$S\ AD\_n(ROI\#i) = AD(ROI\#i)/mean\_controls(AD(ROI\#i))$$

$$S\ RD\_n(ROI\#i) = RD(ROI\#i)/mean\_controls(RD(ROI\#i))$$

Dans lesquels "i" correspond au numéro de la région et "mean_controls" correspond à la valeur normale pour le paramètre mesuré.

Une région cérébrale présentant une variation significative d'un de ces scores par rapport à la normale, c'est-à-dire plus ou moins 2 fois l'écart-type des mesures régionales du groupe de contrôles tel que indiqué dans le tableau 3 suivant, a été considérée comme lésée. L'intensité des lésions est exprimée en pourcentage de variation par rapport à la moyenne des mesures du groupe de contrôles. La figure 1 représente le pourcentage en fonction des valeurs contrôles.

Les écarts-type régionaux des contrôles (n=85) utilisés pour détecter les variations significatives sont présentés dans le tableau 3.

**Tableau 3 : écarts-type régionaux**

| | Ecart-type FA_n (ROI) en % | Ecart-type MD_n (ROI) en % | Ecart-type AD_n (ROI) en % | Ecart-type RD_n (ROI) en % |
|---|---|---|---|---|
| pédoncule cérébelleux moyen (ICBM #1) | 4,5 | 4,1 | 3,8 | 6,2 |
| tronc cérébral antérieur (ICBM #2,7,8) | 4,4 | 5,3 | 4,6 | 6,9 |
| tronc cérébral postérieur (ICBM #9,10,11,12,13,14) | 3,9 | 3,7 | 3,0 | 6,2 |
| genou du corps calleux (ICBM #3) | 4,7 | 4,9 | 3,4 | 11,5 |
| tronc du corps calleux (ICBM #4) | 8,0 | 6,8 | 3,8 | 13,7 |
| splénium du corps calleux (ICBM #5) | 2,8 | 3,7 | 3,2 | 8,9 |
| pédoncule cérébral droit (ICBM #15) | 3,6 | 3,2 | 3,0 | 6,4 |
| pédoncule cérébral gauche (ICBM #16) | 3,8 | 4,1 | 3,3 | 8,3 |
| stratum sagittal droit (ICBM #21,29,31,47) | 4,7 | 3,4 | 2,9 | 6,0 |
| stratum sagittal gauche (ICBM #22,30,32,48) | 4,6 | 3,2 | 2,7 | 6,1 |
| Faisceau longitudinal supérieur droit (ICBM #41) | 5,2 | 3,1 | 3,1 | 5,1 |
| faisceau longitudinal supérieur gauche (ICBM #42) | 5,6 | 3,1 | 3,1 | 5,4 |
| bras antérieur de la capsule interne droit (ICBM #17) | 4,5 | 3,0 | 3,1 | 5,7 |
| bras antérieur de la capsule interne gauche (ICBM #18) | 4,3 | 2,7 | 2,6 | 5,4 |
| bras postérieur de la capsule interne droit (ICBM #19) | 3,5 | 2,6 | 3,0 | 5,6 |
| Bras postérieur de la capsule interne gauche (ICBM #20) | 3,2 | 2,3 | 3,0 | 4,9 |
| capsule externe droite (ICBM #33) | 4,9 | 2,9 | 2,5 | 4,3 |
| capsule externe gauche (ICBM #34) | 5,2 | 2,2 | 2,2 | 3,9 |
| couronne radiée droite (ICBM #23,25,27) | 4,9 | 3,3 | 3,0 | 5,1 |
| couronne radiée gauche (ICBM #24,26,28) | 5,0 | 3,0 | 2,7 | 5,1 |

41 patients dans le coma après traumatisme crânien ont été admis dans le service de neuro-réanimation de la Pitié-Salpêtrière. Ils ont été inclus dans l'étude s'ils remplissaient les critères suivants :

1) nécessité d'une respiration mécaniquement assistée pour des raisons neurologiques,
2) absence de réponse aux ordres simples au moment de la signature du consentement légal par le représentant autorisé, au moins sept jours après l'accident,
3) absence de réponse aux ordres simples non liée à l'administration de sédatifs,

4) état clinique général permettant le transport du patient,

5) Pression intracranienne et compliance cérébrale (en anglais « cerebral compliance ») permettant le maintien de la position allongée pour l'acquisition IRM sans développement d'une hypertension intracrânienne potentiellement dommageable au patient.

15 patients ayant bien récupérés ont été évalués à la phase de consolidation, environ 2 ans après l'accident, par le procédé de l'invention comprenant l'acquisition IRM de diffusion et quantification régionale des lésions. Parallèlement, les séquelles neuropsychologiques ont été évaluées par un expert. A partir de cette expertise, ces 15 patients ont été classés en deux groupes : les patients normaux ne présentant aucune séquelle (n=10) et ceux présentant des séquelles (n=5). Enfin, une acquisition IRM a été effectuée sur la même machine chez 15 sujets contrôles pour permettre la normalisation des mesures de diffusion.

Les détails des acquisitions IRM sont les suivants : séquence pondérée en diffusion, 11 directions, TR/TE =13,000 / 85,9 ms, diffusion b value= 900s/mm, épaisseur de coupe = 3 mm sans trou, 47 coupes, champ de vue = 28 $\times$ 28 cm$^2$, matrice 256 $\times$ 256.

20 valeurs de FA régionales ont été extraites puis normalisées par rapport aux valeurs contrôles, conformément au procédé décrit précédemment. Les valeurs moyennes de ces mesures sur les deux groupes (groupe sans séquelle et groupe avec séquelles neurologiques) sont représentées Figure 2.

Tel que représenté sur la figure 2 les valeurs obtenues entre les patients avec et sans séquelles sont significativement différentes. En outre, le procédé de l'invention permet de définir deux groupes distincts, un avec et l'autre sans séquelles. Ainsi, le procédé de l'invention permet avantageusement de détecter une lésion, de quantifier la lésion et de lier cette lésion avec une éventuelle séquelle

**Exemple 2 : détermination de l'algorithme pour le suivi d'une lésion cérébrale chez un sujet test et mise en œuvre.**

Le procédé de suivi des lésions cérébrales chez un patient, en particulier la quantification régionale des lésions des fibres de matière blanche du cerveau repose sur les mesures d'anisotropie fractionnelle (FA) reflétant l'intégrité globale des fibres, de diffusibilité axiale (AD) reflétant l'intégrité axonale et de diffusibilité radiaire (DR) reflétant l'intégrité de la gaine de myéline.

Pour se faire, il s'agit de quantifier les lésions cérébrales d'un patient suivant le même procédé que celui décrit dans l'exemple 1 à plusieurs instants, par exemple tous les six mois et/ou tous les ans.

Pour un instant donné, la quantification des lésions cérébrales se compose de plusieurs étapes successives:

1. Une acquisition IRM du tenseur de diffusion (DTI) comportant une acquisition pondérée en T2 (correspondant à un facteur b=0) et des acquisitions avec des gradients de pondération en diffusion (b $\sim$=1000 s/mm$^2$). Pour appliquer le modèle du tenseur, l'acquisition avec des gradients dans au moins 6 directions différentes de l'espace est nécessaire.

2. Une série de prétraitement des données DTI brutes a été effectuée à l'aide du logiciel FSL (http://www.fmrib.ox.ac.uk/fsl/, Smith et al. 2004 [7]).

3. correction des distorsions induites par les courants de Foucault (avec la fonction « eddycorrect »). Cette correction a consisté à recaler (recalage rigide) les volumes pondérés en diffusion sur le volume pondéré en T2 comme décrit dans Jenkinson et al. 2002 [4].

4. extraction du masque du cerveau en retirant du volume tous les tissus hors cerveau (avec la fonction bet) comme décrit dans Smith 2002 [6].

5. Calcul des 3 valeurs propres ($\lambda 1$, $\lambda 2$ et $\lambda 3$) du modèle du tenseur pour chaque voxel comme décrit dans Basser et al. 1996 [2] permettant le calcul des cartes paramétrique de FA, AD et DR (avec la fonction dtifit).

De manière à faire correspondre spatialement les cartes paramétriques aux cartes de référence, c'est-à-dire calculées sur des sujets contrôles sains et sur des groupes de patients, pour pouvoir les comparer celles-ci ont été projetées dans un espace standard.

Pour cela, les cartes de FA individuelles ont été tout d'abord recalées par un recalage non-linéaire FNIRT (« FMRIB's Non-linear Image Registration Tool ») Andersson et al. 2007a [10], Andersson et al. 2007b [11] dans un espace de référence caractérisé par une image de référence calculée sur 58 sujets sains (FMRIB58_FA). Pour ne tenir compte que des valeurs maximales de FA le long des faisceaux, ces valeurs locales maximales ont été projetées sur le squelette des principaux faisceaux de FA suivant la méthode TBSS décrite dans Smith et al. 2006 [8]. Ce squelette représente les centres communs au groupe des principaux faisceaux de matière blanche dans le cerveau. Les valeurs correspondantes de AD et DR ont été projetées sur ce même squelette suivant la même transformation.

Par ailleurs, 20 régions d'intérêt (ROIs) ont été définies sur la base de l'atlas de 48 régions de matière blanche construit à partir de données de diffusion de 81 sujets sains (l'atlas 'ICBM-DTI-81' disponible dans le logiciel fsl). Ces 20 ROIs ont été choisis par un collège d'experts (2 neuroradiologues et 1 neuroréanimateur) en tenant compte de leur taille, les petites ROIs d'origines ont été éliminées ou fusionnées, et de leur potentiel intérêt diagnostic. Ces 20 régions d'intérêts sont représentées sur la figure 2, elles sont indiquées par un numéro de 1 à 20 en fonction de la coloration de l'image en corrélation avec l'échelle de dégradé. Il s'agit du Pédoncule cérébelleux moyen indiqué 1 (ICBM #1), du tronc cérébral antérieur indiqué 2 (ICBM #2,7,8), du tronc cérébral postérieur indiqué 3 (ICBM #9,10,11,12,13,14), du genou du corps calleux indiqué 4 (ICBM #3), du tronc du corps calleux indiqué ( (ICBM #4), du splénium du corps calleux indiqué 6 (ICBM #5), du pédoncule cérébral droit indiqué 7(ICBM #15), du pédoncule cérébral gauche indiqué 8 (ICBM #16), du stratum sagittal droit indiqué 9 (ICBM #21,29,31,47), du stratum sagittal gauche indiqué 10 (ICBM #22,30,32,48), du faisceau longitudinal supérieur droit indiqué 11 (ICBM #41), du faisceau longitudinal supérieur gauche indiqué 12 (ICBM #42), du bras antérieur de la capsule interne droit indiqué 13 (ICBM #17), du bras antérieur de la capsule interne gauche indiqué 14 (ICBM #18), du bras postérieur de la capsule interne droit indiqué 15 (ICBM #19), du bras postérieur de la capsule interne gauche indiqué 16 (ICBM #20), de la capsule externe droite indiqué 17 (ICBM #33), de la capsule externe gauche indiqué 18 (ICBM #34), de la couronne radiée droite indiqué 19 (ICBM #23,25,27) et de la couronne radiée gauche indiqué 20 (ICBM #24,26,28).

Les 20 paramètres régionaux de FA de chaque patient sont les moyennes dans chaque ROI de la FA sur le squelette. Les 20 paramètres de MD, AD et DR ont été calculés de la même manière.

Pour un instant donné, un patient a donc été caractérisé par 20 paramètres de FA (moyenne de la FA sur le squelette dans chaque ROI), 20 paramètres de AD et 20 paramètres de DR reflétant l'intégrité régionale des faisceaux de matière blanche. Ces paramètres ont été extraits par masquage des cartes de FA projetées sur le squelette avec le masque des 20 ROIs.

Pour que ces paramètres puissent être interprétables par rapport à un niveau normal de référence, la valeur de FA mesurée dans chaque ROI a été normalisée par rapport à une valeur moyenne calculée sur une population de sujets sains de même âge, à savoir au moins 10 individus, à partir de la même machine et des mêmes protocoles d'acquisition d'IRM.

Pour un instant donné $T_j$, pour chacune des ROIs, on a donc calculé donc les scores suivants :

$$S\ FA\_n\_Tj(ROI\#i) = FA\_Tj(ROI\#i)/mean\_controls(FA(ROI\#i))$$

$$S\ MD\_n\_Tj(ROI\#i) = MD\_Tj(ROI\#i)/mean\_controls(MDROI\#i))$$

$$S\ AD\_n\_Tj(ROI\#i) = AD\_Tj(ROI\#i)/mean\_controls(AD(ROI\#i))$$

$$S\ DR\_n\_Tj(ROI\#i) = DR\_Tj(ROI\#i)/mean\_controls(RD(ROI\#i))$$

Dans lesquels "i" correspond au numéro de la région et "mean_controls" correspond à la valeur normale pour le paramètre mesuré.

Finalement, l'évolution des lésions cérébrales entre deux instant T1 et T2 est déterminée en calculant de la variation $\Delta S_{FA}$, $\Delta S_{DA}$, $\Delta S_{DR}$ selon les formules suivantes :

$$\Delta S_{FA} = S_{FA\_n\_T2} - S_{FA\_n\_T1}$$

$$\Delta S_{DA} = S_{DA\_n\_T2} - S_{DA\_n\_T1}$$

$$\Delta S_{DR} = S_{DR\_n\_T2} - S_{DR\_n\_T1}$$

Une variation d'un de ces scores d'au moins 2 fois l'écart-type des mesures régionales du groupe de contrôles tel qu'indiqué dans le tableau 3, a été considéré comme significative.

Une variation négative significative d'au moins une valeur $\Delta S_{FA}$, $\Delta S_{DA}$, indiquant une aggravation de la lésion, une variation positive significative d'au moins une valeur $\Delta S_{FA}$, $\Delta S_{DA}$, indiquant une récupération, une variation négative significative de $\Delta S_{DR}$ indiquant une récupération, une variation positive significative de $\Delta S_{DR}$ indiquant une aggravation de la lésion.

Prenons l'exemple d'un patient ayant subi un trauma crânien sévère dont l'évolution des lésions a été suivie entre un an après l'accident (T1) et trois ans après l'accident (T2). Ce patient a bien récupéré et n'a pas de séquelles. Pour ce patient, les variations $\Delta S_{FA}$, $\Delta S_{DA}$, $\Delta S_{DR}$ ont été calculées entre T1 et T2 telles que décrit précédemment.

Les résultats des mesures sont représentés dans le tableau 4 ci-dessous :

**Tableau 4 : résultats obtenus par région. Les différences significatives sont indiquées par \***

| Mesures | Temps T1 | | | Temps T2 | | | Variations (*100) | | |
|---|---|---|---|---|---|---|---|---|---|
| | FA | DA | DR | FA | DA | DR | ΔSFA | ΔSDA | ΔSDR |
| pédoncule cérébelleux moyen (ICBM#1) | 1,008 | 1,111 | 1,091 | 0,987 | 1,100 | 1,100 | -2,10 | -1,04% | 0,84% |
| tronc cérébral antérieur (ICBM #2,7,8) | 0,879 | 1,120 | 1,262 | 0,886 | 1,098 | 1,219 | 0,70 | -2,25 | -4,32 |
| tronc cérébral postérieur (ICBM #9,10,11,12,13,14) | 0,968 | 1,020 | 1,060 | 0,971 | 1,033 | 1,092 | -1,70 | 1,29 | 3,23 |
| genou du corps calleux (ICBM #3) | 0,814 | 1,053 | 1,517 | 0,823 | 1,080 | 1,570 | 0,90 | 2,69 | 5,31 |
| tronc du corps calleux (ICBM #4) | 0,791 | 1,013 | 1,427 | 0,863 | 1,050 | 1,345 | 7.20 | 3,62 | -8,19 |
| splénium du corps calleux (ICBM #5) | 0,931 | 1,030 | 1,253 | 0,950 | 1,034 | 1,185 | 1,90 | 0,37 | -6,79 |
| pédoncule cérébral droit (ICBM #15) | 0,947 | 1,012 | 1,115 | 0,935 | 1,011 | 1,142 | -1,20 | -0,15 | 2,75 |
| pédoncule cérébral gauche (ICBM #16) | 0,964 | 1,026 | 1,092 | 0,943 | 1,007 | 1,118 | -2,10 | -1,85 | 2,66 |
| stratum sagittal droit (ICBM #21,29,31,47) | 1,004 | 1,039 | 1,039 | 1,018 | 1,040 | 1,020 | 1,40 | 0,14 | -1,95 |
| stratum sagittal gauche (ICBM #22,30,32,48) | 0,967 | 1.035 | 1,082 | 1,005 | 1.029 | 1.022 | 3,80 | -0,5B | -6,00 |
| Faisceau longitudinal supérieur droit (ICBM #41) | 1,063 | 1,058 | 0,987 | 1,043 | 1,033 | 0,991 | -2,00 | -2,58 | 0,47 |
| faisceau longitudinal supérieur gauche (ICBM #42) | 1,040 | 1,042 | 0,983 | 1,099 | 1,060 | 0,944 | 5,90 * | 1,84 | -3,92 |
| bras antérieur de la capsule interne droit (ICBM #17) | 0,974 | 1,022 | 1,064 | 1,019 | 1,025 | 1,013 | 4,50 * | 0,34 | -5,03 |
| bras antérieur de la capsule interne gauche (ICBM #18) | 0,987 | 1,061 | 1,063 | 0,972 | 1,051 | 1,068 | -1,50 | -0,95 | 0,54 |
| bras postérieur de la capsule interne droit (ICBM #19) | 0,994 | 1,049 | 1,050 | 1,019 | 1,009 | 0,967 | 2,50 | -4,01 | -8,33* |
| Bras postérieur de la capsule interne gauche (ICBM #20) | 1,009 | 1,054 | 1,023 | 1,012 | 1,038 | 1,005 | 0,30 | -1,68 | -1,82 |
| capsule externe droite (ICBM #33) | 0,942 | 1,032 | 1,082 | 0,950 | 1,019 | 1,072 | 0,80 | -1,31 | -1,06 |
| capsule externe gauche (ICBM #34) | 0,927 | 1,003 | 1,076 | 0,935 | 0,998 | 1,066 | 0,80 | -0,46 | -0,97 |

(suite)

| Mesures | Temps T1 | | | Temps T2 | | | Variations (*100) | | |
|---|---|---|---|---|---|---|---|---|---|
| | FA | DA | DR | FA | DA | DR | $\triangle$SFA | $\triangle$SDA | $\triangle$SDR |
| couronne radiée droite (ICBM #23,25,27) | 0,949 | 1,112 | 1,169 | 0,934 | 1,134 | 1,232 | -1,50 | 2,18 | 6,32 |
| couronne radiée gauche (ICBM #24,26,28) | 0,893 | 1,047 | 1,149 | 0,924 | 1,047 | 1,122 | 3,10 | 0,00 | -2,78 |

Tels que démontré dans le tableau 4 ci-dessus les variations significatives montrent une récupération des lésions du faisceau longitudinal supérieur gauche ($\triangle S_{FA}$=+5,9%), du bras antérieur de la capsule interne droit ($\triangle S_{FA}$=+4,5%) et du bras postérieur de la capsule interne droit ($\triangle S_{DR}$=-8,3%).

Le procédé de la présente invention permet donc un suivi de l'évolution d'une lésion chez un sujet test et permet avantageusement d'identifier les régions dans lesquels la lésion ou les lésions ont évoluée.

Le procédé de l'exemple 3 ci-dessous ne fait pas partie de l'invention revendiquée en tant que telle.

**Exemple 3 : détermination de l'algorithme pour calculer le score de prédiction de l'« outcome » ou sortie du coma, de l'état végétatif ou de l'état de conscience minimale pour un patient dans le coma après traumatisme crânien.**

Détermination du modèle de prédiction par apprentissage sur une population de 105 patients.

105 patients dans le coma, dans un état végétatif ou un état de conscience minimale après traumatisme crânien ont été admis dans les services de neuro-réanimation de 10 centres participants. Ils ont été inclus dans l'étude s'ils remplissaient les critères suivants :

1) Adultes entre 18 et 75 ans

2) Absence de réponse aux ordres simples au moment de la signature du consentement légal par le représentant autorisé, au moins sept jours et au plus 45 jours après l'accident

3) Etat clinique général permettant le transport du patient

4) conformité cérébrale (« cerebral compliance ») permettant le maintien de la position allongée pour l'acquisition IRM sans développement d'une hypertension intracrânienne

5) Pas de pathologies du système nerveux central (accident vasculaire cérébral, tumeur cérébrale, maladie neurodégénérative) avant l'accident.

5 à 10 sujets sains ont par ailleurs été recrutés dans chaque centre en tant que sujets contrôles pour la séquence d'acquisition d'IRM de diffusion.

L'état neurologique 1 an après l'accident a été évalué pour chaque patient suivant l'échelle GOS (Glasgow Outcom Scale) modifiée. Le score GOS 3 est alors divisé en deux catégories, le score « 3 - » correspondant à l'état de conscience minimale Giacino and Zassler, 1995 [17] et le score « 3+ » à une incapacité sévère. A partir de cette échelle, les 105 patients ont été répartis en deux groupes: les patients avec un pronostic favorable (GOS 3+, 4 et 5) et ceux avec un pronostic défavorable (GOS 1, 2, 3-).

La construction de l'algorithme de classification supervisée correspondant à l'étape e) du procédé a été menée à l'aide de la librairie LIBSVM tel que décrit dans Chang & Lin, 2011 [15]. Dans ce contexte, chaque patient a été caractérisé par sa classe (-1 pour un patient au pronostic défavorable et 1 pour un patient au pronostic favorable) et par les 80 paramètres régionaux de diffusions (SFA_n, SMD_n, SRD_n et SAD_n dans les 20 ROIs) calculé suivant le procédé décrit dans l'exemple 1. L'ensemble de ces informations a été stocké dans un fichier texte nommé 'Training-Data.txt'.

Plusieurs étapes ont alors été effectuées :

a) changement d'échelle des variables entre -1 et 1. Cette étape s'effectue avec la fonction svm-scale de la librairie LIBSVM en lançant la fonction suivante :
svm-scale -l -1 -u 1 TrainingData.txt

b) choix du noyau gaussien pour la projection des données

c) procédure de sélection pas à pas des variables avec un ajustement conjoint des paramètres du noyau de projection qui optimise l'exactitude de la classification (« classification accuracy »), (algorithme sous le logiciel python dénommé « fselect.py tool » disponible au téléchargement en ligne http://www.csie.ntu.edu.tw/~cjlin/libsvmtools/#feature_selection_ tool[17]).

L'algorithme optimal F déterminé par le logiciel de classification supervisé indiqué ci-dessus comporte finalement 32 variables. Le tableau 1 ci-dessous présente les 32 variables.

**Tableau 5 : variables étudiées**

| Fraction d'Anisotropie (FA) | Diffusion Radiale (RD) | Diffusion Axiale (AD) |
| --- | --- | --- |
| ROI #1 | ROI #2 | ROI #3 |
| ROI #2 | ROI #3 | ROI #5 |
| ROI #3 | ROI #4 | ROI #10 |
| ROI #4 | ROI #6 | ROI #16 |
| ROI #5 | ROI #7 | ROI #20 |
| ROI #6 | ROI #8 | - |
| ROI #7 | ROI #10 | - |
| ROI #8 | ROI #15 | - |
| ROI #10 | ROI #16 | - |
| ROI #13 | ROI #18 | - |
| ROI #14 | ROI #19 | - |
| ROI #15 | ROI #20 | - |
| ROI #16 | - | - |
| ROI #18 | - | - |

La liste des numéros de variable est présentée ci-dessous.

**Tableau 6 : numéro de variable en fonction de la variable**

| Nom variable | Numéro de Variable |
| --- | --- |
| FA - ROI #1 | 1 |
| FA - ROI #2 | 2 |
| FA - ROI #3 | 3 |
| FA - ROI #4 | 4 |
| FA - ROI #5 | 5 |
| FA - ROI #6 | 6 |
| FA - ROI #7 | 7 |
| FA - ROI #8 | 8 |
| FA - ROI #9 | 9 |
| FA - ROI #10 | 10 |
| FA - ROI #13 | 13 |
| FA - ROI #14 | 14 |
| FA - ROI #15 | 15 |

(suite)

| Nom variable | Numéro de Variable |
|---|---|
| FA - ROI #16 | 16 |
| FA - ROI #18 | 18 |
| AD - ROI #3 | 83 |
| AD - ROI #5 | 85 |
| AD - ROI #10 | 90 |
| AD - ROI #16 | 96 |
| AD - ROI #20 | 100 |
| RD - ROI #2 | 122 |
| RD - ROI #3 | 123 |
| RD - ROI #4 | 124 |
| RD - ROI #6 | 126 |
| RD - ROI #7 | 127 |
| RD - ROI #8 | 128 |
| RD - ROI #10 | 130 |
| RD - ROI #15 | 135 |
| RD - ROI #16 | 136 |
| RD - ROI #18 | 138 |
| RD - ROI #19 | 139 |
| RD - ROI #20 | 140 |

La figure 4 présente les résultats obtenus pour chacun des 105 patients. Sur cette figure chaque gamme de mesure est référencée par un numéro et les résultats indiqués en fonction du numéro de variable en gras défini dans le tableau 6.

L'algorithme F comprenant les paramètres optimaux sélectionnés ainsi que les vecteurs supports choisis ont été sauvegardés dans un fichier texte 'predictModel.txt'.

L'application de l'algorithme F permet de calculer un score sDTI ou valeur prédictive pour un patient donné.

Les performances de cet algorithme de classification ont été déterminées par validation croisée, un score sDTI a été calculé pour chacun des 105 patients. A partir de ces scores nous avons déterminé pour chaque intervalle de scores [0, 0,2], [0, 2, 0,3], [0,3, 0,4], [0,4, 0,5], [0,5, 0,6], [0,6, 0,7], [0,7, 0,8], [0,8, 1], le rapport du nombre de patient avec un pronostic défavorable sur le nombre total de patient ayant un score dans ledit intervalle. Les résultats représentés sur la figure 3 ont permis de définir trois plages de prédiction.

Dans cet exemple, une plage positive de scores sDTI ou de valeurs de prédiction est comprise entre 0 et 0,2 et correspond à un pronostic favorable de sortie de coma, de l'état végétatif ou de l'état de conscience minimale, une plage négative de scores sDTI ou de valeurs de prédiction est comprise entre 0,6 et 1 correspond à un pronostic défavorable de sortie de coma, de l'état végétatif ou de l'état de conscience minimale, et une plage intermédiaire de scores sDTI ou de valeurs de prédiction compris entre 0,2 et 0,6 correspondant à un pronostic indécis.

Suite à cette détermination, le procédé a été mis en œuvre pour deux patients donnés.

Pour un nouveau patient donné ayant une lésion cérébrale et étant dans le coma dont la probabilité de sortie éventuelle est inconnue, les paramètres/ valeurs régionaux de SFA, SAD et SRD ont été extraits dans les 20 ROIs suivant le procédé décrit dans l'exemple 1. Le calcul du score de prédiction de la sortie du coma sDTI ont été réalisé à partir alors suivant la procédure suivante :

1. Création du fichier de paramètres du nouveau patient

Les 32 paramètres régionaux SFA_n, SAD_n et SRD_n suivants ont été extraits et sauvegardés dans un fichier texte 'patientData.txt' au format suivant :

« 1 numVar1:Value1 numVar2:Value2 .... numVar32:Value32 »

où « numVarn » est le numéro de la variable tel que défini dans le tableau 6 précité et Value n est la valeur du paramètre correspondant.

Le calcul du score ou valeur de prédiction sDTI à partir de l'algorithme F et des mesures du patient ont été effectué avec la fonction svm-predict de la librairie LIBSVM avec la commande suivante :

svm-predict -b 1 patientData.txt predictModel.txt sDTI.txt ce qui a permis d'obtenir un fichier texte intitulé 'sDTI.txt' comprenant les informations suivantes :

« Classe prédite par le modèle Probabilité d'appartenir à la classe prédite »

où la Classe C prédite par le modèle peut prendre les valeurs 1 (pronostic favorable) ou (-1 pronostic défavorable) et la Probabilité P d'appartenir à la classe prédite peut prendre des valeurs entre 0,5 et 1.

le score de prédiction ou valeur de prédiction sDTI correspondant à la probabilité d'un pronostic défavorable est :

$$sDTI = \quad P \text{ si } C=-1$$

$$1-P \text{ si } C=1$$

Ainsi, pour ce patient donné il est possible de calculer la probabilité d'un pronostic défavorable permettant ainsi de déterminer une prédiction de sortie du coma, de l'état végétatif ou de l'état de conscience minimale dudit sujet test.

Le procédé a été mis en œuvre sur deux patients dans le coma après un traumatisme crânien sévère, nommé dans le présent exemple patient1 et patient2.

Pour ces deux patients, les 32 paramètres régionaux SFA_n, SAD_n et SRD_n ont été extraits et sauvegardés dans un fichier texte 'patientData_patient1.txt' et 'patientData_patient2.txt' selon la procédure décrite précédemment.

Le tableau 7 ci-dessous comprend les mesures obtenues pour chaque patient classé en fonction du numéro de variable tel qu'indiqué dans le tableau 6.

Tableau 7 Valeur des 32 variables pour les patients 1 et 2

| Numéro de variable | 1 | 2 | 3 | 4 | 5 | 6 | 7 | 8 | 9 | 10 | 13 | 14 | 15 | 16 | 18 | 83 | 85 | 90 |
|---|---|---|---|---|---|---|---|---|---|---|---|---|---|---|---|---|---|---|
| Patient1 | 0,95 4 | 0,971 | 0,982 | 0,861 | 1,201 | 1,04 5 | 0,943 | 1,009 | 0,95 6 | 0,981 | 0.789 | 0,948 | 0,945 | 1,033 | 1,039 | 0,95 | 1,012 | 1,006 |
| Patient 2 | 0,73 9 | 0,779 | 0,959 | 0,546 | 0,488 | 0,63 | 0,662 | 0,612 | 0,59 6 | 0,565 | 0,489 | 0,444 | 0,702 | 0,666 | 0,538 | 0,967 | 0,971 | 1,094 |

| Numéro de variable | 96 | | 100 | 122 | 123 | | 124 | 126 | 127 | | 128 | 130 | 135 | 136 | 138 | 140 |
|---|---|---|---|---|---|---|---|---|---|---|---|---|---|---|---|---|
| Patient1 | 0,051 | | 1,02 | 0,964 | 0,966 | | 1,307 | 0,89 | 1,049 | | 1,010 | 1,04 | 1,04 | 0,91 | 0,991 | 1,037 |
| Patient2 | 0,974 | | 1,073 | 1,606 | 1,372 | | 1,966 | 1,612 | 1,493 | | 1,657 | 1,721 | 1,435 | 1,582 | 1,567 | 1,494 |

22

L'application du procédé de calcul du score de prédiction sDTI à partir de l'algorithme F a permis de déterminer un score de prédiction pour chaque patient via l'application de la commande suivante :

svm-predict -b 1 patientData_patient1.txt predictModel.txt sDTI_patient1.txt svm-predict -b 1 patientData_patient2.txt predictModel.txt sDTI_patient2.txt

Les scores (sDTI) de chaque patient ont été alors calculés et étaient respectivement égale à 0,087 pour le patient 1 et 0,837 pour le patient 2. Ces scores ont été générés dans un fichier texte dénommé respectivement : sDTI_patient1 .txt et sDTI_patient2.txt Par comparaison des scores ou valeurs prédictives aux plages de prédiction définies précédemment, le patient 1 avec une valeur prédictive (sDTI) de 0,087 est dans la plage [0,0.2] correspondant à un pronostic favorable de sortie de coma et 0,837 est dans la plage [0.6,1] correspondant à un pronostic défavorable de sortie de coma.

Le patient 2 est finalement décédé en réanimation et le patient 1 s'est réveillé et a été évalué GOS 4 un an après l'accident, correspondant à des séquelles modérées sans dépendance.

Tel que démontré dans cet exemple, le procédé permet de déterminer de manière fiable la prédiction de la sortie ou non du coma d'un patient ayant subi une lésion cérébrale.

Le procédé de l'exemple 4 ci-dessous ne fait pas partie de l'invention revendiquée en tant que telle.

**Exemple 4 : détermination d'une formule de calcul d'un score de prédiction de l'« outcome » ou sortie du coma, de l'état végétatif ou de l'état de conscience minimale pour un patient dans le coma après arrêt cardiaque.**

A partir d'une base de données de patients dans le coma qui ont été inclus dans l'étude s'ils remplissaient les critères suivants :

1) Adultes entre 18 et 75 ans

2) Absence de réponse aux ordres simples au moment de la signature du consentement légal par le représentant autorisé, au moins sept jours et au plus 45 jours après l'accident

3) Etat clinique général permettant le transport du patient

4) conformité cérébrale (« cerebral compliance ») permettant le maintien de la position allongée pour l'acquisition IRM sans développement d'une hypertension intracrânienne

5) Pas de pathologies du système nerveux central (accident vasculaire cérébral, tumeur cérébrale, maladie neurodégénérative) avant l'arrêt cardiaque

**Méthode**

Les 100 patients ont été séparés en deux groupes en fonction de leur état neurologique 1 an après leur accident, déterminé suivant l'échelle de Glasgow étendue (GOSE) (GOSE = [1,3] pour les pronostics défavorables, GOSE = [4-8] pour les pronostics favorables). Chaque patient a été caractérisé par un ensemble de variables $X\_i$ comprenant les mesures régionales de FA, dans les 20 régions d'intérêts définies ci-dessus, l'âge et par le délai en jours *delta* entre l'accident et l'examen d'IRM, les paramètres optimaux *beta_i* tels que le score ont été calculés comme suit :

$$scoreDTI = \frac{1}{1+e^{beta_0 + \sum_i beta_i . X_i}} \quad \text{(formule 1)}$$

Deux formules alternatives ont été calculé, l'une attribuant un poids non nul (a priori) beta_i à chaque variable X_i (scoreDTI complet), l'autre attribuant des poids beta_i nul aux variables X_i les moins discriminante (scoreDTI compact). Dans les deux cas les paramètres optimaux beta_i ont été déterminés par validation croisée selon le procédé/méthode décrite dans Picard R., Cook D. (1984): Cross-Validation of Regression Models. Journal of the American Statistical Association 79 (387): 575-583. [18]

Le scoreDTI a été évalué sur les patients dans le coma après un arrêt cardiaque, après un traumatisme cranien sévère, et une hémorragie méningée.

Le tableau 8 ci-dessous résume par région d'intérêt les valeurs des coefficients beta$_i$ en fonction des régions d'intérêts lorsque la Fraction d'Anisotropie est mesurée dans les 20 régions précitées pour les patients dans le coma après arrêt cardiaque.

Tableau 8: Paramètres du scoreDTI complet pour les patients dans le coma après arrêt cardiaque

| Predicteurs X_i | Paramètres modèles beta_i |
|---|---|
| Intercept (beta_0) | -24,5214885485 |
| pédoncule cérébelleux moyen (ICBM #1) | 0,0 |
| tronc cérébral antérieur (ICBM #2,7,8) | 0,0 |
| tronc cérébral postérieur (ICBM #9,10,11,12,13,14) | 0,0 |
| genou du corps calleux (ICBM #3) | 6,87535790908 |
| tronc du corps calleux (ICBM #4) | 4,06938927662 |
| splénium du corps calleux (ICBM #5) | 11,855208917 |
| pédoncule cérébral droit (ICBM #15) | -4,85084571328 |
| pédoncule cérébral gauche (ICBM #16) | 2,15989083485 |
| stratum sagittal droit (ICBM #21,29,31,47) | 4,2325734777 |
| stratum sagittal gauche (ICBM #22,30,32,48) | -3,34425242349 |
| Faisceau longitudinal supérieur droit (ICBM #41) | -6,85002870016 |
| faisceau longitudinal supérieur gauche (ICBM #42) | -4,99481835312 |
| bras antérieur de la capsule interne droit (ICBM #17) | 2,0487983025 |
| le bras antérieur de la capsule interne gauche (ICBM #18) | 7,22561663595 |
| bras postérieur de la capsule interne droit (ICBM #19) | 1,8759969088 |
| Bras postérieur de la capsule interne gauche (ICBM #20) | -0,232791690832 |
| capsule externe droite (ICBM #33) | 6,39302863473 |
| capsule externe gauche (ICBM #34) | 0,0222995384139 |
| couronne radiée droite (ICBM #23,25,27) | -0,0594003555767 |
| couronne radiée gauche (ICBM #24,26,28) | 0,145161164427 |

La figure 5 représente une courbe ROC obtenue suivant le procédé décrit par exemple dans [19] pour la prédiction du pronostic défavorable des patients après arrêt cardiaque, l'ordonné représente la sensibilité, l'abscisse 1-spécificité. Tel que démontré sur cette figure, la sensibilité de prédiction du pronostic défavorable est de 73% pour une spécificité de 100%

La figure 6 représente les valeurs de scoreDTI obtenus en fonction des patients classifiés selon le score de GOS.

Ainsi, tel que représenté dans la figure 6, un scoreDTI compris de 0.83 à 1 signifie une prédiction défavorable de sortie du coma.

Le tableau 9 ci-dessous résume par région d'intérêt les valeurs des coefficients beta$_i$ en fonction des régions d'intérêts lorsque la Fraction d'Anisotropie est uniquement mesurée dans les 5 régions précitées indépendamment de l'âge et du temps après l'arrêt cardiaque. Ainsi les coefficients beta_i sont mentionnés comme égal à zéro dans le tableau 9 ci-dessous lorsque la mesure de la FA dans la ou les régions d'intérêt n'a pas été effectuée.

Tableau 9: Paramètres du scoreDTI complet pour les patients dans le coma après arrêt cardiaque

| Predicteurs X_i | Paramètres modèles beta_i |
|---|---|
| Intercept (beta_0) | -20,7785254295 |
| Age | 0,0 |
| delta | 0,0 |

(suite)

| Predicteurs X_i | Paramètres modèles beta_i |
|---|---|
| pédoncule cérébelleux moyen (ICBM #1) | 0,0 |
| tronc cérébral antérieur (ICBM #2,7,8) | 0,0 |
| tronc cérébral postérieur (ICBM #9,10,11,12,13,14) | 0,0 |
| genou du corps calleux (ICBM #3) | 0,0 |
| tronc du corps calleux (ICBM #4) | 0,0 |
| splénium du corps calleux (ICBM #5) | 0,0 |
| pédoncule cérébral droit (ICBM #15) | 0,0 |
| pédoncule cérébral gauche (ICBM #16) | 1,87189152758 |
| stratum sagittal droit (ICBM #21,29,31,47) | 0,0 |
| stratum sagittal gauche (ICBM #22,30,32,48) | 0,0 |
| Faisceau longitudinal supérieur droit (ICBM #41) | 0,0 |
| faisceau longitudinal supérieur gauche (ICBM #42) | 0,0 |
| bras antérieur de la capsule interne droit (ICBM #17) | 1,19502993841 |
| le bras antérieur de la capsule interne gauche (ICBM #18) | 1,7329368258 |
| bras postérieur de la capsule interne droit (ICBM #19) | 0,0 |
| Bras postérieur de la capsule interne gauche (ICBM #20) | 0,0 |
| capsule externe droite (ICBM #33) | 10,1577820013 |
| capsule externe gauche (ICBM #34) | 0,0 |
| couronne radiée droite (ICBM #23,25,27) | 0,0 |
| couronne radiée gauche (ICBM #24,26,28) | 7,3919471341 |

La figure 7 représente une courbe ROC obtenue suivant le procédé décrit par exemple dans Fawcett T. (2006): An introduction to ROC analysis. Pattern Recognition Letters, 27, 861-874. [19] pour la prédiction du pronostic défavorable des patients après arrêt cardiaque, l'ordonné représente la sensibilité, l'abscisse 1-spécificité. Tel que démontré sur cette figure, la sensibilité de prédiction du pronostic défavorable est de 80% pour une spécificité de 100%

La figure 8 représente les valeurs de scoreDTI obtenus en fonction des patients classifiés selon le score de GOS.

Ainsi, tel que représenté dans la figure 8, un scoreDTI compris de 0.8 à 1 signifie une prédiction défavorable de sortie du coma.

De manière surprenante, les inventeurs ont démontré ainsi que le scoreDTI ainsi déterminé permet de prédire la sortie du coma après un arrêt cardiaque avec une sensibilité et une spécificité de 80% et 100% respectivement.

1. [Basser et al. 1994] P.J. Basser, J. Mattiello, D. LeBihan, MR diffusion tensor spectroscopy and imaging. Biophysical Journal, 66(1):259-267, 1994.

2. [Basser and Pierpaoli 1996] P. J. Basser and C. Pierpaoli, Microstructuraland Physiological Features of Tissues Elucidated by Quantitative-Diffusion-Tensor MRI. Journal of Magnetic Resonance, 111(3):209-219, 1996.

3. [Jenkinson and Smith 2001] M. Jenkinson and S.M. Smith, A global optimisation method for robust affine registration of brain images. Medical Image Analysis, 5(2):143-156, 2001.

4. [Jenkinson et al. 2002] M. Jenkinson, P.R. Bannister, J.M. Brady, and S.M. Smith, Improved optimisation for the robust and accurate linear registration and motion correction of brain images. NeuroImage, 17(2):825-841, 2002.

5. [Lescot et al. 2008] T. Lescot, L. Abdennour, A.-L. Boch, L. Puybasset, Treatment of intracranialhypertension.

Curr Opin Crit Care, 14:129-134, 2008.

6. [Smith 2002] S.M. Smith, Fast robust automated brain extraction. Human Brain Mapping, 17(3):143-155, 2002.

7. [Smith et al. 2004] S.M. Smith, M. Jenkinson, M.W. Woolrich, C.F. Beckmann, T.E.J. Behrens, H. Johansen-Berg, P.R. Bannister, M. De Luca, I. Drobnjak, D.E. Flitney, R. Niazy, J. Saunders, J. Vickers, Y. Zhang, N. De Stefano, J.M. Brady, and P.M. Matthews, Advances in functional and structural MR image analysis and implementation as FSL. NeuroImage, 23(S1):208-219, 2004.

8. [Smith et al. 2006] S.M. Smith, M. Jenkinson, H. Johansen-Berg, D. Rueckert, T.E. Nichols, C.E. Mackay, K.E. Watkins, O. Ciccarelli, M.Z. Cader, P.M. Matthews, and T.E.J. Behrens, Tract-based spatial statistics: Voxelwise analysis of multi-subject diffusion data. NeuroImage, 31:1487-1505, 2006.

9. [Mori et al. 2005] S. Mori, S. Wakana, L.M. Nagae-Poetscher, and P.C.M. van Zijl. MRI Atlas of Human White Matter. Elsevier, Amsterdam, The Netherlands (2005)

10. [Andersson 2007a] J.L.R. Andersson, M. Jenkinson and S. Smith. Non-linear optimisation. FMRIB technical report TR07JA1 from www.fmrib.ox.ac.uk/analysis/techrep

11. [Andersson 2007b] J.L.R. Andersson, M. Jenkinson and S. Smith. Non-linear registration, aka Spatial normalisation. FMRIB technical report TR07JA2 from www.fmrib.ox.ac.uk/analysis/techrep

12. [Cox et al. 2004] Robert W Cox, John Ashburner, Hester Breman, Kate Fissell, Christian Haselgrove, Colin J Holmes, Jack L Lancaster, David E Rex, Stephen M Smith, Jeffrey B Woodward, Stephen C Strother (2004). A (Sort of) New Image Data Format Standard: NifTI-1. NeuroImage, Vol. 22 (2004)

13. [Rorden & Brett, 2000] Rorden, C., Brett, M. (2000). Stereotaxic display of brain lesions. Behavioural Neurology, 12, 191-200.

14. [Chang & Lin, 2011] Chih-Chung Chang and Chih-Jen Lin, LIBSVM : a library for support vector machines. ACM Transactions on Intelligent Systems and Technology, 2:27:1--27:27, 2011. Software available at http://www.csie.ntu.edu.tw/-cjlin/libsvm

15. [Chen & Lin, 2005] Y.-W. Chen and C.-J. Lin, Combining SVMs with various feature selection strategies. Chapter of the book « Feature Extraction: Foundations and Applications (Studies in Fuzziness and Soft Computing) » By Isabelle Guyon, Steve Gunn, Masoud Nikravesh, Lotfi A. Zadeh. Publisher: Springer | ISBN: 3540354875 | edition 2006

16. http://www.csie.ntu.edu.tw/~cjlin/libsvmtools/#feature_selection_tool

17. Giacino J.T., Zasler N.D. (1995): Outcome after severe traumatic brain injury: coma, the vegetative state, and the minimally responsive state. J Head Trauma Rehabil 10:40-56.

18. Picard R., Cook D. (1984): Cross-Validation of Regression Models. Journal of the American Statistical Association 79 (387): 575-583.

19. Fawcett T. (2006): An introduction to ROC analysis. Pattern Recognition Letters, 27, 861-874.

## Revendications

1. Procédé ex-vivo de suivi de l'évolution d'une lésion cérébrale chez un sujet test, ayant subi une cérébrolésion, une hémorragie méningée, une hémorragie méningée anévrysmale, un accident hémorragique intraparenchymateux, ou une anoxie cérébrale des suites d'un arrêt cardiaque ou circulatoire, le procédé étant exécuté sur un ordinateur, le procédé comprenant les étapes suivantes à un temps to et à un temps $t_1$ :

   a) Mesures de la Fraction d'Anisotropie $FA_1$ à to et $FA_2$ à $t_1$ dans au moins une première région du cerveau sur une image, ladite image ayant été obtenue, par imagerie par Résonance Magnétique (IRM) du cerveau d'un sujet test, préalablement à l'exécution des étapes a), b) et c) dudit procédé,

b) Mesures de la Diffusibilité Axiale $DA_1$ à to et $DA_2$ à ti dans au moins une deuxième région du cerveau sur une image, ladite image ayant été obtenue par imagerie par Résonance Magnétique (IRM) du cerveau dudit sujet test, préalablement à l'exécution des étapes a), b) et c) dudit procédé,

c) Mesures de la Diffusibilité Radiale $DR_1$ à to et $DR_2$ à ti dans au moins une troisième région du cerveau sur une image, ladite image ayant été obtenue par imagerie par Résonance Magnétique (IRM) du cerveau dudit sujet test, préalablement à l'exécution des étapes a), b) et c) dudit procédé,

d) Détermination des rapports SFA1, $S_{DA}1$, $S_{DR}1$, $S_{FA}2$, $S_{DA}2$, et $S_{DR}2$ tels que définis ci-dessous par comparaison des valeurs $FA_1$, $DA_1$, $DR_1$, $FA_2$, $DA_2$, $DR_2$ mesurées par rapport à des valeurs régionales normales, respectivement de Fraction d'anisotropie $FA_n$ pour ladite première région, de Diffusibilité Axiale $DA_n$ pour ladite deuxième région et de Diffusibilité Radiale $DR_n$ pour ladite troisième région, lesdites valeurs régionales normales étant mesurées pour un groupe de sujets sains de référence, selon les formules suivantes :

$$S_{FA}1 = (FA_1/FA_n)$$

$$S_{DA}1 = (DA_1/DA_n)$$

$$S_{DR}1 = (DR_1/DR_n)$$

$$S_{FA}2 = (FA_2/FA_n)$$

$$S_{DA}2 = (DA_2/DA_n)$$

$$S_{DR}2 = (DR_2/DR_n)$$

e) Détermination de la variation $\Delta S_{FA}$, $\Delta S_{DA}$, $\Delta S_{DR}$ selon les formules suivantes :

$$\Delta S_{FA} = S_{FA}2 - S_{FA}1$$

$$\Delta S_{DA} = S_{DA}2 - S_{DA}1$$

$$\Delta S_{DR} = S_{DR}2 - S_{DR}1$$

la variation $\Delta S_{FA}$, $\Delta S_{DA}$, $\Delta S_{DR}$ étant significative s'il s'agit d'une variation d'au moins deux fois l'écart-type des mesures régionales pour le groupe de sujets sains de référence,

une variation négative significative d'au moins une valeur $\Delta S_{FA}$, $\Delta S_{DA}$, indiquant une aggravation de la lésion, une variation significative positive d'au moins une valeur $\Delta S_{FA}$, $\Delta S_{DA}$, indiquant une récupération, une variation négative significative de $\Delta S_{DR}$ indiquant une récupération, une variation positive significative de $\Delta S_{DR}$ indiquant une aggravation de la lésion,

et dans lequel les mesures de Fraction d'anisotropie, Diffusibilité Axiale, Diffusibilité Radiale sont effectuées dans au moins une des régions du cerveau choisie parmi le pédoncule cérébelleux moyen (ICBM #1), le tronc cérébral antérieur (ICBM #2,7,8), le tronc cérébral postérieur (ICBM #9,10,11,12,13,14), le genou du corps calleux (ICBM #3), le tronc du corps calleux (ICBM #4), le splénium du corps calleux (ICBM #5), le pédoncule cérébral droit (ICBM #15), le pédoncule cérébral gauche (ICBM #16), le stratum sagittal droit (ICBM #21,29,31,47), le stratum sagittal gauche (ICBM #22,30,32,48), le Faisceau longitudinal supérieur droit (ICBM #41), le faisceau longitudinal supérieur gauche (ICBM #42), le bras antérieur de la capsule interne droit (ICBM #17) le bras antérieur de la capsule interne gauche (ICBM #18), le bras postérieur de la capsule interne droit (ICBM #19), la Bras postérieur de la capsule interne gauche (ICBM #20), le capsule externe droite (ICBM #33), le capsule externe gauche (ICBM #34), la couronne radiée droite (ICBM #23,25,27), la couronne radiée gauche (ICBM #24,26,28).

2. Procédé selon la revendication 1, dans lequel les valeurs régionales de références sont des valeurs moyennes de référence mesurées chez au moins un patient de référence différent dudit sujet test.

3. Procédé selon l'une quelconque des revendications 1 à 2, dans lequel les mesures de Fraction d'anisotropie, Diffusibilité Axiale, Diffusibilité Radiale) sont effectuées dans l'ensemble des régions du cerveau suivantes le pédoncule cérébelleux moyen (ICBM #1), le tronc cérébral antérieur (ICBM #2,7,8), le tronc cérébral postérieur (ICBM #9,10,11,12,13,14), le genou du corps calleux (ICBM #3), le tronc du corps calleux (ICBM #4), le splénium du corps calleux (ICBM #5), le pédoncule cérébral droit (ICBM #15), le pédoncule cérébral gauche (ICBM #16), le stratum sagittal droit (ICBM #21,29,31,47), le stratum sagittal gauche (ICBM #22,30,32,48), le Faisceau longitudinal supérieur droit (ICBM #41), le faisceau longitudinal supérieur gauche (ICBM #42), le bras antérieur de la capsule interne droit (ICBM #17) le bras antérieur de la capsule interne gauche (ICBM #18), le bras postérieur de la capsule interne droit (ICBM #19), la Bras postérieur de la capsule interne gauche (ICBM #20), le capsule externe droite (ICBM #33), le capsule externe gauche (ICBM #34), la couronne radiée droite (ICBM #23,25,27), la couronne radiée gauche (ICBM #24,26,28).

4. Procédé selon l'une quelconque des revendications 1 à 3, dans lequel les mesures de la Fraction d'anisotropie, Diffusibilité Axiale et Diffusibilité Radiale sont réalisées sur une image IRM prise sur un sujet test ayant subi un traumatisme crânien et/ou cérébral.

**Patentansprüche**

1. Ex-vivo-Verfahren zur Verfolgung der Entwicklung einer Hirnschädigung bei einer Testperson, die einen Hirnschaden, eine Hirnblutung, eine aneurysmatische Hirnblutung, einen intraparenchymatösen hämorrhagischen Unfall oder eine zerebrale Anoxie aufgrund der Folgen eines Herz- oder Kreislaufstillands erlitten hat, wobei das Verfahren auf einem Computer ausgeführt wird, wobei das Verfahren die folgenden Schritte in einer Zeit to und einer Zeit ti umfasst:

a) Messungen der Anisotropiefraktion $FA_1$ bei to und $FA_2$ bei ti in wenigstens einer ersten Region des Gehirns auf einem Bild, wobei das genannte Bild per bildgebender Kernspintomographie (MRT) des Gehirns einer Testperson vor der Ausführung der Schritte a), b) und c) des genannten Verfahrens erhalten wird,
b) Messungen des axialen Diffusionsvermögens $DA_1$ bei to und $DA_2$ bei ti in wenigstens einer zweiten Region des Gehirns auf einem Bild, wobei das genannte Bild per bildgebender Kernspintomographie (MRT) des Gehirns der genannten Testperson vor der Ausführung der Schritte a), b) und c) des genannten Verfahrens erhalten wird,
c) Messungen des radialen Diffusionsvermögens $DR_1$ bei to und $DR_2$ bei ti in wenigstens einer dritten Region des Gehirns auf einem Bild, wobei das genannte Bild per bildgebender Kernspintomographie (MRT) des Gehirns der genannten Testperson vor der Ausführung der Schritte a), b) und c) des genannten Verfahrens erhalten wird,
d) Bestimmung der Beziehungen SFA1, $S_{DA}1$, $S_{DR}1$, SFA2, $S_{DA}2$ und $S_{DR}2$ wie nachstehend definiert per Vergleich der Werte $FA_1$, $DA_1$, $DR_1$, $FA_2$, $DA_2$, $DR_2$, die in Bezug auf normale regionale Werte gemessen werden, jeweils der Anisotropiefraktion $FA_n$ für die genannte erste Region, des axialen Diffusionsvermögens $DA_n$ für die genannte zweite Region und des radialen Diffusionsvermögens $DR_n$ für die genannte dritte Region, wobei die genannten regionalen normalen Werte für eine Gruppe von gesunden Referenzpersonen gemäß den folgenden Formeln gemessen werden:

$$S_{FA}1 = (FA_1/FA_n)$$

$$S_{DA}1 = (DA_1/DA_n)$$

$$S_{DR}1 = (DR_1/DR_n)$$

$$S_{FA}2 = (FA_2/FA_n)$$

$$S_{DA2} = (DA_2/DA_n)$$

$$S_{DR2} = (DR_2/DR_n)$$

e) Bestimmung der Variation $AS_{FA}$, $AS_{DA}$, $\Delta S_{DR}$ gemäß den folgenden Formeln:

$$\Delta S_{FA} = S_{FA2} - S_{FA}1$$

$$\Delta S_{DA} = S_{DA2} - S_{DA}1$$

$$\Delta S_{DR} = S_{DR2} - S_{DR}1$$

wobei die Variation $AS_{FA}$, $AS_{DA}$, $\Delta S_{DR}$ erheblich ist, wenn es sich um eine Variation wenigstens des Zweifachen der Standardabweichung der regionalen Messungen für die Gruppe von gesunden Referenzpersonen handelt, eine negative Variation, die wenigstens für einen Wert $AS_{FA}$, $AS_{DA}$, erheblich ist, der eine Verschlechterung der Schädigung anzeigt, eine positive Variation, die wenigstens für einen Wert $AS_{FA}$, $AS_{DA}$, erheblich ist, eine Erholung anzeigt, wobei eine negative Variation für den Wert $\Delta S_{DR}$, erheblich ist, der eine Erholung anzeigt, wobei eine positive Variation für den Wert $\Delta S_{DR}$, erheblich ist, der eine Verschlechterung der Schädigung anzeigt, und bei dem die Messungen der Anisotropiefraktion, des axialen Diffusionsvermögens, des radialen Diffusionsvermögens in wenigstens einer der Region des Gehirns ausgeführt werden, die aus dem mittleren zerebralen Pedunculus (ICBM Nr. 1), dem vorderen Hirnstamm (ICBM Nr. 2, 7, 8), dem hinteren Hirnstamm (ICBM Nr. 9, 10, 11, 12, 13, 14), dem Knie des Schwielenkörpers (ICBM Nr. 3), dem Stamm des Schwielenkörpers (ICBM Nr. 4), dem Splenium des Schwielenkörpers (ICBM Nr. 5), dem rechten zerebralen Pedunculus (ICBM Nr. 15), dem linken zerebralen Pedunculus (ICBM Nr. 16), dem rechten sagittalen Stratum (ICBM Nr. 21, 29, 31, 47), dem linken sagittalen Stratum (ICBM Nr. 22, 30, 32, 48), der oberen rechten länglichen Faszie (ICBM Nr. 41), der oberen linken länglichen Faszie (ICBM Nr. 42), dem vorderen Zweig der rechten internen Kapsel (ICBM Nr. 17), dem vorderen Zweig der linken internen Kapsel (ICBM Nr. 18), dem hinteren Zweig der rechten internen Kapsel (ICBM Nr. 19), dem hinteren Zweig der linken internen Kapsel (ICBM Nr. 20), der rechten externen Kapsel (ICBM Nr. 33), der linken externen Kapsel (ICBM Nr. 34), der rechten strahlenförmigen Krone (ICBM Nr. 23, 25, 27), der linken strahlenförmigen Krone (ICBM Nr. 24, 26, 28) ausgeführt werden.

2. Verfahren gemäß Anspruch 1, bei dem die regionalen Bezugswerte durchschnittliche Bezugswerte sind, die bei wenigstens einem von der Testperson unterschiedlichen Referenzpatienten gemessen werden.

3. Verfahren gemäß irgendeinem der Ansprüche 1 bis 2, bei dem die Messungen der Anisotropiefraktion, des axialen Diffusionsvermögens, des radialen Diffusionsvermögens in allen folgenden Regionen des Gehirns ausgeführt werden: dem mittleren zerebralen Pedunculus (ICBM Nr. 1), dem vorderen Hirnstamm (ICBM Nr. 2, 7, 8), dem hinteren Hirnstamm (ICBM Nr. 9, 10, 11, 12, 13, 14), dem Knie des Schwielenkörpers (ICBM Nr. 3), dem Stamm des Schwielenkörpers (ICBM Nr. 4), dem Splenium des Schwielenkörpers (ICBM Nr. 5), dem rechten zerebralen Pedunculus (ICBM Nr. 15), dem linken zerebralen Pedunculus (ICBM Nr. 16), dem rechten sagittalen Stratum (ICBM Nr. 21, 29, 31, 47), dem linken sagittalen Stratum (ICBM Nr. 22, 30, 32, 48), der oberen rechten länglichen Faszie (ICBM Nr. 41), der oberen linken länglichen Faszie (ICBM Nr. 42), dem vorderen Zweig der rechten internen Kapsel (ICBM Nr. 17), dem vorderen Zweig der linken internen Kapsel (ICBM Nr. 18), dem hinteren Zweig der rechten internen Kapsel (ICBM Nr. 19), dem hinteren Zweig der linken internen Kapsel (ICBM Nr. 20), der rechten externen Kapsel (ICBM Nr. 33), der linken externen Kapsel (ICBM Nr. 34), der rechten strahlenförmigen Krone (ICBM Nr. 23, 25, 27), der linken strahlenförmigen Krone (ICBM Nr. 24, 26, 28).

4. Verfahren gemäß irgendeinem der Ansprüche 1 bis 3, bei dem die Messungen der Anisotropiefraktion, des axialen Diffusionsvermögens und des radialen Diffusionsvermögens auf einem MRT-Bild realisiert werden, das bei einer Testperson aufgenommen wird, die ein Schädel- und/oder Hirntrauma erlitten hat.

**Claims**

1. An ex-vivo method of monitoring the trend of a brain injury in a test subject, having been subject to a brain injury, a meningeal hemorrhage, an aneurismal meningeal hemorrhage, an intraparenchymal hemorrhagic accident, or a cerebral anoxia following a cardiac or circulatory arrest, the method being performed on a computer, the method comprising the following steps at a time to and at a time $t_1$:

   a) measuring the fractional anisotropy $FA_1$ at to and $FA_2$ at ti in at least one first region of the brain on an image, said image having been obtained, by magnetic resonance imaging (MRI) of the brain of a test subject, prior to the execution of steps a), b) and c) of said method,
   b) measuring the axial diffusivity $DA_1$ at to and $DA_2$ at ti in at least one second region of the brain on an image, said image having been obtained, by magnetic resonance imaging (MRI) of the brain of said test subject, prior to the execution of steps a), b) and c) of said method,
   c) measuring the radial diffusivity $DR_1$ at to and $DR_2$ at ti in at least one third region of the brain on an image, said image having been obtained, by magnetic resonance imaging (MRI) of the brain of said test subject, prior to the execution of steps a), b) and c) of said method,
   d) determining the ratios $S_{FA}1$, $S_{DA}1$, $S_{DR}1$, $S_{FA}2$, $S_{DA}2$, and $S_{DR}2$ as defined below by comparison of the measured values $FA_1$, $DA_1$, $DR_1$, $FA_2$, $DA_2$, $DR_2$ against normal regional values, respectively of fractional anisotropy Fan for said first region, of axial diffusivity $DA_n$ for said second region and of radial diffusivity $DR_n$ for said third region, said normal regional values being measured for a reference group of healthy subjects, according to the following formulae:

$$S_{FA}1=(FA_1/FA_n)$$

$$S_{DA}1=(DA_1/DA_n)$$

$$S_{DR}1=(DR_1/DR_n)$$

$$S_{FA}2=(FA_2/FA_n)$$

$$S_{DA}2=(DA_2/DA_n)$$

$$SDR2=(DR_2/DR_n)$$

   e) determination of the variation $AS_{FA}$, $\Delta S_{DA}$, $\Delta S_{DR}$ according to the following formulae:

$$\Delta S_{FA}=S_{FA}2-S_{FA}1$$

$$\Delta S_{DA}=S_{DA}2-S_{DA}1$$

$$\Delta S_{DR}=S_{DR}2-S_{DR}1$$

   the variation $AS_{FA}$, $\Delta S_{DA}$, $\Delta S_{DR}$ being significant if it is a variation of at least two times the standard deviation of the regional measurements for the reference group of healthy subjects,

   a significant negative variation of at least one value $AS_{FA}$, $\Delta S_{DA}$ indicating an aggravation of the injury, a significant positive variation of at least one value $AS_{FA}$, $\Delta S_{DA}$ indicating a recovery of the injury, a significant negative variation of $\Delta S_{DR}$ indicating a recovery of the injury, a significant positive variation of $\Delta S_{DR}$ indicating an aggravation of the injury,

wherein the measurements of fractional anisotropy, axial diffusivity, radial diffusivity are performed in at least one of the regions of the brain selected from the middle cerebellar peduncle (ICBM #1), the anterior brain stem (ICBM #2,7,8), the posterior brain stem (ICBM #9,10,11,12,13,14), the genu of the corpus callosium (ICBM #3), the body of the corpus callosium (ICBM #4), the splenium of the corpus callosium (ICBM #5), the right cerebral peduncle (ICBM #15), the left cerebral peduncle (ICBM #16), the right sagittal stratum (ICBM #21,29,31,47), the left sagittal stratum (ICBM #22,30,32,48), the right superior longitudinal fasciculus (ICBM #41), the left superior longitudinal fasciculus (ICBM #42), the anterior limb of the right internal capsule (ICBM #17), the anterior limb of the left internal capsule (ICBM #18), the posterior limb of the right internal capsule (ICBM #19), the posterior limb of the left internal capsule (ICBM #20), the right external capsule (ICBM #33), the left external capsule (ICBM #34), the right corona radiata (ICBM #23,25,27), the left corona radiata (ICBM #24,26,28).

2. Method according to claim 1, wherein the reference regional values are reference average values measured in at least one reference patient different from the test subject.

3. Method according to claim 1 or 2, wherein the measurements of fractional anisotropy, axial diffusivity, radial diffusivity are performed in all the following regions of the brain: the middle cerebellar peduncle (ICBM #1), the anterior brain stem (ICBM #2,7,8), the posterior brain stem (ICBM #9,10,11,12,13,14), the genu of the corpus callosium (ICBM #3), the body of the corpus callosium (ICBM #4), the splenium of the corpus callosium (ICBM #5), the right cerebral peduncle (ICBM #15), the left cerebral peduncle (ICBM #16), the right sagittal stratum (ICBM #21,29,31,47), the left sagittal stratum (ICBM #22,30,32,48), the right superior longitudinal fasciculus (ICBM #41), the left superior longitudinal fasciculus (ICBM #42), the anterior limb of the right internal capsule (ICBM #17), the anterior limb of the left internal capsule (ICBM #18), the posterior limb of the right internal capsule (ICBM #19), the posterior limb of the left internal capsule (ICBM #20), the right external capsule (ICBM #33), the left external capsule (ICBM #34), the right corona radiata (ICBM #23,25,27), the left corona radiata (ICBM #24,26,28).

4. Method according to claim 1, 2 or 3, wherein the measurements of the fractional anisotropy, axial diffusivity and radial diffusivity are performed on an MRI image taken on a test subject having suffered a cranial and/or brain trauma.

Convention radiologique < gauche – droite>

FIGURE 1

FIGURE 2A

FIGURE 2B

FIGURE 2C

FIGURE 3

1) -1 **1**:0,94 **2**:0,889 **3**:0,874 **4**:0,957 **5**:1,0 **6**:0,943 **7**:0,818 **8**:0,852 **9**:0,937 **10**:0,958 **13**:1,031 **14**:0,976 **15**:0,975 **16**:1,011 **18**:0,948 **83**:0,861 **85**:0,99 **90**:0,922 **96**:0,869 **100**:0,95 **122**:1,209 **123**:1,053 **124**:1,225 **126**:1,09 **127**:1,165 **128**:1,092 **130**:0,99 **135**:0,946 **136**:0,874 **138**:0,982 **139**:1,01 **140**:1,005

2) -1 **1**:1,024 **2**:1,126 **3**:0,961 **4**:0,938 **5**:0,962 **6**:0,891 **7**:1,082 **8**:1,008 **9**:0,911 **10**:0,902 **13**:1,028 **14**:1,081 **15**:0,996 **16**:1,001 **18**:1,015 **83**:0,838 **85**:0,847 **90**:0,888 **96**:0,853 **100**:0,927 **122**:0,89 **123**:0,955 **124**:0,991 **126**:1,103 **127**:0,781 **128**:0,889 **130**:1,023 **135**:0,895 **136**:0,876 **138**:0,963 **139**:0,954 **140**:0,966

3) 1 **1**:0,891 **2**:0,963 **3**:0,854 **4**:0,742 **5**:0,769 **6**:0,822 **7**:0,9 **8**:0,829 **9**:0,781 **10**:0,82 **13**:0,831 **14**:0,818 **15**:0,925 **16**:0,896 **18**:0,864 **83**:1,048 **85**:1,012 **90**:1,017 **96**:1,052 **100**:1,185 **122**:1,142 **123**:1,246 **124**:1,777 **126**:1,489 **127**:1,196 **128**:1,271 **130**:1,266 **135**:1,154 **136**:1,267 **138**:1,13 **139**:1,252 **140**:1,381

4) -1 **1**:0,874 **2**:0,787 **3**:0,856 **4**:0,893 **5**:0,955 **6**:0,898 **7**:0,768 **8**:0,809 **9**:0,923 **10**:0,876 **13**:0,93 **14**:0,91 **15**:0,97 **16**:0,897 **18**:0,884 **83**:0,916 **85**:1,001 **90**:0,968 **96**:0,917 **100**:0,974 **122**:1,17 **123**:1,074 **124**:1,178 **126**:1,275 **127**:1,308 **128**:1,208 **130**:1,126 **135**:1,044 **136**:1,103 **138**:1,017 **139**:1,088 **140**:1,082

5) -1 **1**:0,946 **2**:0,927 **3**:0,785 **4**:0,846 **5**:0,76 **6**:0,556 **7**:0,848 **8**:0,758 **9**:0,783 **10**:0,714 **13**:0,915 **14**:0,827 **15**:0,957 **16**:0,961 **18**:0,814 **83**:0,951 **85**:1,022 **90**:1,823 **96**:1,009 **100**:1,499 **122**:1,254 **123**:1,198 **124**:1,406 **126**:4,68 **127**:1,203 **128**:1,362 **130**:2,515 **135**:1,084 **136**:1,072 **138**:1,112 **139**:1,546 **140**:1,692

6) -1 **1**:0,977 **2**:0,942 **3**:0,913 **4**:0,77 **5**:0,753 **6**:0,828 **7**:0,884 **8**:0,934 **9**:0,751 **10**:0,904 **13**:0,91 **14**:0,836 **15**:0,886 **16**:0,938 **18**:0,855 **83**:1,027 **85**:1,032 **90**:1,023 **96**:1,04 **100**:1,14 **122**:1,207 **123**:1,185 **124**:1,899 **126**:1,61 **127**:1,208 **128**:1,21 **130**:1,192 **135**:1,261 **136**:1,149 **138**:1,143 **139**:1,499 **140**:1,34

7) -1 **1**:0,771 **2**:0,833 **3**:0,693 **4**:0,629 **5**:0,715 **6**:0,65 **7**:0,787 **8**:0,849 **9**:0,725 **10**:0,763 **13**:0,692 **140**644 **15**:0,835 **16**:0,797 **18**:0,728 **83**:0,864 **85**:0,848 **90**:0,936 **96**:0,877 **100**:1,157 **122**:1,254 **123**:1,246 **124**1,527 **126**:1,675 **127**:1,208 **128**:1,106 **130**:1,256 **135**:1,187 **136**:1,237 **138**:1,128 **139**:1,174 **140**:1,51

8) -1 **1**:0,981 **2**:0,852 **3**:0,922 **4**:0,744 **5**:0,822 **6**:0,883 **7**:0,807 **8**:0,902 **9**:0,922 **10**:0,894 **13**:0,926 **14**:0,813 **15**:0,895 **16**:0,958 **18**:0,818 **83**:1,017 **85**:0,984 **90**:1,013 **96**:1,009 **100**:1,08 **122**:1,205 **123**:1,09 **124**:1,448 **126**:1,327 **127**:1,298 **128**:1,152 **130**:1,166 **135**:1,094 **136**:1,08 **138**:1,171 **139**:1,2 **140**:1,26

9) -1 **1**:1,009 **2**:1,045 **3**:0,977 **4**:0,921 **5**:0,924 **6**:0,788 **7**:0,906 **8**:0,705 **9**:0,934 **10**:0,972 **13**:0,864 **14**:0,91 **15**:1,012 **16**:1,094 **18**:0,85 **83**:0,907 **85**:0,907 **90**:0,932 **96**:0,749 **100**:0,991 **122**:0,915 **123**:0,959 **124**:1,168 **126**:1,237 **127**:1,023 **128**:1,306 **130**:0,988 **135**:0,94 **136**:0,72 **138**:1,144 **139**:0,995 **140**:0,974

10) -1 **1**:0,85 **2**:0,897 **3**:0,936 **4**:0,586 **5**:0,744 **6**:0,656 **7**:0,805 **8**:0,779 **9**:0,806 **10**:0,858 **13**:0,819 **14**:0,755 **15**:0,96 **16**:0,995 **18**:0,897 **83**:1,071 **85**:1,083 **90**:0,942 **96**:0,867 **100**:1,071 **122**:1,159 **123**:1,162 **124**:2,318 **126**:2,48 **127**:1,153 **128**:1,112 **130**:1,173 **135**:1,0 **136**:1,094 **138**:1,276 **139**:1,176 **140**:1,202

11) -1 **1**:0,805 **2**:0,843 **3**:0,756 **4**:0,779 **5**:0,505 **6**:0,729 **7**:0,672 **8**:0,704 **9**:0,728 **10**:0,781 **13**:0,914 **14**:0,826 **15**:0,729 **16**:0,814 **18**:0,872 **83**:0,992 **85**:1,185 **90**:0,984 **96**:0,993 **100**:1,076 **122**:1,298 **123**:1,383 **124**:1,407 **126**:1,643 **127**:1,381 **128**:1,478 **130**:1,27 **135**:1,455 **136**:1,335 **138**:1,129 **139**:1,371 **140**:1,29

12) 1 **1**:0,92 **2**:0,989 **3**:0,963 **4**:0,743 **5**:0,84 **6**:0,923 **7**:0,957 **8**:0,924 **9**:0,842 **10**:0,865 **13**:0,824 **14**:0,802 **15**:0,948 **16**:0,947 **18**:0,902 **83**:1,045 **85**:1,091 **90**:1,074 **96**:1,059 **100**:1,116 **122**:1,137 **123**:1,103 **124**:1,734 **126**:1,337 **127**:1,166 **128**:1,239 **130**:1,288 **135**:1,177 **136**:1,149 **138**:1,177 **139**:1,457 **140**:1,376

13) 1 **1**:0,973 **2**:0,893 **3**:0,964 **4**:0,954 **5**:0,915 **6**:0,895 **7**:0,778 **8**:0,921 **9**:0,885 **10**:0,907 **13**:0,985 **14**:0,972 **15**:0,888 **16**:1,005 **18**:0,968 **83**:1,047 **85**:1,003 **90**:1,036 **96**:1,086 **100**:1,084 **122**:1,133 **123**:1,119 **124**:1,17 **126**:1,363 **127**:1,367 **128**:1,191 **130**:1,18 **135**:1,215 **136**:1,073 **138**:1,052 **139**:1,151 **140**:1,163

14) 1 **1**:0,928 **2**:0,958 **3**:0,891 **4**:0,776 **5**:0,805 **6**:0,857 **7**:0,834 **8**:0,856 **9**:0,66 **10**:0,914 **13**:0,885 **14**:0,881 **15**:0,781 **16**:1,0 **18**:0,884 **83**:1,013 **85**:1,034 **90**:0,94 **96**:1,067 **100**:1,138 **122**:1,068 **123**:1,237 **124**:1,807 **126**:1,574 **127**:1,435 **128**:1,337 **130**:1,145 **135**:1,538 **136**:1,041 **138**:1,189 **139**:1,271 **140**:1,26

15) 1 **1**:1,005 **2**:1,073 **3**:0,927 **4**:0,961 **5**:0,993 **6**:0,995 **7**:0,987 **8**:1,025 **9**:0,946 **10**:1,015 **13**:1,035 **14**:1,066 **15**:0,995 **16**:1,047 **18**:0,971 **83**:1,046 **85**:1,033 **90**:0,983 **96**:1,158 **100**:1,07 **122**:1,095 **123**:1,189 **124**:1,206 **126**:1,119 **127**:1,124 **128**:1,153 **130**:1,061 **135**:1,088 **136**:1,043 **138**:1,095 **139**:1,155 **140**:1,145

16) 1 **1**:0,926 **2**:1,001 **3**:0,995 **4**:0,858 **5**:0,939 **6**:0,897 **7**:0,996 **8**:0,947 **9**:0,832 **10**:0,862 **13**:1,005 **14**:0,951 **15**:0,99 **16**:0,99 **18**:0,893 **83**:1,149 **85**:1,074 **90**:1,039 **96**:1,08 **100**:1,1 **122**:1,113 **123**:1,141 **124**:1,405 **126**:1,439 **127**:1,068 **128**:1,185 **130**:1,251 **135**:1,096 **136**:1,099 **138**:1,126 **139**:1,144 **140**:1,125

17) 1 **1**:0,986 **2**:1,082 **3**:0,96 **4**:0,947 **5**:0,922 **6**:0,77 **7**:0,977 **8**:1,0 **9**:0,967 **10**:0,919 **13**:0,989 **14**:0,864 **15**:1,086 **16**:1,006 **18**:1,029 **83**:1,032 **85**:1,011 **90**:1,052 **96**:1,117 **100**:1,1 **122**:0,954 **123**:1,123 **124**:1,241 **126**:1,736 **127**:1,105 **128**:1,103 **130**:1,194 **135**:0,953 **136**:1,078 **138**:1,057 **139**:1,172 **140**:1,212

18) 1 **1**:0,947 **2**:1,012 **3**:0,944 **4**:0,887 **5**:0,86 **6**:0,853 **7**:0,907 **8**:0,961 **9**:0,9 **10**:0,873 **13**:0,907 **14**:0,899 **15**:1,011 **16**:1,018 **18**:0,969 **83**:1,037 **85**:0,936 **90**:1,03 **96**:1,074 **100**:1,138 **122**:1,042 **123**:1,117 **124**:1,351 **126**:1,52 **127**:1,23 **128**:1,155 **130**:1,245 **135**:1,067 **136**:1,039 **138**:1,075 **139**:1,227 **140**:1,241

19) -1 **1**:0,944 **2**:0,899 **3**:0,965 **4**:0,782 **5**:0,92 **6**:0,948 **7**:0,915 **8**:0,93 **9**:0,897 **10**:0,973 **13**:0,861 **14**:0,886 **15**:0,94 **16**:0,965 **18**:0,872 **83**:1,013 **85**:0,931 **90**:0,982 **96**:1,029 **100**:0,968 **122**:1,132 **123**:1,063 **124**:1,426 **126**:1,164 **127**:1,178 **128**:1,221 **130**:1,043 **135**:1,165 **136**:1,088 **138**:1,12 **139**:1,214 **140**:1,137

FIGURE 4 (1/6)

20) -1 **1**:0,879 **2**:0,814 **3**:0,848 **4**:0,788 **5**:0,744 **6**:0,751 **7**:0,82 **8**:0,777 **9**:0,85 **10**:0,708 **13**:0,844 **14**:0,808 **15**:0,969 **16**:0,867 **18**:0,678 **83**:0,943 **85**:0,998 **90**:1,015 **96**:0,978 **100**:1,093 **122**:1,166 **123**:1,09 **124**:1,373 **126**:1,562 **127**:1,192 **128**:1,15 **130**:1,39 **135**:1,076 **136**:1,159 **138**:1,4 **139**:1,242 **140**:1,402

21) -1 **1**:0,612 **2**:0,704 **3**:0,578 **4**:0,409 **5**:0,47 **6**:0,385 **7**:0,577 **8**:0,624 **9**:0,565 **10**:0,499 **13**:0,579 **14**:0,542 **15**:0,703 **16**:0,697 **18**:0,626 **83**:0,876 **85**:0,864 **90**:0,908 **96**:0,82 **100**:0,978 **122**:1,746 **123**:1,294 **124**:1,794 **126**:1,892 **127**:1,343 **128**:1,4 **130**:1,425 **135**:1,199 **136**:1,195 **138**:1,34 **139**:1,396 **140**:1,405

22) -1 **1**:0,798 **2**:0,77 **3**:0,751 **4**:0,607 **5**:0,588 **6**:0,629 **7**:0,663 **8**:0,519 **9**:0,683 **10**:0,763 **13**:0,839 **14**:0,781 **15**:0,657 **16**:0,803 **18**:1,077 **83**:0,65 **85**:0,786 **90**:0,874 **96**:1,014 **100**:1,119 **122**:1,201 **123**:0,972 **124**:1,672 **126**:1,438 **127**:1,402 **128**:2,293 **130**:1,11 **135**:1,598 **136**:1,398 **138**:1,114 **139**:1,327 **140**:1,357

23) -1 **1**:0,873 **2**:0,94 **3**:0,846 **4**:0,897 **5**:0,832 **6**:0,871 **7**:0,895 **8**:0,862 **9**:0,898 **10**:0,898 **13**:0,986 **14**:1,043 **15**:1,0 **16**:0,981 **18**:1,181 **83**:0,818 **85**:0,778 **90**:0,86 **96**:0,831 **100**:0,888 **122**:0,99 **123**:0,974 **124**:0,953 **126**:1,044 **127**:0,852 **128**:0,958 **130**:0,997 **135**:0,841 **136**:0,9 **138**:0,855 **139**:0,868 **140**:0,91

24) 1 **1**:0,922 **2**:0,872 **3**:0,79 **4**:0,931 **5**:0,959 **6**:0,891 **7**:0,9 **8**:0,873 **9**:0,894 **10**:0,979 **13**:1,043 **14**:0,955 **15**:0,95 **16**:1,031 **18**:0,934 **83**:0,896 **85**:0,969 **90**:1,014 **96**:0,954 **100**:1,025 **122**:1,12 **123**:1,108 **124**:1,06 **126**:1,153 **127**:1,041 **128**:1,098 **130**:1,052 **135**:1,039 **136**:0,928 **138**:1,027 **139**:1,104 **140**:1,046

25) 1 **1**:0,9 **2**:0,949 **3**:0,94 **4**:0,967 **5**:0,874 **6**:0,735 **7**:0,722 **8**:0,932 **9**:0,995 **10**:0,955 **13**:0,977 **14**:1,033 **15**:0,807 **16**:1,02 **18**:1,031 **83**:0,966 **85**:0,751 **90**:0,933 **96**:0,956 **100**:0,907 **122**:0,978 **123**:1,03 **124**:0,982 **126**:1,137 **127**:1,137 **128**:1,066 **130**:0,992 **135**:1,101 **136**:0,939 **138**:0,959 **139**:0,964 **140**:0,953

26) -1 **1**:0,953 **2**:0,944 **3**:0,861 **4**:0,792 **5**:0,757 **6**:0,719 **7**:0,678 **8**:0,754 **9**:0,759 **10**:0,882 **13**:0,843 **14**:0,875 **15**:0,774 **16**:0,922 **18**:0,866 **83**:0,912 **85**:0,867 **90**:0,956 **96**:0,917 **100**:0,987 **122**:1,079 **123**:1,078 **124**:1,265 **126**:1,434 **127**:1,139 **128**:1,214 **130**:1,103 **135**:1,119 **136**:1,04 **138**:1,037 **139**:1,257 **140**:1,118

27) -1 **1**:0,856 **2**:0,851 **3**:0,846 **4**:0,865 **5**:0,911 **6**:0,821 **7**:0,719 **8**:0,871 **9**:0,888 **10**:0,821 **13**:0,973 **14**:0,916 **15**:0,755 **16**:0,947 **18**:0,856 **83**:0,949 **85**:0,881 **90**:0,895 **96**:0,914 **100**:0,95 **122**:1,069 **123**:1,173 **124**:1,206 **126**:1,214 **127**:1,108 **128**:0,998 **130**:1,127 **135**:1,17 **136**:1,005 **138**:1,037 **139**:1,07 **140**:1,081

28) -1 **1**:0,84 **2**:0,665 **3**:0,766 **4**:0,893 **5**:0,864 **6**:0,846 **7**:0,848 **8**:0,828 **9**:0,863 **10**:0,896 **13**:0,882 **14**:0,918 **15**:0,916 **16**:0,853 **18**:0,859 **83**:0,87 **85**:0,957 **90**:0,941 **96**:0,912 **100**:0,979 **122**:1,16 **123**:1,153 **124**:1,277 **126**:1,349 **127**:1,1 **128**:1,069 **130**:1,095 **135**:1,1 **136**:1,189 **138**:1,092 **139**:1,153 **140**:1,133

29) 1 **1**:0,82 **2**:0,788 **3**:0,799 **4**:0,813 **5**:0,597 **6**:0,788 **7**:0,812 **8**:0,705 **9**:0,791 **10**:0,789 **13**:0,931 **14**:0,883 **15**:0,804 **16**:0,742 **18**:0,842 **83**:0,987 **85**:0,824 **90**:1,024 **96**:0,939 **100**:1,004 **122**:1,218 **123**:1,277 **124**:1,369 **126**:1,477 **127**:1,217 **128**:1,325 **130**:1,318 **135**:1,334 **136**:1,408 **138**:1,094 **139**:1,29 **140**:1,298

30) -1 **1**:0,903 **2**:0,939 **3**:0,92 **4**:0,897 **5**:0,917 **6**:0,806 **7**:0,908 **8**:0,92 **9**:0,826 **10**:0,875 **13**:0,947 **14**:0,959 **15**:0,825 **16**:0,985 **18**:0,932 **83**:0,945 **85**:0,916 **90**:1,052 **96**:1,006 **100**:1,028 **122**:1,035 **123**:1,037 **124**:1,226 **126**:1,585 **127**:1,036 **128**:1,062 **130**:1,249 **135**:1,144 **136**:1,028 **138**:1,156 **139**:1,122 **140**:1,143

31) -1 **1**:0,834 **2**:0,788 **3**:0,793 **4**:0,656 **5**:0,476 **6**:0,664 **7**:0,72 **8**:0,794 **9**:0,846 **10**:0,855 **13**:0,797 **14**:0,74 **15**:0,724 **16**:0,783 **18**:0,648 **83**:0,959 **85**:0,758 **90**:0,955 **96**:0,93 **100**:0,951 **122**:1,287 **123**:1,241 **124**:1,499 **126**:1,695 **127**:1,32 **128**:1,261 **130**:1,166 **135**:1,42 **136**:1,304 **138**:1,307 **139**:1,303 **140**:1,317

32) -1 **1**:0,64 **2**:0,733 **3**:0,705 **4**:0,722 **5**:0,748 **6**:0,732 **7**:0,613 **8**:0,915 **9**:0,897 **10**:0,774 **13**:0,796 **14**:0,821 **15**:0,663 **16**:0,727 **18**:0,861 **83**:0,904 **85**:0,708 **90**:0,918 **96**:0,852 **100**:1,107 **122**:1,144 **123**:1,257 **124**:1,44 **126**:1,632 **127**:1,192 **128**:0,936 **130**:1,227 **135**:1,224 **136**:1,35 **138**:1,078 **139**:1,277 **140**:1,133

33) -1 **1**:0,874 **2**:0,776 **3**:0,806 **4**:0,729 **5**:0,717 **6**:0,816 **7**:0,695 **8**:0,742 **9**:0,837 **10**:0,906 **13**:0,832 **14**:0,736 **15**:0,812 **16**:0,757 **18**:0,84 **83**:0,92 **85**:0,867 **90**:0,978 **96**:0,885 **100**:0,941 **122**:1,116 **123**:1,177 **124**:1,532 **126**:1,348 **127**:1,251 **128**:1,226 **130**:1,107 **135**:1,253 **136**:1,318 **138**:1,043 **139**:1,174 **140**:1,156

34) -1 **1**:0,888 **2**:0,894 **3**:0,73 **4**:0,789 **5**:0,864 **6**:0,874 **7**:0,834 **8**:0,815 **9**:0,908 **10**:0,738 **13**:0,953 **14**:0,888 **15**:0,89 **16**:0,877 **18**:0,669 **83**:0,803 **85**:0,991 **90**:1,13 **96**:0,927 **100**:1,123 **122**:1,064 **123**:1,136 **124**:1,529 **126**:1,579 **127**:1,147 **128**:1,221 **130**:1,623 **135**:1,152 **136**:1,166 **138**:1,245 **139**:1,156 **140**:1,419

35) -1 **1**:0,935 **2**:0,834 **3**:0,909 **4**:0,724 **5**:0,842 **6**:0,833 **7**:0,796 **8**:0,768 **9**:0,708 **10**:0,836 **13**:0,902 **14**:0,917 **15**:0,837 **16**:0,91 **18**:0,847 **83**:0,965 **85**:0,887 **90**:0,979 **96**:0,972 **100**:0,972 **122**:1,069 **123**:1,086 **124**:1,472 **126**:1,36 **127**:1,043 **128**:1,182 **130**:1,221 **135**:1,223 **136**:1,129 **138**:1,167 **139**:1,231 **140**:1,158

36) -1 **1**:0,819 **2**:0,819 **3**:0,817 **4**:0,681 **5**:0,814 **6**:0,764 **7**:0,706 **8**:0,768 **9**:0,852 **10**:0,906 **13**:0,968 **14**:0,968 **15**:0,843 **16**:0,921 **18**:1,007 **83**:0,909 **85**:0,949 **90**:0,929 **96**:0,915 **100**:0,944 **122**:1,16 **123**:1,166 **124**:1,681 **126**:1,552 **127**:1,272 **128**:1,258 **130**:1,055 **135**:1,157 **136**:1,067 **138**:0,938 **139**:1,114 **140**:1,095

37) -1 **1**:0,825 **2**:0,769 **3**:0,747 **4**:0,592 **5**:0,622 **6**:0,509 **7**:0,664 **8**:0,718 **9**:0,7 **10**:0,7 **13**:0,736 **14**:0,816 **15**:0,627 **16**:0,816 **18**:0,803 **83**:0,971 **85**:0,833 **90**:0,925 **96**:0,941 **100**:1,007 **122**:1,311 **123**:1,32 **124**:1,71 **126**:2,183 **127**:1,423 **128**:1,345 **130**:1,353 **135**:1,624 **136**:1,294 **138**:1,106 **139**:1,331 **140**:1,312

38) -1 **1**:0,979 **2**:0,972 **3**:0,89 **4**:0,927 **5**:0,725 **6**:0,777 **7**:0,839 **8**:0,704 **9**:0,919 **10**:0,907 **13**:0,905 **14**:0,89 **15**:0,813 **16**:0,798 **18**:0,901 **83**:0,941 **85**:0,901 **90**:0,989 **96**:0,836 **100**:1,021 **122**:1,103 **123**:1,188 **124**:1,308 **126**:1,614 **127**:1,355 **128**:1,469 **130**:1,19 **135**:1,384 **136**:1,326 **138**:1,134 **139**:1,127 **140**:1,065

39) -1 **1**:0,972 **2**:0,929 **3**:0,854 **4**:0,755 **5**:0,831 **6**:0,71 **7**:0,749 **8**:0,726 **9**:0,821 **10**:0,817 **13**:0,817 **14**:0,865 **15**:0,812 **16**:0,756 **18**:0,824 **83**:0,916 **85**:0,712 **90**:0,983 **96**:0,826 **100**:0,985 **122**:1,186 **123**:1,202 **124**:1,92 **126**:1,736 **127**:1,416 **128**:1,349 **130**:1,318 **135**:1,37 **136**:1,387 **138**:1,104 **139**:1,289 **140**:1,185

FIGURE 4 (2/6)

40) -1 **1**:1,023 **2**:0,977 **3**:0,946 **4**:0,915 **5**:0,805 **6**:0,738 **7**:0,88 **8**:0,811 **9**:0,958 **10**:0,933 **13**:0,931 **14**:0,961 **15**:0,889 **16**:0,907 **18**:0,981 **83**:1,024 **85**:0,722 **90**:1,029 **96**:0,943 **100**:1,019 **122**:1,038 **123**:1,149 **124**:1,32 **126**:1,716 **127**:1,246 **128**:1,36 **130**:1,16 **135**:1,22 **136**:1,203 **138**:1,02 **139**:1,152 **140**:1,088

41) -1 **1**:0,995 **2**:0,913 **3**:0,883 **4**:0,814 **5**:0,756 **6**:0,857 **7**:0,937 **8**:0,706 **9**:0,788 **10**:0,817 **13**:0,862 **14**:0,678 **15**:0,929 **16**:0,607 **18**:0,927 **83**:1,039 **85**:0,912 **90**:1,117 **96**:0,896 **100**:1,04 **122**:1,201 **123**:1,261 **124**:1,659 **126**:1,395 **127**:1,143 **128**:1,826 **130**:1,406 **135**:1,201 **136**:1,826 **138**:1,167 **139**:1,368 **140**:1,316

42) -1 **1**:1,011 **2**:0,995 **3**:0,954 **4**:0,599 **5**:0,94 **6**:1,0 **7**:0,907 **8**:0,96 **9**:0,766 **10**:0,823 **13**:0,664 **14**:0,968 **15**:0,987 **16**:1,0 **18**:0,985 **83**:0,934 **85**:0,979 **90**:0,984 **96**:0,993 **100**:1,002 **122**:0,978 **123**:1,024 **124**:2,285 **126**:0,957 **127**:1,171 **128**:0,998 **130**:1,296 **135**:1,069 **136**:1,003 **138**:1,01 **139**:2,058 **140**:1,089

43) 1 **1**:1,01 **2**:1,055 **3**:0,936 **4**:0,964 **5**:1,019 **6**:0,915 **7**:0,947 **8**:0,964 **9**:0,948 **10**:0,94 **13**:0,946 **14**:0,981 **15**:1,057 **16**:0,94 **18**:1,031 **83**:0,937 **85**:0,966 **90**:0,946 **96**:0,965 **100**:0,963 **122**:1,015 **123**:1,061 **124**:1,103 **126**:0,977 **127**:1,108 **128**:1,053 **130**:1,046 **135**:0,934 **136**:1,132 **138**:1,02 **139**:1,001 **140**:0,93

44) 1 **1**:1,023 **2**:1,032 **3**:0,939 **4**:1,037 **5**:0,995 **6**:0,96 **7**:0,932 **8**:0,912 **9**:0,904 **10**:0,961 **13**:0,896 **14**:0,959 **15**:0,972 **16**:0,996 **18**:0,994 **83**:0,98 **85**:0,856 **90**:0,906 **96**:0,991 **100**:0,812 **122**:0,909 **123**:1,119 **124**:0,462 **126**:0,618 **127**:1,048 **128**:1,131 **130**:0,959 **135**:1,027 **136**:0,998 **138**:1,088 **139**:0,802 **140**:0,829

45) -1 **1**:0,904 **2**:0,844 **3**:0,846 **4**:0,855 **5**:0,689 **6**:0,851 **7**:0,856 **8**:0,793 **9**:0,882 **10**:0,894 **13**:0,801 **14**:0,795 **15**:0,881 **16**:0,836 **18**:0,872 **83**:0,946 **85**:0,872 **90**:0,987 **96**:0,877 **100**:0,979 **122**:1,219 **123**:1,249 **124**:1,487 **126**:1,341 **127**:1,183 **128**:1,316 **130**:1,153 **135**:1,156 **136**:1,237 **138**:1,03 **139**:1,15 **140**:1,123

46) -1 **1**:0,712 **2**:0,791 **3**:0,842 **4**:0,72 **5**:0,772 **6**:0,724 **7**:0,677 **8**:0,834 **9**:0,774 **10**:0,765 **13**:0,75 **14**:0,702 **15**:0,845 **16**:0,673 **18**:0,807 **83**:0,882 **85**:1,103 **90**:0,995 **96**:1,131 **100**:1,182 **122**:1,715 **123**:1,162 **124**:2,699 **126**:2,347 **127**:1,849 **128**:1,343 **130**:1,429 **135**:1,883 **136**:2,122 **138**:1,995 **139**:2,119 **140**:1,31

47) -1 **1**:0,744 **2**:0,739 **3**:0,655 **4**:0,693 **5**:0,767 **6**:0,641 **7**:0,517 **8**:0,699 **9**:0,798 **10**:0,898 **13**:0,739 **14**:0,746 **15**:0,815 **16**:0,612 **18**:0,888 **83**:0,74 **85**:0,735 **90**:0,88 **96**:1,059 **100**:1,165 **122**:1,491 **123**:1,294 **124**:2,181 **126**:1,942 **127**:2,862 **128**:2,527 **130**:1,114 **135**:1,282 **136**:2,293 **138**:1,868 **139**:1,619 **140**:1,381

48) -1 **1**:1,053 **2**:1,038 **3**:0,939 **4**:0,743 **5**:0,967 **6**:0,926 **7**:0,87 **8**:0,845 **9**:0,855 **10**:0,905 **13**:0,862 **14**:0,747 **15**:0,929 **16**:0,937 **18**:0,875 **83**:0,977 **85**:0,834 **90**:1,007 **96**:1,056 **100**:1,145 **122**:1,04 **123**:1,118 **124**:1,84 **126**:1,308 **127**:1,3 **128**:1,354 **130**:1,218 **135**:1,209 **136**:1,226 **138**:1,337 **139**:1,226 **140**:1,397

49) -1 **1**:1,049 **2**:1,19 **3**:1,036 **4**:0,698 **5**:0,821 **6**:0,918 **7**:0,94 **8**:0,944 **9**:0,775 **10**:0,876 **13**:0,585 **14**:0,891 **15**:0,888 **16**:0,926 **18**:0,787 **83**:0,934 **85**:0,999 **90**:0,994 **96**:0,944 **100**:1,02 **122**:0,867 **123**:0,903 **124**:1,69 **126**:1,176 **127**:0,948 **128**:1,059 **130**:1,171 **135**:1,199 **136**:1,114 **138**:1,229 **139**:1,317 **140**:1,237

50) 1 **1**:1,005 **2**:0,907 **3**:0,953 **4**:0,886 **5**:0,984 **6**:0,98 **7**:0,858 **8**:0,989 **9**:0,971 **10**:1,019 **13**:0,811 **14**:1,074 **15**:0,918 **16**:0,962 **18**:0,97 **83**:0,931 **85**:0,979 **90**:1,01 **96**:0,964 **100**:1,027 **122**:0,983 **123**:0,97 **124**:1,262 **126**:0,971 **127**:1,094 **128**:0,895 **130**:0,972 **135**:1,157 **136**:1,034 **138**:0,954 **139**:1,275 **140**:0,968

51) 1 **1**:0,977 **2**:1,014 **3**:1,005 **4**:0,957 **5**:0,773 **6**:0,922 **7**:0,997 **8**:0,826 **9**:1,007 **10**:0,996 **13**:1,002 **14**:0,937 **15**:1,072 **16**:0,764 **18**:0,92 **83**:1,036 **85**:0,895 **90**:1,025 **96**:0,921 **100**:0,985 **122**:0,891 **123**:1,059 **124**:1,139 **126**:1,065 **127**:0,938 **128**:1,262 **130**:1,037 **135**:0,86 **136**:1,362 **138**:1,073 **139**:1,048 **140**:1,092

52) 1 **1**:0,92 **2**:1,017 **3**:0,977 **4**:0,909 **5**:0,874 **6**:0,92 **7**:0,904 **8**:0,876 **9**:0,926 **10**:0,906 **13**:0,972 **14**:1,008 **15**:0,879 **16**:0,924 **18**:0,928 **83**:0,976 **85**:1,037 **90**:1,045 **96**:0,979 **100**:1,057 **122**:0,954 **123**:1,011 **124**:1,259 **126**:1,313 **127**:1,109 **128**:1,12 **130**:1,187 **135**:1,172 **136**:1,154 **138**:1,089 **139**:1,16 **140**:1,131

53) -1 **1**:0,999 **2**:1,002 **3**:0,955 **4**:0,937 **5**:0,701 **6**:0,875 **7**:0,875 **8**:0,874 **9**:0,908 **10**:0,964 **13**:0,988 **14**:0,96 **15**:0,881 **16**:0,984 **18**:0,94 **83**:0,973 **85**:0,74 **90**:0,936 **96**:0,89 **100**:0,917 **122**:0,932 **123**:1,032 **124**:1,004 **126**:1,155 **127**:1,082 **128**:1,147 **130**:1,003 **135**:1,141 **136**:0,931 **138**:1,02 **139**:1,015 **140**:1,009

54) 1 **1**:0,669 **2**:1,004 **3**:1,035 **4**:0,701 **5**:0,64 **6**:0,729 **7**:0,829 **8**:0,818 **9**:0,741 **10**:0,725 **13**:0,786 **14**:0,761 **15**:0,813 **16**:0,762 **18**:0,764 **83**:1,05 **85**:0,998 **90**:1,0 **96**:0,926 **100**:1,006 **122**:0,999 **123**:0,952 **124**:1,726 **126**:1,702 **127**:1,181 **128**:1,319 **130**:1,388 **135**:1,186 **136**:1,42 **138**:1,269 **139**:1,324 **140**:1,313

55) -1 **1**:0,984 **2**:0,93 **3**:0,909 **4**:0,875 **5**:0,787 **6**:0,898 **7**:0,8 **8**:0,94 **9**:0,885 **10**:0,843 **13**:0,941 **14**:0,971 **15**:0,906 **16**:0,943 **18**:0,843 **83**:0,875 **85**:0,95 **90**:0,946 **96**:0,906 **100**:0,971 **122**:1,027 **123**:1,014 **124**:1,357 **126**:1,289 **127**:1,284 **128**:1,007 **130**:1,192 **135**:1,06 **136**:1,034 **138**:1,093 **139**:1,14 **140**:1,139

56) -1 **1**:0,973 **2**:0,871 **3**:0,857 **4**:0,822 **5**:0,571 **6**:0,755 **7**:0,801 **8**:0,78 **9**:0,737 **10**:0,708 **13**:0,782 **14**:0,865 **15**:0,831 **16**:0,822 **18**:0,851 **83**:0,974 **85**:0,845 **90**:0,969 **96**:0,91 **100**:1,024 **122**:1,021 **123**:1,181 **124**:1,394 **126**:1,628 **127**:1,23 **128**:1,286 **130**:1,381 **135**:1,252 **136**:1,245 **138**:1,118 **139**:1,322 **140**:1,322

57) 1 **1**:0,988 **2**:0,972 **3**:0,946 **4**:0,817 **5**:0,793 **6**:0,765 **7**:0,908 **8**:0,887 **9**:0,821 **10**:0,724 **13**:0,799 **14**:0,882 **15**:0,87 **16**:0,848 **18**:0,916 **83**:1,035 **85**:0,928 **90**:0,973 **96**:1,01 **100**:1,036 **122**:1,071 **123**:1,084 **124**:1,426 **126**:1,602 **127**:1,151 **128**:1,18 **130**:1,373 **135**:1,229 **136**:1,313 **138**:1,087 **139**:1,205 **140**:1,232

58) 1 **1**:1,008 **2**:1,043 **3**:0,934 **4**:0,959 **5**:0,943 **6**:0,924 **7**:0,924 **8**:1,011 **9**:0,902 **10**:0,914 **13**:0,955 **14**:0,947 **15**:0,971 **16**:0,993 **18**:0,961 **83**:1,027 **85**:0,924 **90**:1,019 **96**:1,032 **100**:0,997 **122**:0,995 **123**:1,135 **124**:1,032 **126**:1,189 **127**:1,003 **128**:0,914 **130**:1,128 **135**:1,044 **136**:1,052 **138**:1,04 **139**:1,088 **140**:1,08

59) -1 **1**:1,028 **2**:0,951 **3**:0,919 **4**:0,878 **5**:0,825 **6**:0,941 **7**:0,932 **8**:0,86 **9**:0,946 **10**:0,772 **13**:0,917 **14**:0,831 **15**:1,026 **16**:1,013 **18**:0,723 **83**:0,944 **85**:0,912 **90**:0,961 **96**:1,07 **100**:1,028 **122**:1,079 **123**:1,075 **124**:1,248 **126**:1,12 **127**:1,079 **128**:1,211 **130**:1,354 **135**:0,998 **136**:1,037 **138**:1,557 **139**:1,014 **140**:1,116

60) 1 **1**:1,005 **2**:0,919 **3**:0,937 **4**:0,776 **5**:0,891 **6**:0,918 **7**:0,885 **8**:0,915 **9**:0,886 **10**:0,905 **13**:0,889 **14**:0,845 **15**:0,938 **16**:0,938 **18**:0,924 **83**:1,043 **85**:0,964 **90**:1,017 **96**:1,012 **100**:1,039 **122**:1,187 **123**:1,136 **124**:1,366 **126**:1,18 **127**:1,18 **128**:1,117 **130**:1,151 **135**:1,116 **136**:1,14 **138**:1,088 **139**:1,132 **140**:1,154

FIGURE 4 (3/6)

61) -1 **1**:0,923 **2**:1,042 **3**:0,96 **4**:0,831 **5**:0,796 **6**:0,943 **7**:0,906 **8**:0,903 **9**:0,904 **10**:0,893 **13**:0,893 **14**:0,899 **15**:1,055 **16**:1,039 **18**:0,893 **83**:0,976 **85**:1,008 **90**:0,967 **96**:1,019 **100**:1,036 **122**:0,953 **123**:1,043 **124**:1,446 **126**:1,098 **127**:1,132 **128**:1,107 **130**:1,138 **135**:0,921 **136**:0,93 **138**:1,082 **139**:1,164 **140**:1,134

62) 1 **1**:1,063 **2**:1,131 **3**:1,031 **4**:1,002 **5**:1,013 **6**:0,984 **7**:1,061 **8**:1,056 **9**:1,045 **10**:1,058 **13**:1,055 **14**:1,063 **15**:1,059 **16**:1,032 **18**:1,048 **83**:1,019 **85**:0,986 **90**:0,978 **96**:1,028 **100**:0,978 **122**:0,841 **123**:0,99 **124**:0,987 **126**:1,006 **127**:0,933 **128**:0,895 **130**:0,906 **135**:0,932 **136**:0,974 **138**:0,977 **139**:0,975 **140**:0,968

63) -1 **1**:1,007 **2**:0,961 **3**:0,966 **4**:0,933 **5**:0,767 **6**:0,939 **7**:0,906 **8**:0,938 **9**:0,915 **10**:0,953 **13**:0,949 **14**:0,924 **15**:0,947 **16**:0,963 **18**:0,95 **83**:1,034 **85**:0,992 **90**:0,987 **96**:0,977 **100**:1,009 **122**:1,066 **123**:1,097 **124**:1,2 **126**:1,138 **127**:1,142 **128**:1,057 **130**:1,056 **135**:1,087 **136**:1,052 **138**:1,029 **139**:1,078 **140**:1,107

64) 1 **1**:0,995 **2**:0,947 **3**:1,0 **4**:1,023 **5**:0,93 **6**:0,955 **7**:0,879 **8**:0,941 **9**:1,018 **10**:1,036 **13**:0,984 **14**:0,974 **15**:0,889 **16**:0,972 **18**:1,026 **83**:1,04 **85**:0,948 **90**:1,002 **96**:1,008 **100**:1,014 **122**:1,04 **123**:1,059 **124**:0,957 **126**:1,109 **127**:1,109 **128**:1,069 **130**:0,952 **135**:1,152 **136**:1,054 **138**:0,981 **139**:1,011 **140**:1,004

65) -1 **1**:1,035 **2**:1,028 **3**:0,978 **4**:0,948 **5**:0,972 **6**:0,933 **7**:1,006 **8**:1,073 **9**:0,951 **10**:0,877 **13**:0,975 **14**:0,977 **15**:1,083 **16**:0,98 **18**:0,938 **83**:1,089 **85**:1,016 **90**:1,016 **96**:1,084 **100**:1,041 **122**:1,013 **123**:1,154 **124**:1,104 **126**:1,192 **127**:1,062 **128**:0,883 **130**:1,187 **135**:0,893 **136**:1,116 **138**:1,053 **139**:1,032 **140**:1,024

66) 1 **1**:1,016 **2**:1,042 **3**:1,085 **4**:0,855 **5**:0,929 **6**:1,0 **7**:1,007 **8**:1,047 **9**:1,035 **10**:0,986 **13**:0,969 **14**:0,971 **15**:1,042 **16**:1,048 **18**:0,934 **83**:1,023 **85**:0,981 **90**:1,049 **96**:1,063 **100**:1,038 **122**:0,998 **123**:0,927 **124**:1,291 **126**:0,966 **127**:0,994 **128**:0,909 **130**:1,069 **135**:0,964 **136**:0,967 **138**:1,105 **139**:1,039 **140**:1,047

67) 1 **1**:0,995 **2**:1,106 **3**:1,005 **4**:0,928 **5**:0,894 **6**:0,942 **7**:0,968 **8**:0,921 **9**:0,94 **10**:0,946 **13**:0,951 **14**:0,993 **15**:0,916 **16**:0,946 **18**:1,008 **83**:1,043 **85**:0,922 **90**:1,021 **96**:1,002 **100**:1,01 **122**:0,836 **123**:1,027 **124**:1,146 **126**:1,097 **127**:1,036 **128**:1,054 **130**:1,097 **135**:1,162 **136**:1,104 **138**:1,025 **139**:1,084 **140**:1,075

68) -1 **1**:1,011 **2**:1,029 **3**:1,012 **4**:0,803 **5**:0,831 **6**:0,998 **7**:1,017 **8**:1,012 **9**:0,978 **10**:0,93 **13**:0,787 **14**:0,916 **15**:0,943 **16**:0,957 **18**:0,885 **83**:1,022 **85**:0,933 **90**:0,928 **96**:0,99 **100**:0,988 **122**:0,974 **123**:1,024 **124**:1,457 **126**:1,018 **127**:0,89 **128**:0,887 **130**:1,055 **135**:1,154 **136**:1,059 **138**:1,084 **139**:1,046 **140**:1,037

69) -1 **1**:0,93 **2**:1,021 **3**:0,997 **4**:0,929 **5**:0,869 **6**:0,88 **7**:1,004 **8**:1,057 **9**:0,963 **10**:0,905 **13**:0,951 **14**:0,99 **15**:1,039 **16**:1,059 **18**:1,03 **83**:1,025 **85**:0,926 **90**:0,976 **96**:1,039 **100**:1,032 **122**:0,966 **123**:1,039 **124**:1,137 **126**:1,163 **127**:0,968 **128**:0,863 **130**:1,129 **135**:0,963 **136**:0,929 **138**:0,978 **139**:1,092 **140**:1,09

70) 1 **1**:0,956 **2**:0,916 **3**:0,979 **4**:0,806 **5**:0,925 **6**:0,983 **7**:0,934 **8**:0,986 **9**:0,994 **10**:0,992 **13**:0,95 **14**:0,961 **15**:0,987 **16**:1,002 **18**:1,014 **83**:1,063 **85**:1,053 **90**:1,048 **96**:1,071 **100**:1,036 **122**:1,136 **123**:1,1 **124**:1,358 **126**:1,059 **127**:1,151 **128**:1,136 **130**:1,047 **135**:1,081 **136**:1,062 **138**:1,038 **139**:1,082 **140**:1,075

71) -1 **1**:0,873 **2**:0,964 **3**:0,848 **4**:0,929 **5**:0,604 **6**:0,782 **7**:0,813 **8**:0,882 **9**:1,038 **10**:0,909 **13**:0,987 **14**:0,941 **15**:0,857 **16**:0,847 **18**:0,84 **83**:0,989 **85**:0,677 **90**:0,891 **96**:0,751 **100**:0,891 **122**:1,009 **123**:1,197 **124**:1,018 **126**:1,297 **127**:1,17 **128**:1,027 **130**:1,009 **135**:1,136 **136**:0,955 **138**:1,033 **139**:1,003 **140**:1,02

72) 1 **1**:0,934 **2**:0,907 **3**:0,925 **4**:0,917 **5**:0,934 **6**:0,899 **7**:0,819 **8**:0,929 **9**:0,962 **10**:0,922 **13**:0,93 **14**:0,875 **15**:0,994 **16**:0,936 **18**:0,737 **83**:0,96 **85**:0,984 **90**:0,976 **96**:0,93 **100**:0,955 **122**:1,069 **123**:1,066 **124**:1,166 **126**:1,187 **127**:1,195 **128**:0,996 **130**:1,083 **135**:1,006 **136**:1,047 **138**:1,188 **139**:1,046 **140**:1,073

73) 1 **1**:0,999 **2**:0,99 **3**:1,037 **4**:0,863 **5**:0,964 **6**:0,976 **7**:0,877 **8**:0,808 **9**:1,017 **10**:0,837 **13**:0,942 **14**:0,931 **15**:0,949 **16**:0,778 **18**:0,781 **83**:1,058 **85**:0,902 **90**:0,989 **96**:0,9 **100**:0,946 **122**:0,895 **123**:1,016 **124**:1,202 **126**:0,992 **127**:1,088 **128**:1,109 **130**:1,249 **135**:1,032 **136**:1,28 **138**:1,353 **139**:0,986 **140**:1,041

74) 1 **1**:0,945 **2**:0,975 **3**:0,947 **4**:0,824 **5**:0,726 **6**:0,954 **7**:0,771 **8**:0,899 **9**:0,966 **10**:0,89 **13**:0,891 **14**:0,784 **15**:0,918 **16**:0,91 **18**:0,839 **83**:0,967 **85**:0,902 **90**:0,926 **96**:0,881 **100**:0,949 **122**:0,916 **123**:1,053 **124**:1,378 **126**:1,054 **127**:1,093 **128**:0,971 **130**:1,076 **135**:0,991 **136**:1,05 **138**:1,039 **139**:1,088 **140**:1,12

75) 1 **1**:0,937 **2**:0,883 **3**:0,939 **4**:0,929 **5**:0,709 **6**:0,905 **7**:0,788 **8**:0,747 **9**:0,984 **10**:0,95 **13**:0,844 **14**:0,963 **15**:0,939 **16**:0,831 **18**:0,873 **83**:0,944 **85**:0,845 **90**:1,018 **96**:0,945 **100**:1,009 **122**:1,065 **123**:1,03 **124**:1,151 **126**:1,135 **127**:1,365 **128**:1,521 **130**:1,094 **135**:1,112 **136**:1,302 **138**:1,085 **139**:1,159 **140**:1,137

76) -1 **1**:0,929 **2**:0,883 **3**:0,899 **4**:0,932 **5**:0,787 **6**:0,922 **7**:0,84 **8**:0,647 **9**:0,956 **10**:0,949 **13**:0,872 **14**:0,811 **15**:0,911 **16**:0,737 **18**:0,818 **83**:0,914 **85**:0,958 **90**:0,99 **96**:0,836 **100**:1,01 **122**:0,982 **123**:1,071 **124**:1,219 **126**:1,205 **127**:1,204 **128**:1,392 **130**:1,074 **135**:1,096 **136**:1,32 **138**:1,121 **139**:1,175 **140**:1,174

77) -1 **1**:0,807 **2**:0,754 **3**:0,68 **4**:0,672 **5**:0,594 **6**:0,486 **7**:0,607 **8**:0,606 **9**:0,748 **10**:0,671 **13**:0,691 **14**:0,647 **15**:0,52 **16**:0,541 **18**:0,697 **83**:0,959 **85**:0,807 **90**:0,929 **96**:0,855 **100**:1,013 **122**:1,26 **123**:1,395 **124**:1,449 **126**:2,238 **127**:1,523 **128**:1,47 **130**:1,352 **135**:1,69 **136**:1,627 **138**:1,188 **139**:1,287 **140**:1,352

78) -1 **1**:0,842 **2**:0,928 **3**:0,834 **4**:0,795 **5**:0,759 **6**:0,739 **7**:0,749 **8**:0,849 **9**:0,778 **10**:0,759 **13**:0,882 **14**:0,753 **15**:0,893 **16**:0,994 **18**:0,721 **83**:1,052 **85**:0,987 **90**:0,997 **96**:1,017 **100**:1,07 **122**:1,1 **123**:1,29 **124**:1,442 **126**:1,393 **127**:1,392 **128**:1,34 **130**:1,359 **135**:1,149 **136**:1,04 **138**:1,279 **139**:1,179 **140**:1,262

79) -1 **1**:0,842 **2**:0,98 **3**:0,693 **4**:0,556 **5**:0,667 **6**:0,381 **7**:0,739 **8**:0,746 **9**:0,957 **10**:0,814 **13**:0,815 **14**:0,825 **15**:0,944 **16**:0,744 **18**:0,75 **83**:0,936 **85**:0,799 **90**:0,851 **96**:0,98 **100**:1,225 **122**:1,269 **123**:1,346 **124**:1,585 **126**:3,14 **127**:1,36 **128**:1,409 **130**:1,106 **135**:1,146 **136**:1,426 **138**:1,367 **139**:1,439 **140**:1,415

80) -1 **1**:0,821 **2**:0,878 **3**:0,859 **4**:0,541 **5**:0,656 **6**:0,61 **7**:0,561 **8**:0,701 **9**:0,655 **10**:0,785 **13**:0,583 **14**:0,626 **15**:0,63 **16**:0,761 **18**:0,814 **83**:0,983 **85**:0,847 **90**:0,971 **96**:0,952 **100**:0,934 **122**:1,177 **123**:1,158 **124**:1,487 **126**:1,914 **127**:1,66 **128**:1,454 **130**:1,262 **135**:1,535 **136**:1,376 **138**:1,125 **139**:1,261 **140**:1,234

FIGURE 4 (4/6)

81) -1 **1**:0,945 **2**:1,086 **3**:0,957 **4**:0,74 **5**:0,856 **6**:0,933 **7**:0,937 **8**:0,599 **9**:1,012 **10**:0,676 **13**:0,983 **14**:0,407 **15**:0,983 **16**:0,571 **18**:0,63 **83**:0,98 **85**:0,866 **90**:1,031 **96**:0,675 **100**:1,746 **122**:0,888 **123**:1,056 **124**:1,41 **126**:1,11 **127**:1,037 **128**:1,242 **130**:1,596 **135**:1,013 **136**:1,377 **138**:2,696 **139**:0,992 **140**:2,396

82) -1 **1**:0,855 **2**:0,824 **3**:0,86 **4**:0,782 **5**:0,774 **6**:0,903 **7**:0,715 **8**:0,789 **9**:0,955 **10**:0,907 **13**:0,86 **14**:0,735 **15**:0,888 **16**:0,883 **18**:0,828 **83**:1,023 **85**:0,91 **90**:0,979 **96**:0,955 **100**:1,031 **122**:1,185 **123**:1,221 **124**:1,283 **126**:1,18 **127**:1,301 **128**:1,204 **130**:1,109 **135**:1,142 **136**:1,151 **138**:1,11 **139**:1,121 **140**:1,141

83) -1 **1**:1,036 **2**:0,984 **3**:1,023 **4**:0,962 **5**:1,037 **6**:0,96 **7**:0,991 **8**:0,957 **9**:0,936 **10**:1,024 **13**:1,014 **14**:0,997 **15**:1,015 **16**:1,023 **18**:1,022 **83**:1,024 **85**:0,857 **90**:0,913 **96**:0,971 **100**:0,935 **122**:0,951 **123**:0,99 **124**:0,987 **126**:0,896 **127**:0,887 **128**:0,96 **130**:0,905 **135**:0,89 **136**:0,935 **138**:0,97 **139**:0,978 **140**:0,93

84) -1 **1**:0,918 **2**:0,98 **3**:0,896 **4**:0,804 **5**:0,697 **6**:0,892 **7**:0,666 **8**:0,891 **9**:0,966 **10**:0,913 **13**:0,845 **14**:0,868 **15**:0,833 **16**:0,992 **18**:0,871 **83**:1,045 **85**:1,056 **90**:1,022 **96**:1,111 **100**:1,031 **122**:1,193 **123**:1,225 **124**:1,482 **126**:1,286 **127**:1,761 **128**:1,298 **130**:1,19 **135**:1,311 **136**:1,107 **138**:1,159 **139**:1,309 **140**:1,078

85) -1 **1**:0,961 **2**:0,999 **3**:0,938 **4**:0,716 **5**:0,808 **6**:0,731 **7**:0,831 **8**:0,895 **9**:0,743 **10**:0,898 **13**:0,894 **14**:0,808 **15**:0,834 **16**:0,907 **18**:0,814 **83**:1,019 **85**:0,885 **90**:1,001 **96**:0,995 **100**:1,036 **122**:1,052 **123**:1,095 **124**:1,276 **126**:1,271 **127**:1,14 **128**:1,139 **130**:1,144 **135**:1,08 **136**:1,185 **138**:1,236 **139**:1,103 **140**:1,105

86) -1 **1**:0,9 **2**:0,908 **3**:0,913 **4**:0,795 **5**:0,85 **6**:0,835 **7**:0,795 **8**:0,858 **9**:0,857 **10**:0,826 **13**:0,824 **14**:0,795 **15**:0,927 **16**:0,955 **18**:0,818 **83**:0,986 **85**:0,892 **90**:0,937 **96**:0,772 **100**:0,952 **122**:1,025 **123**:1,123 **124**:1,372 **126**:1,395 **127**:0,947 **128**:0,931 **130**:1,181 **135**:0,978 **136**:0,936 **138**:1,092 **139**:1,126 **140**:1,121

87) -1 **1**:0,972 **2**:1,054 **3**:0,905 **4**:0,941 **5**:0,811 **6**:0,708 **7**:0,886 **8**:0,704 **9**:0,899 **10**:0,917 **13**:0,955 **14**:0,907 **15**:0,977 **16**:0,873 **18**:0,928 **83**:0,792 **85**:0,902 **90**:0,983 **96**:0,934 **100**:0,985 **122**:0,868 **123**:0,943 **124**:1,192 **126**:1,604 **127**:1,173 **128**:1,424 **130**:1,135 **135**:1,049 **136**:1,18 **138**:1,096 **139**:1,111 **140**:1,152

88) -1 **1**:0,956 **2**:0,935 **3**:0,897 **4**:0,702 **5**:0,777 **6**:0,831 **7**:0,855 **8**:0,826 **9**:0,841 **10**:0,771 **13**:0,883 **14**:0,769 **15**:0,868 **16**:0,867 **18**:0,708 **83**:0,903 **85**:0,918 **90**:0,968 **96**:0,939 **100**:1,031 **122**:1,159 **123**:1,069 **124**:1,722 **126**:1,534 **127**:1,164 **128**:1,088 **130**:1,323 **135**:1,172 **136**:1,194 **138**:1,213 **139**:1,278 **140**:1,341

89) -1 **1**:0,9 **2**:0,888 **3**:0,874 **4**:0,716 **5**:0,722 **6**:0,703 **7**:0,758 **8**:0,681 **9**:0,777 **10**:0,81 **13**:0,781 **14**:0,869 **15**:0,742 **16**:0,711 **18**:0,731 **83**:0,861 **85**:0,818 **90**:0,957 **96**:0,836 **100**:0,996 **122**:1,014 **123**:1,04 **124**:1,456 **126**:1,607 **127**:1,098 **128**:1,2 **130**:1,245 **135**:1,105 **136**:1,354 **138**:1,175 **139**:1,328 **140**:1,346

90) -1 **1**:0,8 **2**:0,834 **3**:0,809 **4**:0,633 **5**:0,621 **6**:0,75 **7**:0,796 **8**:0,767 **9**:0,746 **10**:0,72 **13**:0,729 **14**:0,697 **15**:0,817 **16**:0,839 **18**:0,749 **83**:0,986 **85**:0,894 **90**:1,029 **96**:0,961 **100**:1,009 **122**:1,396 **123**:1,233 **124**:1,931 **126**:1,757 **127**:1,263 **128**:1,361 **130**:1,442 **135**:1,304 **136**:1,256 **138**:1,208 **139**:1,321 **140**:1,37

91) 1 **1**:0,74 **2**:0,864 **3**:0,78 **4**:0,708 **5**:0,833 **6**:0,832 **7**:0,836 **8**:0,849 **9**:0,729 **10**:0,725 **13**:0,803 **14**:0,837 **15**:0,939 **16**:0,977 **18**:0,751 **83**:0,943 **85**:0,969 **90**:1,008 **96**:1,004 **100**:0,947 **122**:1,274 **123**:1,228 **124**:1,402 **126**:1,601 **127**:1,275 **128**:1,17 **130**:1,412 **135**:1,131 **136**:1,067 **138**:1,19 **139**:1,154 **140**:1,167

92) 1 **1**:0,973 **2**:0,864 **3**:0,908 **4**:0,869 **5**:0,843 **6**:0,853 **7**:0,86 **8**:0,863 **9**:0,821 **10**:0,941 **13**:0,877 **14**:0,82 **15**:0,846 **16**:0,975 **18**:0,977 **83**:0,859 **85**:0,846 **90**:1,024 **96**:0,991 **100**:0,964 **122**:1,172 **123**:0,982 **124**:1,254 **126**:1,16 **127**:1,185 **128**:1,207 **130**:1,078 **135**:1,228 **136**:1,034 **138**:1,013 **139**:1,155 **140**:1,097

93) -1 **1**:0,843 **2**:0,739 **3**:0,757 **4**:0,68 **5**:0,695 **6**:0,715 **7**:0,732 **8**:0,801 **9**:0,706 **10**:0,86 **13**:0,865 **14**:0,899 **15**:0,737 **16**:0,936 **18**:0,802 **83**:0,936 **85**:0,88 **90**:0,944 **96**:0,95 **100**:0,936 **122**:1,223 **123**:1,238 **124**:1,728 **126**:1,682 **127**:1,328 **128**:1,41 **130**:1,156 **135**:1,303 **136**:1,072 **138**:1,203 **139**:1,211 **140**:1,221

94) -1 **1**:0,919 **2**:1,009 **3**:0,882 **4**:0,947 **5**:0,97 **6**:0,95 **7**:0,812 **8**:0,855 **9**:0,863 **10**:1,015 **13**:0,887 **14**:0,832 **15**:0,972 **16**:0,946 **18**:0,895 **83**:0,92 **85**:0,99 **90**:0,969 **96**:0,923 **100**:0,974 **122**:0,997 **123**:1,071 **124**:1,116 **126**:1,16 **127**:1,284 **128**:1,122 **130**:1,013 **135**:1,082 **136**:1,041 **138**:1,08 **139**:1,15 **140**:1,063

95) -1 **1**:0,87 **2**:0,848 **3**:0,873 **4**:0,769 **5**:0,677 **6**:0,784 **7**:0,817 **8**:0,741 **9**:0,918 **10**:0,898 **13**:0,793 **14**:0,806 **15**:0,949 **16**:0,827 **18**:0,82 **83**:0,903 **85**:0,797 **90**:0,96 **96**:0,849 **100**:0,963 **122**:1,168 **123**:1,059 **124**:1,347 **126**:1,382 **127**:1,277 **128**:1,237 **130**:1,109 **135**:1,003 **136**:1,161 **138**:0,987 **139**:1,196 **140**:1,17

96) 1 **1**:0,896 **2**:0,921 **3**:0,937 **4**:0,915 **5**:1,057 **6**:0,98 **7**:0,889 **8**:0,924 **9**:0,908 **10**:0,941 **13**:0,875 **14**:0,818 **15**:0,896 **16**:0,892 **18**:0,943 **83**:0,959 **85**:1,053 **90**:1,057 **96**:0,996 **100**:1,02 **122**:1,112 **123**:1,037 **124**:1,202 **126**:1,084 **127**:1,175 **128**:1,06 **130**:1,119 **135**:1,207 **136**:1,199 **138**:1,09 **139**:1,238 **140**:1,198

97) 1 **1**:0,788 **2**:0,852 **3**:0,763 **4**:0,683 **5**:0,71 **6**:0,811 **7**:0,872 **8**:0,854 **9**:0,745 **10**:0,734 **13**:0,725 **14**:0,67 **15**:0,864 **16**:0,844 **18**:0,753 **83**:0,957 **85**:1,043 **90**:1,058 **96**:1,001 **100**:1,07 **122**:1,149 **123**:1,286 **124**:1,799 **126**:1,726 **127**:1,175 **128**:1,161 **130**:1,486 **135**:1,302 **136**:1,291 **138**:1,341 **139**:1,456 **140**:1,48

98) 1 **1**:0,841 **2**:0,905 **3**:0,8 **4**:0,617 **5**:0,654 **6**:0,667 **7**:0,8 **8**:0,818 **9**:0,826 **10**:0,807 **13**:0,747 **14**:0,735 **15**:0,846 **16**:0,901 **18**:0,804 **83**:0,935 **85**:0,83 **90**:0,964 **96**:0,862 **100**:0,919 **122**:1,172 **123**:1,184 **124**:1,741 **126**:1,7 **127**:1,319 **128**:1,257 **130**:1,275 **135**:1,187 **136**:1,09 **138**:1,067 **139**:1,185 **140**:1,234

99) -1 **1**:0,927 **2**:0,876 **3**:0,88 **4**:0,87 **5**:0,655 **6**:0,861 **7**:0,804 **8**:0,775 **9**:0,901 **10**:0,956 **13**:0,805 **14**:0,788 **15**:0,893 **16**:0,913 **18**:0,836 **83**:0,907 **85**:0,756 **90**:0,978 **96**:0,911 **100**:0,91 **122**:1,098 **123**:1,079 **124**:1,205 **126**:1,396 **127**:1,226 **128**:1,299 **130**:1,045 **135**:1,016 **136**:1,07 **138**:1,038 **139**:1,088 **140**:1,067

100) -1 **1**:0,822 **2**:0,896 **3**:0,848 **4**:0,547 **5**:0,596 **6**:0,708 **7**:0,75 **8**:0,775 **9**:0,847 **10**:0,796 **13**:0,749 **14**:0,699 **15**:0,861 **16**:0,948 **18**:0,769 **83**:0,885 **85**:0,783 **90**:0,963 **96**:1,002 **100**:0,946 **122**:1,572 **123**:1,085 **124**:1,759 **126**:1,745 **127**:1,345 **128**:1,282 **130**:1,279 **135**:1,243 **136**:1,103 **138**:1,124 **139**:1,298 **140**:1,304

101) 1 **1**:0,798 **2**:0,771 **3**:0,701 **4**:0,81 **5**:0,812 **6**:0,895 **7**:0,777 **8**:0,817 **9**:0,743 **10**:0,826 **13**:0,771 **14**:0,725 **15**:0,823 **16**:0,842 **18**:0,894 **83**:0,926 **85**:0,996 **90**:1,064 **96**:0,989 **100**:1,079 **122**:1,315 **123**:1,338 **124**:1,383 **126**:1,38 **127**:1,41 **128**:1,24 **130**:1,323 **135**:1,364 **136**:1,308 **138**:1,138 **139**:1,311 **140**:1,288

FIGURE 4 (5/6)

102) 1 **1**:0,926 **2**:0,993 **3**:0,918 **4**:0,99 **5**:0,988 **6**:0,978 **7**:0,953 **8**:0,979 **9**:1,023 **10**:1,002 **13**:0,936 **14**:0,969 **15**:0,991 **16**:0,984 **18**:0,977 **83**:0,934 **85**:1,062 **90**:0,985 **96**:0,937 **100**:1,017 **122**:0,966 **123**:1,039 **124**:1,058 **126**:1,093 **127**:1,112 **128**:0,981 **130**:1,004 **135**:0,988 **136**:0,985 **138**:1,044 139:1,097 **140**:1,111

103) 1 **1**:0,938 **2**:0,951 **3**:0,829 **4**:0,873 **5**:0,914 **6**:0,967 **7**:0,923 **8**:0,941 **9**:0,95 **10**:0,986 **13**:0,919 **14**:0,771 **15**:0,952 **16**:0,921 **18**:0,947 **83**:0,831 **85**:1,001 **90**:1,011 **96**:0,95 **100**:0,991 **122**:1,01 **123**:1,034 **124**:1,279 **126**:1,096 **127**:1,062 **128**:0,968 **130**:1,038 **135**:1,088 **136**:1,101 **138**:1,038 139:1,124 **140**:1,125

104) 1 **1**:0,913 **2**:0,904 **3**:0,903 **4**:0,928 **5**:0,934 **6**:0,875 **7**:0,907 **8**:1,004 **9**:0,878 **10**:0,939 **13**:0,832 **14**:0,842 **15**:0,98 **16**:1,088 **18**:0,904 **83**:0,918 **85**:0,879 **90**:0,978 **96**:0,893 **100**:0,923 **122**:1,063 **123**:1,03 **124**:0,996 **126**:1,412 **127**:0,887 **128**:0,808 **130**:1,06 **135**:0,936 **136**:0,805 **138**:1,012 139:1,035 **140**:1,032

105) 1 **1**:1,042 **2**:0,977 **3**:1,021 **4**:1,006 **5**:0,869 **6**:0,952 **7**:0,885 **8**:0,99 **9**:0,961 **10**:0,957 **13**:0,967 **14**:0,994 **15**:0,858 **16**:0,94 **18**:0,937 **83**:0,951 **85**:0,866 **90**:0,985 **96**:0,931 **100**:1,013 **122**:0,963 **123**:0,927 **124**:0,944 **126**:1,01 **127**:1,034 **128**:0,895 **130**:1,048 **135**:1,117 **136**:1,055 **138**:1,029 139:1,072 **140**:1,065

FIGURE 4 (6/6)

FIGURE 5

FIGURE 6

FIGURE 7

FIGURE 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **CAEYENBERGHS et al.** *Human Brain Mapping,* 2010, vol. 31 (7), 992-1002
- **WANG et al.** *American Journal of Neuroradiology,* 2009, vol. 30 (10), 1907-1913
- **SIDAROS et al.** *Brain,* 2008, vol. 131 (2), 559-572
- **PICARD R. ; COOK D.** Cross-Validation of Regression Models. *Journal of the American Statistical Association,* 1984, vol. 79 (387), 575-583
- **FAWCETT T.** An introduction to ROC analysis. *Pattern Recognition Letters,* 2006, vol. 27, 861-874
- **P.J. BASSER ; J. MATTIELLO ; D. LEBIHAN.** MR diffusion tensor spectroscopy and imaging. *Biophysical Journal,* 1994, vol. 66 (1), 259-267
- **P. J. BASSER ; C. PIERPAOLI.** Microstructuraland Physiological Features of Tissues Elucidated by Quantitative-Diffusion-Tensor MRI. *Journal of Magnetic Resonance,* 1996, vol. 111 (3), 209-219
- **M. JENKINSON ; S.M. SMITH.** A global optimisation method for robust affine registration of brain images. *Medical Image Analysis,* 2001, vol. 5 (2), 143-156
- **M. JENKINSON ; P.R. BANNISTER ; J.M. BRADY ; S.M. SMITH.** Improved optimisation for the robust and accurate linear registration and motion correction of brain images. *NeuroImage,* 2002, vol. 17 (2), 825-841
- **T. LESCOT ; L. ABDENNOUR ; A.-L. BOCH ; L. PUYBASSET.** Treatment of intracranialhypertension. *Curr Opin Crit Care,* 2008, vol. 14, 129-134
- **S.M. SMITH.** Fast robust automated brain extraction. *Human Brain Mapping,* 2002, vol. 17 (3), 143-155
- **S.M. SMITH ; M. JENKINSON ; M.W. WOOLRICH ; C.F. BECKMANN ; T.E.J. BEHRENS ; H. JOHANSEN-BERG ; P.R. BANNISTER ; M. DE LUCA ; I. DROBNJAK ; D.E. FLITNEY.** Advances in functional and structural MR image analysis and implementation as FSL. *NeuroImage,* 2004, vol. 23 (S1), 208-219
- **S.M. SMITH ; M. JENKINSON ; H. JOHANSEN-BERG ; D. RUECKERT ; T.E. NICHOLS ; C.E. MACKAY ; K.E. WATKINS ; O. CICCARELLI ; M.Z. CADER ; P.M. MATTHEWS.** Tract-based spatial statistics: Voxelwise analysis of multi-subject diffusion data. *NeuroImage,* 2006, vol. 31, 1487-1505
- **S. MORI ; S. WAKANA ; L.M. NAGAE-POETSCHER ; P.C.M. VAN ZIJL.** MRI Atlas of Human White Matter. Elsevier, 2005
- **J.L.R. ANDERSSON ; M. JENKINSON ; S. SMITH.** Non-linear optimisation. *FMRIB technical report TR07JA1,* www.fmrib.ox.ac.uk/analysis/techrep
- **J.L.R. ANDERSSON ; M. JENKINSON ; S. SMITH.** Non-linear registration, aka Spatial normalisation. *FMRIB technical report TR07JA2,* www.fmrib.ox.ac.uk/analysis/techrep
- **ROBERT W COX ; JOHN ASHBURNER ; HESTER BREMAN ; KATE FISSELL ; CHRISTIAN HASELGROVE ; COLIN J HOLMES ; JACK L LANCASTER ; DAVID E REX ; STEPHEN M SMITH ; JEFFREY B WOODWARD.** A (Sort of) New Image Data Format Standard: NifTI-1. *NeuroImage,* 2004, vol. 22
- **RORDEN, C. ; BRETT, M.** Stereotaxic display of brain lesions. *Behavioural Neurology,* 2000, vol. 12, 191-200
- **CHIH-CHUNG CHANG ; CHIH-JEN LIN.** LIBSVM : a library for support vector machines. *ACM Transactions on Intelligent Systems and Technology,* 2011, vol. 2, http://www.csie.ntu.edu.tw/-cjlin/libsvm
- Combining SVMs with various feature selection strategies. Chapter of the book. **Y.-W. CHEN ; C.-J. LIN ; ISABELLE GUYON ; STEVE GUNN ; MASOUD NIKRAVESH ; LOTFI A. ZADEH.** Feature Extraction: Foundations and Applications (Studies in Fuzziness and Soft Computing). Springer, 2006
- **GIACINO J.T. ; ZASLER N.D.** Outcome after severe traumatic brain injury: coma, the vegetative state, and the minimally responsive state. *J Head Trauma Rehabil,* 1995, vol. 10, 40-56